# EUROPEAN PATENT SPECIFICATION

(11) **EP 2 825 168 B1**
(45) Date of publication and mention of the grant of the patent: **09.05.2018**
(21) Application number: 13712647.0
(22) Date of filing: 14.03.2013
(51) Int. Cl.: A61K 31/4985, A61P 35/00

(54) **TREATMENT OF CANCER WITH TOR KINASE INHIBITORS**
BEHANDLUNG VON KREBS MIT TOR-KINASEHEMMERN
TRAITEMENT DU CANCER AVEC DES INHIBITEURS DE LA KINASE TOR

(30) Priority: 15.03.2012 US 201261611370 P; 18.10.2012 US 201261715329 P
(43) Date of publication of application: 21.01.2015
(73) Proprietor: Signal Pharmaceuticals, LLC, San Diego, CA 92121 (US)
(72) Inventor: XU, Shuichan, San Diego, CA 92127 (US); HEGE, Kristen, Mae, Burlingame, CA 94010 (US)
(74) Representative: Jones Day
(86) International application number: PCT/US2013/031202
(87) International publication number: WO 2013/138556

(56) References cited:
- WO-A1-2008/051494
- WO-A1-2010/062571
- WO-A2-2008/051493
- WO-A2-2011/133668
- VIVEK SUBBIAH ET AL: "Targeted Morphoproteomic Profiling of Ewing's Sarcoma Treated with Insulin-Like Growth Factor 1 Receptor (IGF1R) Inhibitors: Response/Resistance Signatures", PLOS ONE, vol. 6, no. 4, 1 January 2011 (2011-01-01) , pages e18424-e18424, XP55061616, ISSN: 1932-6203, DOI: 10.1371/journal.pone.0018424
- VINCENZI BRUNO ET AL: "Targeted therapy in sarcomas: mammalian target of rapamycin inhibitors from bench to bedside", EXPERT OPINION ON INVESTIGATIONAL DRUGS, INFORMA HEALTHCARE, UNITED KINGDOM, vol. 20, no. 12, 1 December 2011 (2011-12-01), pages 1685-1705, XP009169335, ISSN: 1744-7658, DOI: 10.1517/13543784.2011.628984
- DANCEY JANET E ET AL: "Ridaforolimus: a promising drug in the treatment of soft-tissue sarcoma and other malignancies", FUTURE ONCOLOGY, LONDON, vol. 7, no. 7, 1 July 2011 (2011-07-01), pages 827-839, XP009169333, ISSN: 1744-8301, DOI: 10.2217/FON.11.57
- MARK AGULNIK: "New developments in mammalian target of rapamycin inhibitors for the treatment of sarcoma", CANCER, vol. 118, no. 6, 11 August 2011 (2011-08-11), pages 1486-1497, XP55061618, ISSN: 0008-543X, DOI: 10.1002/cncr.26361
- R. M. SQUILLACE ET AL: "Antitumor Activity of Ridaforolimus and Potential Cell-Cycle Determinants of Sensitivity in Sarcoma and Endometrial Cancer Models", MOLECULAR CANCER THERAPEUTICS, vol. 10, no. 10, 1 October 2011 (2011-10-01), pages 1959-1968, XP55061614, ISSN: 1535-7163, DOI: 10.1158/1535-7163.MCT-11-0273
- MITA MONICA M ET AL: "Phase I trial of the novel mammalian target of rapamycin inhibitor deforolimus (AP23573; MK-8669) administered intravenously daily for 5 days every 2 weeks to patients with advanced malignancies.", JOURNAL OF CLINICAL ONCOLOGY : OFFICIAL JOURNAL OF THE AMERICAN SOCIETY OF CLINICAL ONCOLOGY 20 JAN 2008, vol. 26, no. 3, 20 January 2008 (2008-01-20), pages 361-367, XP002696908, ISSN: 1527-7755
- BALAMUTH N J ET AL: "Ewing's sarcoma", LANCET ONCOLOGY, LANCET PUBLISHING GROUP, LONDON, GB, vol. 11, no. 2, 1 February 2010 (2010-02-01), pages 184-192, XP026884321, ISSN: 1470-2045, DOI: 10.1016/S1470-2045(09)70286-4 [retrieved on 2010-02-01]
- SILVIA MATEO-LOZANO ET AL: "Rapamycin induces the fusion-type independent downregulation of the EWS/FLI-1 proteins and inhibits Ewing's sarcoma cell proliferation", ONCOGENE, NATURE PUBLISHING GROUP, GB, vol. 22, no. 58, 18 December 2003 (2003-12-18), pages 9282-9287, XP002665931, ISSN: 0950-9232, DOI: 10.1038/SJ.ONC.1207081
- ANA S MARTINS ET AL: "Insulin-Like Growth Factor IReceptor Pathway Inhibition byADW742, Alone or in Combinationwith Imatinib,Doxorubicin, orVincristine, Is a Novel Therapeutic Approach in EwingTumor", CLINICAL CANCER RESEARCH, THE AMERICAN ASSOCIATION FOR CANCER RESEARCH, US, vol. 12, no. 11, 1 June 2006 (2006-06-01), pages 3532-3540, XP002551195, ISSN: 1078-0432, DOI: 10.1158/1078-0432.CCR-05-1778
- P. C. HOLLENHORST ET AL: "Oncogenic ETS proteins mimic activated RAS/MAPK signaling in prostate cells", GENES & DEVELOPMENT, vol. 25, no. 20, 15 October 2011 (2011-10-15), pages 2147-2157, XP55061642, ISSN: 0890-9369, DOI: 10.1101/gad.17546311
- OIKAWA T: "ETS TRANSCRIPTION FACTORS: POSSIBLE TARGETS FOR CANCER THERAPY", CANCER SCIENCE, JAPANESE CANCER ASSOCIATION, TOKYO, JP, vol. 95, no. 8, 1 August 2004 (2004-08-01) , pages 626-633, XP009039358, ISSN: 1347-9032, DOI: 10.1111/J.1349-7006.2004.TB03320.X
- LORI BERK ET AL: "Analysis of the pharmacodynamic activity of the mTOR inhibitor ridaforolimus (AP23573, MK-8669) in a phase 1 clinical trial", CANCER CHEMOTHERAPY AND PHARMACOLOGY, SPRINGER, BERLIN, DE, vol. 69, no. 5, 10 January 2012 (2012-01-10), pages 1369-1377, XP035047366, ISSN: 1432-0843, DOI: 10.1007/S00280-011-1813-7

## Description

### 1. FIELD

Provided herein are methods for treating or preventing ETS overexpressing Ewings sarcoma, comprising administering an effective amount of a TOR kinase inhibitor to a patient having ETS overexpressing Ewings sarcoma.

### 2. BACKGROUND

The connection between abnormal protein phosphorylation and the cause or consequence of diseases has been known for over 20 years. Accordingly, protein kinases have become a very important group of drug targets. *See* Cohen, Nature, 1:309-315 (2002). Various protein kinase inhibitors have been used clinically in the treatment of a wide variety of diseases, such as cancer and chronic inflammatory diseases, including diabetes and stroke. *See* Cohen, Eur. J. Biochem., 268:5001-5010 (2001), Protein Kinase Inhibitors for the Treatment of Disease: The Promise and the Problems, Handbook of Experimental Pharmacology, Springer Berlin Heidelberg, 167 (2005).

The protein kinases are a large and diverse family of enzymes that catalyze protein phosphorylation and play a critical role in cellular signaling. Protein kinases may exert positive or negative regulatory effects, depending upon their target protein. Protein kinases are involved in specific signaling pathways which regulate cell functions such as, but not limited to, metabolism, cell cycle progression, cell adhesion, vascular function, apoptosis, and angiogenesis. Malfunctions of cellular signaling have been associated with many diseases, the most characterized of which include cancer and diabetes. The regulation of signal transduction by cytokines and the association of signal molecules with protooncogenes and tumor suppressor genes have been well documented. Similarly, the connection between diabetes and related conditions, and deregulated levels of protein kinases, has been demonstrated. *See e.g.,* Sridhar et al. Pharmaceutical Research, 17(11):1345-1353 (2000). Viral infections and the conditions related thereto have also been associated with the regulation of protein kinases. Park et al. Cell 101 (7): 777-787 (2000).

Because protein kinases regulate nearly every cellular process, including metabolism, cell proliferation, cell differentiation, and cell survival, they are attractive targets for therapeutic intervention for various disease states. For example, cell-cycle control and angiogenesis, in which protein kinases play a pivotal role are cellular processes associated with numerous disease conditions such as but not limited to cancer, inflammatory diseases, abnormal angiogenesis and diseases related thereto, atherosclerosis, macular degeneration, diabetes, obesity, and pain.

Protein kinases have become attractive targets for the treatment of cancers. Fabbro et al., Pharmacology & Therapeutics 93:79-98 (2002). It has been proposed that the involvement of protein kinases in the development of human malignancies may occur by: (1) genomic rearrangements (*e.g.,* BCR-ABL in chronic myelogenous leukemia), (2) mutations leading to constitutively active kinase activity, such as acute myelogenous leukemia and gastrointestinal tumors, (3) deregulation of kinase activity by activation of oncogenes or loss of tumor suppressor functions, such as in cancers with oncogenic RAS, (4) deregulation of kinase activity by over-expression, as in the case of EGFR and (5) ectopic expression of growth factors that can contribute to the development and maintenance of the neoplastic phenotype. Fabbro et al., Pharmacology & Therapeutics 93:79-98 (2002).

The elucidation of the intricacy of protein kinase pathways and the complexity of the relationship and interaction among and between the various protein kinases and kinase pathways highlights the importance of developing pharmaceutical agents capable of acting as protein kinase modulators, regulators or inhibitors that have beneficial activity on multiple kinases or multiple kinase pathways. Accordingly, there remains a need for new kinase modulators.

The protein named mTOR (mammalian target of rapamycin), which is also called FRAP, RAFTI or RAPT1), is a 2549-amino acid Ser/Thr protein kinase, that has been shown to be one of the most critical proteins in the mTOR/PI3K/Akt pathway that regulates cell growth and proliferation. Georgakis and Younes Expert Rev. Anticancer Ther. 6(1):131-140 (2006). mTOR exists within two complexes, mTORC1 and mTORC2. While mTORC1 is sensitive to rapamycin analogs (such as temsirolimus or everolimus), mTORC2 is largely rapamycin-insensitive. Notably, rapamycin is not a TOR kinase inhibitor. Several mTOR inhibitors have been or are being evaluated in clinical trials for the treatment of cancer. Temsirolimus was approved for use in renal cell carcinoma in 2007 and sirolimus was approved in 1999 for the prophylaxis of renal transplant rejection. Everolimus was approved in 2009 for renal cell carcinoma patients that have progressed on vascular endothelial growth factor receptor inhibitors, in 2010 for subependymal giant cell astrocytoma (SEGA) associated with tuberous sclerosis (TS) in patients who require therapy but are not candidates for surgical resection, and in 2011 for progressive neuroendocrine tumors of pancreatic origin (PNET) in patients with unresectable, locally advanced or metastatic disease. There remains a need for additional TOR kinase inhibitors.

The Ewings family of sarcomas are aggressive tumors of bone and soft tissue, defined by chromosomal translocations resulting in E-twenty six (ETS) gene fusions. The most common fusion gene constructs include the Ewings Sarcoma Breakpoint Region-1 (EWS) gene fused to the DNA binding domain of an ETS transcription factor (FLI-1, ETV1 or ERG). Dysregulation of the resulting chimeric proteins is associated with abnormal proliferation, invasion and tumorigenesis. Brenner et al., Biochemica et Biophysica Acta 1796: 201-215 (2009). Inhibition of either DNA-PK or PARP1 via siRNA inhibits tumor cell invasion of ETS+ Ewings sarcoma *in vitro* and ETS+ Ewings sarcoma is sensitive to treatment with PARP-1 inhibitors *in vivo* (Brenner et al., Cancer Res. Jan 27 Epub ahead of print (2012)). In addition, ETS fusion proteins are associated with activation of the mTOR pathway in both cell lines and primary tumors and rapamycin exposure downregulates ETS chimeric protein levels and inhibits tumor cell proliferation. Mateo-Lozano et al., Oncogene 22: 9282-9287 (2003); Zanali et al., Ann. Clin. Lab. Sci. 39(2): 160-166 (2009).

Citation or identification of any reference in Section 2 of this application is not to be construed as an admission that the reference is prior art to the present application.

### 3. SUMMARY

Provided herein is 1-ethyl-7-(2-methyl-6-(1H-1,2,4-triazol-3-yl)pyridin-3-yl)-3,4-dihydropyrazino[2,3-b]pyrazin-2(1H)-one or a pharmaceutically acceptable salt, clathrate, solvate, stereoisomer, tautomer or isotopologue thereof for use in a method for treating E-twenty six (ETS) overexpressing Ewings sarcoma, the method comprising administering an effective amount of 1-ethyl-7-(2-methyl-6-(1H-1,2,4-triazol-3-yl)pyridin-3-yl)-3,4-dihydropyrazino[2,3-b]pyrazin-2(1H)-one or a pharmaceutically acceptable salt, clathrate, solvate, stereoisomer, tautomer or isotopologue thereof to a patient having ETS overexpressing Ewings sarcoma.

Further provided herein is 1-ethyl-7-(2-methyl-6-(1H-1,2,4-triazol-3-yl)pyridin-3-yl)-3,4-dihydropyrazino[2,3-b]pyrazin-2(1H)-one or a pharmaceutically acceptable salt, clathrate, solvate, stereoisomer, tautomer or isotopologue thereof for use in a method for improving the Response Evaluation Criteria in Solid Tumors (RECIST 1.1) of a patient, the method comprising administering an effective amount of 1-ethyl-7-(2-methyl-6-(1H-1,2,4-triazol-3-yl)pyridin-3-yl)-3,4-dihydropyrazino[2,3-b]pyrazin-2(1H)-one or a pharmaceutically acceptable salt, clathrate, solvate, stereoisomer, tautomer or isotopologue thereof to a patient having E-twenty six (ETS) overexpressing Ewings sarcoma.

Throughout the application, 1-ethyl-7-(2-methyl-6-(1H-1,2,4-triazol-3-yl)pyridin-3-yl)-3,4-dihydropyrazino [2,3-b]pyrazin-2(1H)-one is denoted as "Compound 1".

The present embodiments can be understood more fully by reference to the detailed description and examples, which are intended to exemplify non-limiting embodiments.

### 4. DETAILED DESCRIPTION

### 4.1 DEFINITIONS

An "alkyl" group is a saturated, partially saturated, or unsaturated straight chain or branched non-cyclic hydrocarbon having from 1 to 10 carbon atoms, typically from 1 to 8 carbons or, in some embodiments, from 1 to 6, 1 to 4, or 2 to 6 or carbon atoms. Representative alkyl groups include -methyl, -ethyl, -n-propyl, -n-butyl, -n-pentyl and -n-hexyl; while saturated branched alkyls include -isopropyl, -*sec*-butyl, -isobutyl, -*tert*-butyl, -isopentyl, 2-methylpentyl, 3-methylpentyl, 4-methylpentyl, 2,3-dimethylbutyl and the like. Examples of unsaturared alkyl groups include, but are not limited to, vinyl, allyl, -CH=CH(CH₃), -CH=C(CH₃)₂, -C(CH₃)=CH₂, -C(CH₃)=CH(CH₃), -C(CH₂CH₃)=CH₂, -C≡CH, -C≡C(CH₃), -C≡C(CH₂CH₃), -CH₂C≡CH, -CH₂C≡C(CH₃) and -CH₂C≡C(CH₇CH₃), among others. An alkyl group can be substituted or unsubstituted. In certain embodiments, when the alkyl groups described herein are said to be "substituted," they may be substituted with any substituent or substituents as those found in the exemplary compounds and embodiments disclosed herein, as well as halogen (chloro, iodo, bromo, or fluoro); hydroxyl; alkoxy; alkoxyalkyl; amino; alkylamino; carboxy; nitro; cyano; thiol; thioether; imine; imide; amidine; guanidine; enamine; aminocarbonyl; acylamino; phosphonato; phosphine; thiocarbonyl; sulfonyl; sulfone; sulfonamide; ketone; aldehyde; ester; urea; urethane; oxime; hydroxyl amine; alkoxyamine; aralkoxyamine; N-oxide; hydrazine; hydrazide; hydrazone; azide; isocyanate; isothiocyanate; cyanate; thiocyanate; B(OH)₂, or O(alkyl)aminocarbonyl.

An "alkenyl" group is a straight chain or branched non-cyclic hydrocarbon having from 2 to 10 carbon atoms, typically from 2 to 8 carbon atoms, and including at least one carbon-carbon double bond. Representative straight chain and branched (C₂-C₈)alkenyls include -vinyl, -allyl, -1-butenyl, -2-butenyl, -isobutylenyl, -1-pentenyl, -2-pentenyl, -3-methyl-1-butenyl, -2-methyl-2-butenyl, -2,3-dimethyl-2-butenyl, -1-hexenyl, -2-hexenyl, -3-hexenyl, -1-heptenyl, -2-heptenyl, -3-heptenyl, -1-octenyl, -2-octenyl, -3-octenyl and the like. The double bond of an alkenyl group can be unconjugated or conjugated to another unsaturated group. An alkenyl group can be unsubstituted or substituted.

A "cycloalkyl" group is a saturated, partially saturated, or unsaturated cyclic alkyl group of from 3 to 10 carbon atoms having a single cyclic ring or multiple condensed or bridged rings which can be optionally substituted with from 1 to 3 alkyl groups. In some embodiments, the cycloalkyl group has 3 to 8 ring members, whereas in other embodiments the number of ring carbon atoms ranges from 3 to 5, 3 to 6, or 3 to 7. Such cycloalkyl groups include, by way of example, single ring structures such as cyclopropyl, cyclobutyl, cyclopentyl, cyclohexyl, cycloheptyl, cyclooctyl, 1-methylcyclopropyl, 2-methylcyclopentyl, 2-methylcyclooctyl, and the like, or multiple or bridged ring structures such as adamantyl and the like. Examples of unsaturared cycloalkyl groups include cyclohexenyl, cyclopentenyl, cyclohexadienyl, butadienyl, pentadienyl, hexadienyl, among others. A cycloalkyl group can be substituted or unsubstituted. Such substituted cycloalkyl groups include, by way of example, cyclohexanone and the like.

An "aryl" group is an aromatic carbocyclic group of from 6 to 14 carbon atoms having a single ring (*e.g.,* phenyl) or multiple condensed rings (*e.g.,* naphthyl or anthryl). In some embodiments, aryl groups contain 6-14 carbons, and in others from 6 to 12 or even 6 to 10 carbon atoms in the ring portions of the groups. Particular aryls include phenyl, biphenyl, naphthyl and the like. An aryl group can be substituted or unsubstituted. The phrase "aryl groups" also includes groups containing fused rings, such as fused aromatic-aliphatic ring systems (e.g., indanyl, tetrahydronaphthyl, and the like).

A "heteroaryl" group is an aryl ring system having one to four heteroatoms as ring atoms in a heteroaromatic ring system, wherein the remainder of the atoms are carbon atoms. In some embodiments, heteroaryl groups contain 5 to 6 ring atoms, and in others from 6 to 9 or even 6 to 10 atoms in the ring portions of the groups. Suitable heteroatoms include oxygen, sulfur and nitrogen. In certain embodiments, the heteroaryl ring system is monocyclic or bicyclic. Non-limiting examples include but are not limited to, groups such as pyrrolyl, pyrazolyl, imidazolyl, triazolyl, tetrazolyl, oxazolyl, isoxazolyl, thiazolyl, pyrolyl, pyridyl, pyridazinyl, pyrimidinyl, pyrazinyl, thiophenyl, benzothiophenyl, furanyl, benzofuranyl (for example, isobenzofuran-1,3-diimine), indolyl, azaindolyl (for example, pyrrolopyridyl or 1H-pyrrolo[2,3-b]pyridyl), indazolyl, benzimidazolyl (for example, 1H-benzo[d]imidazolyl), imidazopyridyl (for example, azabenzimidazolyl, 3H-imidazo[4,5-b]pyridyl or 1H-imidazo[4,5-b]pyridyl), pyrazolopyridyl, triazolopyridyl, benzotriazolyl, benzoxazolyl, benzothiazolyl, benzothiadiazolyl, isoxazolopyridyl, thianaphthalenyl, purinyl, xanthinyl, adeninyl, guaninyl, quinolinyl, isoquinolinyl, tetrahydroquinolinyl, quinoxalinyl, and quinazolinyl groups.

A "heterocyclyl" is an aromatic (also referred to as heteroaryl) or non-aromatic cycloalkyl in which one to four of the ring carbon atoms are independently replaced with a heteroatom from the group consisting of O, S and N. In some embodiments, heterocyclyl groups include 3 to 10 ring members, whereas other such groups have 3 to 5, 3 to 6, or 3 to 8 ring members. Heterocyclyls can also be bonded to other groups at any ring atom (*i.e*., at any carbon atom or heteroatom of the heterocyclic ring). A heterocyclylalkyl group can be substituted or unsubstituted. Heterocyclyl groups encompass unsaturated, partially saturated and saturated ring systems, such as, for example, imidazolyl, imidazolinyl and imidazolidinyl groups. The phrase heterocyclyl includes fused ring species, including those comprising fused aromatic and non-aromatic groups, such as, for example, benzotriazolyl, 2,3-dihydrobenzo[1,4]dioxinyl, and benzo[1,3]dioxolyl. The phrase also includes bridged polycyclic ring systems containing a heteroatom such as, but not limited to, quinuclidyl. Representative examples of a heterocyclyl group include, but are not limited to, aziridinyl, azetidinyl, pyrrolidyl, imidazolidinyl, pyrazolidinyl, thiazolidinyl, tetrahydrothiophenyl, tetrahydrofuranyl, dioxolyl, furanyl, thiophenyl, pyrrolyl, pyrrolinyl, imidazolyl, imidazolinyl, pyrazolyl, pyrazolinyl, triazolyl, tetrazolyl, oxazolyl, isoxazolyl, thiazolyl, thiazolinyl, isothiazolyl, thiadiazolyl, oxadiazolyl, piperidyl, piperazinyl, morpholinyl, thiomorpholinyl, tetrahydropyranyl (for example, tetrahydro-2H-pyranyl), tetrahydrothiopyranyl, oxathiane, dioxyl, dithianyl, pyranyl, pyridyl, pyrimidinyl, pyridazinyl, pyrazinyl, triazinyl, dihydropyridyl, dihydrodithiinyl, dihydrodithionyl, homopiperazinyl, quinuclidyl, indolyl, indolinyl, isoindolyl, azaindolyl (pyrrolopyridyl), indazolyl, indolizinyl, benzotriazolyl, benzimidazolyl, benzofuranyl, benzothiophenyl, benzthiazolyl, benzoxadiazolyl, benzoxazinyl, benzodithiinyl, benzoxathiinyl, benzothiazinyl, benzoxazolyl, benzothiazolyl, benzothiadiazolyl, benzo[1,3]dioxolyl, pyrazolopyridyl, imidazopyridyl (azabenzimidazolyl; for example, 1H-imidazo[4,5-b]pyridyl, or 1H-imidazo[4,5-b]pyridin-2(3H)-onyl), triazolopyridyl, isoxazolopyridyl, purinyl, xanthinyl, adeninyl, guaninyl, quinolinyl, isoquinolinyl, quinolizinyl, quinoxalinyl, quinazolinyl, cinnolinyl, phthalazinyl, naphthyridinyl, pteridinyl, thianaphthalenyl, dihydrobenzothiazinyl, dihydrobenzofuranyl, dihydroindolyl, dihydrobenzodioxinyl, tetrahydroindolyl, tetrahydroindazolyl, tetrahydrobenzimidazolyl, tetrahydrobenzotriazolyl, tetrahydropyrrolopyridyl, tetrahydropyrazolopyridyl, tetrahydroimidazopyridyl, tetrahydrotriazolopyridyl, and tetrahydroquinolinyl groups. Representative substituted heterocyclyl groups may be mono- substituted or substituted more than once, such as, but not limited to, pyridyl or morpholinyl groups, which are 2-, 3-, 4-, 5-, or 6-substituted, or disubstituted with various substituents such as those listed below.

An "cycloalkylalkyl" group is a radical of the formula: -alkyl-cycloalkyl, wherein alkyl and cycloalkyl are defined above. Substituted cycloalkylalkyl groups may be substituted at the alkyl, the cycloalkyl, or both the alkyl and the cycloalkyl portions of the group. Representative cycloalkylalkyl groups include but are not limited to cyclopentylmethyl, cyclopentylethyl, cyclohexylmethyl, cyclohexylethyl, and cyclohexylpropyl. Representative substituted cycloalkylalkyl groups may be monosubstituted or substituted more than once.

An "aralkyl" group is a radical of the formula: -alkyl-aryl, wherein alkyl and aryl are defined above. Substituted aralkyl groups may be substituted at the alkyl, the aryl, or both the alkyl and the aryl portions of the group. Representative aralkyl groups include but are not limited to benzyl and phenethyl groups and fused (cycloalkylaryl)alkyl groups such as 4-ethyl-indanyl.

A "heterocyclylalkyl" group is a radical of the formula: -alkyl-heterocyclyl, wherein alkyl and heterocyclyl are defined above. Substituted heterocyclylalkyl groups may be substituted at the alkyl, the heterocyclyl, or both the alkyl and the heterocyclyl portions of the group. Representative heterocylylalkyl groups include but are not limited to 4-ethyl-morpholinyl, 4-propylmorpholinyl, furan-2-yl methyl, furan-3-yl methyl, pyrdine-3-yl methyl, (tetrahydro-2H-pyran-4-yl)methyl, (tetrahydro-2H-pyran-4-yl)ethyl, tetrahydrofuran-2-yl methyl, tetrahydrofuran-2-yl ethyl, and indol-2-yl propyl.

A "halogen" is fluorine, chlorine, bromine or iodine.

A "hydroxyalkyl" group is an alkyl group as described above substituted with one or more hydroxy groups.

An "alkoxy" group is -O-(alkyl), wherein alkyl is defined above.

An "alkoxyalkyl" group is -(alkyl)-O-(alkyl), wherein alkyl is defined above.

An "amino" group is a radical of the formula: -NH₂.

An "alkylamino" group is a radical of the formula: -NH-alkyl or -N(alkyl)₂, wherein each alkyl is independently as defined above.

A "carboxy" group is a radical of the formula: -C(O)OH.

An "aminocarbonyl" group is a radical of the formula: -C(O)N(R^{#})₂, -C(O)NH(R^{#}) or -C(O)NH₂, wherein each R^{#} is independently a substituted or unsubstituted alkyl, cycloalkyl, aryl, aralkyl, heterocyclyl or heterocyclyl group as defined herein.

An "acylamino" group is a radical of the formula: -NHC(O)(R^{#}) or -N(alkyl)C(O)(R^{#}), wherein each alkyl and R^{#} are independently as defined above.

An "alkylsulfonylamino" group is a radical of the formula: -NHSO₂(R^{#}) or -N(alkyl)SO₂(R^{#}), wherein each alkyl and R^{#} are defined above.

A "urea" group is a radical of the formula: -N(alkyl)C(O)N(R^{#})₂, -N(alkyl)C(O)NH(R^{#}), -N(alkyl)C(O)NH₂, -NHC(O)N(R)₂, -NHC(O)NH(R^{#}), or -NH(CO)NHR^{#}, wherein each alkyl and R^{#} are independently as defined above.

When the groups described herein, with the exception of alkyl group are said to be "substituted," they may be substituted with any appropriate substituent or substituents. Illustrative examples of substituents are those found in the exemplary compounds and embodiments disclosed herein, as well as halogen (chloro, iodo, bromo, or fluoro); alkyl; hydroxyl; alkoxy; alkoxyalkyl; amino; alkylamino; carboxy; nitro; cyano; thiol; thioether; imine; imide; amidine; guanidine; enamine; aminocarbonyl; acylamino; phosphonato; phosphine; thiocarbonyl; sulfonyl; sulfone; sulfonamide; ketone; aldehyde; ester; urea; urethane; oxime; hydroxyl amine; alkoxyamine; aralkoxyamine; N-oxide; hydrazine; hydrazide; hydrazone; azide; isocyanate; isothiocyanate; cyanate; thiocyanate; oxygen (=O); B(OH)₂, O(alkyl)aminocarbonyl; cycloalkyl, which may be monocyclic or fused or non-fused polycyclic (*e.g.,* cyclopropyl, cyclobutyl, cyclopentyl, or cyclohexyl), or a heterocyclyl, which may be monocyclic or fused or non-fused polycyclic (*e.g.,* pyrrolidyl, piperidyl, piperazinyl, morpholinyl, or thiazinyl); monocyclic or fused or non-fused polycyclic aryl or heteroaryl (*e.g.,* phenyl, naphthyl, pyrrolyl, indolyl, furanyl, thiophenyl, imidazolyl, oxazolyl, isoxazolyl, thiazolyl, triazolyl, tetrazolyl, pyrazolyl, pyridinyl, quinolinyl, isoquinolinyl, acridinyl, pyrazinyl, pyridazinyl, pyrimidinyl, benzimidazolyl, benzothiophenyl, or benzofuranyl) aryloxy; aralkyloxy; heterocyclyloxy; and heterocyclyl alkoxy.

As used herein, the term "pharmaceutically acceptable salt(s)" refers to a salt prepared from a pharmaceutically acceptable non-toxic acid or base including an inorganic acid and base and an organic acid and base. Suitable pharmaceutically acceptable base addition salts of Compound I include, but are not limited to metallic salts made from aluminum, calcium, lithium, magnesium, potassium, sodium and zinc or organic salts made from lysine, N,N'-dibenzylethylenediamine, chloroprocaine, choline, diethanolamine, ethylenediamine, meglumine (N-methylglucamine) and procaine. Suitable non-toxic acids include, but are not limited to, inorganic and organic acids such as acetic, alginic, anthranilic, benzenesulfonic, benzoic, camphorsulfonic, citric, ethenesulfonic, formic, fumaric, furoic, galacturonic, gluconic, glucuronic, glutamic, glycolic, hydrobromic, hydrochloric, isethionic, lactic, maleic, malic, mandelic, methanesulfonic, mucic, nitric, pamoic, pantothenic, phenylacetic, phosphoric, propionic, salicylic, stearic, succinic, sulfanilic, sulfuric, tartaric acid, and p-toluenesulfonic acid. Specific non-toxic acids include hydrochloric, hydrobromic, phosphoric, sulfuric, and methanesulfonic acids. Examples of specific salts thus include hydrochloride and mesylate salts. Others are well-known in the art, see for example, Remington's Pharmaceutical Sciences, 18th eds., Mack Publishing, Easton PA (1990) or Remington: The Science and Practice of Pharmacy, 19th eds., Mack Publishing, Easton PA (1995).

As used herein and unless otherwise indicated, the term "clathrate" means a TOR kinase inhibitor, or a salt thereof, in the form of a crystal lattice that contains spaces (*e.g.,* channels) that have a guest molecule (*e.g.,* a solvent or water) trapped within or a crystal lattice wherein a TOR kinase inhibitor is a guest molecule.

As used herein and unless otherwise indicated, the term "solvate" means a TOR kinase inhibitor, or a salt thereof, that further includes a stoichiometric or non-stoichiometric amount of a solvent bound by non-covalent intermolecular forces. As described herein, the solvate may be a hydrate.

As used herein and unless otherwise indicated, the term "hydrate" means a TOR kinase inhibitor, or a salt thereof, that further includes a stoichiometric or non-stoichiometric amount of water bound by non-covalent intermolecular forces.

As used herein and unless otherwise indicated, the term "prodrug" means a TOR kinase inhibitor derivative that can hydrolyze, oxidize, or otherwise react under biological conditions (*in vitro* or *in vivo*) to provide an active compound, particularly a TOR kinase inhibitor. Examples of prodrugs include, but are not limited to, derivatives and metabolites of a TOR kinase inhibitor that include biohydrolyzable moieties such as biohydrolyzable amides, biohydrolyzable esters, biohydrolyzable carbamates, biohydrolyzable carbonates, biohydrolyzable ureides, and biohydrolyzable phosphate analogues. Produgs of compounds with carboxyl functional groups may be the lower alkyl esters of the carboxylic acid. The carboxylate esters are conveniently formed by esterifying any of the carboxylic acid moieties present on the molecule. Prodrugs can typically be prepared using well-known methods, such as those described by Burger's Medicinal Chemistry and Drug Discovery 6th ed. (Donald J. Abraham ed., 2001, Wiley) and Design and Application of Prodrugs (H. Bundgaard ed., 1985, Harwood Academic Publishers Gmfh).

As used herein and unless otherwise indicated, the term "stereoisomer" or "stereomerically pure" means one stereoisomer of Compound I that is substantially free of other stereoisomers of that compound. For example, a stereomerically pure compound having one chiral center will be substantially free of the opposite enantiomer of the compound. A stereomerically pure compound having two chiral centers will be substantially free of other diastereomers of the compound. A typical stereomerically pure compound comprises greater than about 80% by weight of one stereoisomer of the compound and less than about 20% by weight of other stereoisomers of the compound, greater than about 90% by weight of one stereoisomer of the compound and less than about 10% by weight of the other stereoisomers of the compound, greater than about 95% by weight of one stereoisomer of the compound and less than about 5% by weight of the other stereoisomers of the compound, or greater than about 97% by weight of one stereoisomer of the compound and less than about 3% by weight of the other stereoisomers of the compound. Compound I can have chiral centers and can occur as racemates, individual enantiomers or diastereomers, and mixtures thereof. All such isomeric forms are included within the embodiments disclosed herein, including mixtures thereof. The use of stereomerically pure forms of such Compound I, as well as the use of mixtures of those forms are encompassed by the embodiments disclosed herein. For example, mixtures comprising equal or unequal amountsv of the enantiomers of Compound I may be used in the invention and compositions disclosed herein. These isomers may be asymmetrically synthesized or resolved using standard techniques such as chiral columns or chiral resolving agents. *See, e.g.,* Jacques, J., et al., Enantiomers, Racemates and Resolutions (Wiley-Interscience, New York, 1981); Wilen, S. H., et al., Tetrahedron 33:2725 (1977); Eliel, E. L., Stereochemistry of Carbon Compounds (McGraw-Hill, NY, 1962); and Wilen, S. H., Tables of Resolving Agents and Optical Resolutions p. 268 (E.L. Eliel, Ed., Univ. of Notre Dame Press, Notre Dame, IN, 1972).

It should also be noted Compound I can include E and Z isomers, or a mixture thereof, and cis and trans isomers or a mixture thereof. In certain embodiments, Compound I are isolated as either the cis or trans isomer. In other embodiments, Compound I are a mixture of the cis and trans isomers.

"Tautomers" refers to isomeric forms of a compound that are in equilibrium with each other. The concentrations of the isomeric forms will depend on the environment the compound is found in and may be different depending upon, for example, whether the compound is a solid or is in an organic or aqueous solution. For example, in aqueous solution, pyrazoles may exhibit the following isomeric forms, which are referred to as tautomers of each other:

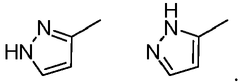

As readily understood by one skilled in the art, a wide variety of functional groups and other stuctures may exhibit tautomerism and all tautomers of Compound I are within the scope of the present invention.

It should also be noted Compound I can contain unnatural proportions of atomic isotopes at one or more of the atoms. For example, the compounds may be radiolabeled with radioactive isotopes, such as for example tritium (³H), iodine-125 (¹²⁵I), sulfur-35 (³⁵S), or carbon-14 (¹⁴C), or may be isotopically enriched, such as with deuterium (²H), carbon-13 (¹³C), or nitrogen-15 (¹⁵N). As used herein, an "isotopologue" is an isotopically enriched compound. The term "isotopically enriched" refers to an atom having an isotopic composition other than the natural isotopic composition of that atom. "Isotopically enriched" may also refer to a compound containing at least one atom having an isotopic composition other than the natural isotopic composition of that atom. The term "isotopic composition" refers to the amount of each isotope present for a given atom. Radiolabeled and isotopically encriched compounds are useful as therapeutic agents, e.g., cancer and inflammation therapeutic agents, research reagents, e.g., binding assay reagents, and diagnostic agents, e.g., in vivo imaging agents. All isotopic variations of Compound I as described herein, whether radioactive or not, are intended to be encompassed within the scope of the embodiments provided herein. In some embodiments, there are provided isotopologues of Compound I, for example, the isotopologues are deuterium, carbon-13, or nitrogen-15 enriched Compound I.

"Treating" as used herein, means an alleviation, in whole or in part, of ETS overexpressing Ewings sarcoma, or a symptom thereof, or slowing, or halting of further progression or worsening of ETS overexpressing Ewings sarcoma or a symptom thereof.

"Preventing" as used herein, means the prevention of the onset, recurrence or spread, in whole or in part, of ETS overexpressing Ewings sarcoma, or a symptom thereof.

The term "effective amount" in connection with Compound I means an amount capable of alleviating, in whole or in part, symptoms associated with ETS overexpressing Ewings sarcoma, or slowing or halting further progression or worsening of those symptoms, or treating or preventing ETS overexpressing Ewings sarcoma. The effective amount of Compound I, for example in a pharmaceutical composition, may be at a level that will exercise the desired effect; for example, about 0.005 mg/kg of a subject's body weight to about 100 mg/kg of a patient's body weight in unit dosage for both oral and parenteral administration. As will be apparent to those skilled in the art, it is to be expected that the effective amount of the TOR kinase inhibitor disclosed herein may vary depending on the severity of the indication being treated.

The terms "patient" and "subject" as used herein include an animal, including, but not limited to, an animal such as a cow, monkey, horse, sheep, pig, chicken, turkey, quail, cat, dog, mouse, rat, rabbit or guinea pig, in one embodiment a mammal, in another embodiment a human. In one embodiment, a "patient" or "subject" is a human having ETS overexpressing Ewings sarcoma. In one embodiment, a patient is a human having histologically or cytologically-confirmed ETS overexpressing Ewings sarcoma, including subjects who have progressed on (or not been able to tolerate) standard anticancer therapy or for whom no standard anticancer therapy exists.

In the context of ETS overexpressing Ewings sarcoma, treatment may be assessed by inhibition of disease progression, inhibition of tumor growth, reduction of primary and/or secondary tumor(s), relief of tumor-related symptoms, improvement in quality of life, inhibition of tumor secreted factors (including tumor secreted hormones, such as those that contribute to carcinoid syndrome), delayed appearance of primary and/or secondary tumor(s), slowed development of primary and/or secondary tumor(s), decreased occurrence of primary and/or secondary tumor(s), slowed or decreased severity of secondary effects of disease, arrested tumor growth and/or regression of tumors, among others. In certain embodiments, treatment of ETS overexpressing Ewings sarcoma may be assessed by the inhibition of phosphorylation of S6RP, 4E-BP1 and/or AKT in circulating blood and/or tumor cells and/or skin biopsies or tumor biopsies/aspirates, before, during, and/or after treatment with a TOR kinase inhibitor. In other embodiments, treatment of ETS overexpressing Ewings sarcoma may be assessed by the inhibition of DNA-dependent protein kinase (DNA-PK) activity in skin samples and/or tumor biopsies/aspirates, such as by assessment of the amount of pDNA-PK S2056 as a biomarker for DNA damage pathways before, during and/or after TOR kinase inhibitor treatment. In one embodiment, the skin sample is irradiated by UV light. In the extreme, complete inhibition, is referred to herein as prevention or chemoprevention. In this context, the term "prevention" includes either preventing the onset of clinically evident ETS overexpressing Ewings sarcoma altogether or preventing the onset of a preclinically evident stage of ETS overexpressing Ewings sarcoma. Also intended to be encompassed by this definition is the prevention of transformation into malignant cells or to arrest or reverse the progression of premalignant cells to malignant cells. This includes prophylactic treatment of those at risk of developing ETS overexpressing Ewings sarcoma.

### 4.2 BRIEF DESCRIPTION OF THE DRAWINGS

FIG. 1 provides a clonogenic growth inhibition assay for Compound 1 with ETS positive and ETS negative cell lines.
FIG. 2 provides the potentiation of DNA damage by Compound 1 in ETS positive cells.

### 4.3 TOR KINASE INHIBITORS

Compound I provided herein is generally referred to as "TOR kinase inhibitor." TOR kinase inhibitors do not include rapamycin or rapamycin analogs (rapalogs).

As described herein, the TOR kinase inhibitors may include compounds having the following formula (I): 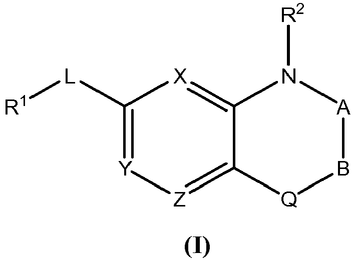 and pharmaceutically acceptable salts, clathrates, solvates, stereoisomers, tautomers, and prodrugs thereof, wherein:
X, Y and Z are at each occurrence independently N or CR³, wherein at least one of X, Y and Z is N and at least one of X, Y and Z is CR³;
-A-B-Q- taken together form -CHR⁴C(O)NH-, -C(O)CHR⁴NH-, -C(O)NH-, -CH₂C(O)O-, -C(O)CH₂O-, -C(O)O- or C(O)NR³;
L is a direct bond, NH or O;
R¹ is H, substituted or unsubstituted C₁₋₈alkyl, substituted or unsubstituted C₂₋₈alkenyl, substituted or unsubstituted aryl, substituted or unsubstituted heteroaryl, substituted or unsubstituted cycloalkyl or substituted or unsubstituted heterocyclylalkyl;
R² is H, substituted or unsubstituted C₁₋₈alkyl, substituted or unsubstituted aryl, substituted or unsubstituted heteroaryl, substituted or unsubstituted cycloalkyl, or substituted or unsubstituted heterocyclylalkyl;
R³ is H, substituted or unsubstituted C₁₋₈alkyl, substituted or unsubstituted aryl, substituted or unsubstituted heteroaryl, substituted or unsubstituted cycloalkyl, substituted or unsubstituted heterocyclylalkyl, -NHR⁴ or -N(R⁴)₂; and
R⁴ is at each occurrence independently substituted or unsubstituted C₁₋₈alkyl, substituted or unsubstituted aryl, substituted or unsubstituted heteroaryl, substituted or unsubstituted cycloalkyl, or substituted or unsubstituted heterocyclylalkyl.

The TOR kinase inhibitors of formula (I) may be those wherein -A-B-Q- taken together form -CH₂C(O)NH-.

The TOR kinase inhibitors of formula (I) may be those wherein -A-B-Q- taken together form -C(O)CH₂NH-.

The TOR kinase inhibitors of formula (I) may be those wherein -A-B-Q- taken together form -C(O)NH-.

The TOR kinase inhibitors of formula (I) may be those wherein -A-B-Q- taken together form -CH₂C(O)O-.

The TOR kinase inhibitors of formula (I) may be those wherein -A-B-Q- taken together form -C(O)CH₂O-.

The TOR kinase inhibitors of formula (I) may be those wherein -A-B-Q- taken together form -C(O)O-.

The TOR kinase inhibitors of formula (I) may be those wherein -A-B-Q- taken together form -C(O)NR³-.

The TOR kinase inhibitors of formula (I) may be those wherein Y is CR³.

The TOR kinase inhibitors of formula (I) may be those wherein X and Z are N and Y is CR³.

The TOR kinase inhibitors of formula (I) may be those wherein X and Z are N and Y is CH.

The TOR kinase inhibitors of formula (I) may be those wherein X and Z are CH and Y is N.

The TOR kinase inhibitors of formula (I) may be those wherein Y and Z are CH and X is N.

The TOR kinase inhibitors of formula (I) may be those wherein X and Y are CH and Z is N.

The TOR kinase inhibitors of formula (I) may be those wherein R¹ is substituted aryl, such as substituted phenyl.

The TOR kinase inhibitors of formula (I) may be those wherein R¹ is substituted or unsubstituted aryl, such as substituted or unsubstituted phenyl or substituted or unsubstituted naphthyl.

The TOR kinase inhibitors of formula (I) may be those wherein R¹ is substituted or unsubstituted heteroaryl, such as substituted or unsubstituted quinoline, substituted or unsubstituted pyridine, substituted or unsubstituted pyrimidine, substituted or unsubstituted indole, or substituted or unsubstituted thiophene.

The TOR kinase inhibitors of formula (I) may be those wherein R¹ is H.

The TOR kinase inhibitors of formula (I) may be those wherein R² is substituted C₁₋₈alkyl.

The TOR kinase inhibitors of formula (I) may be those wherein R² is methyl or ethyl substituted with substituted or unsubstituted aryl, substituted or unsubstituted heteroaryl, substituted or unsubstituted cycloalkyl, or substituted or unsubstituted heterocyclylalkyl.

The TOR kinase inhibitors of formula (I) may be those wherein R² is substituted or unsubstituted cycloalkyl or substituted or unsubstituted heterocyclylalkyl.

The TOR kinase inhibitors of formula (I) may be those wherein R² is substituted or unsubstituted aryl, such as substituted or unsubstituted phenyl.

The TOR kinase inhibitors of formula (I) may be those wherein R² is H.

The TOR kinase inhibitors of formula (I) may be those wherein L is a direct bond.

The TOR kinase inhibitors of formula (I) may be those wherein -A-B-Q- taken together form -C(O)NH-, X and Z are N and Y is CH, R¹ is substituted or unsubstituted aryl or substituted or unsubstituted heteroaryl, L is a direct bond, and R² is substituted or unsubstituted C₁₋₈alkyl.

The TOR kinase inhibitors of formula (I) may be those wherein -A-B-Q- taken together form -C(O)NH-, X and Z are N and Y is CH, R¹ is substituted or unsubstituted aryl, L is a direct bond, and R² is substituted or unsubstituted C₁₋₈alkyl.

The TOR kinase inhibitors of formula (I) may be those wherein -A-B-Q- taken together form -C(O)NH-, X and Z are N and Y is CH, R¹ is substituted or unsubstituted aryl, and R² is C₁₋₈alkyl substituted with one or more substituents selected from alkoxy, amino, hydroxy, cycloalkyl, or heterocyclylalkyl.

The TOR kinase inhibitors of formula (I) may be those wherein -A-B-Q- taken together form -C(O)NH-, X and Z are N and Y is CH, R¹ is substituted or unsubstituted aryl, and R² is substituted or unsubstituted cycloalkyl, or substituted or unsubstituted heterocyclylalkyl.

The TOR kinase inhibitors of formula (I) may be those wherein -A-B-Q- taken together form -C(O)NH-, X and Z are N and Y is CH, R¹ is substituted phenyl, L is a direct bond, and R² is substituted C₁₋₈alkyl.

The TOR kinase inhibitors of formula (I) may not include compounds wherein X and Z are both N and Y is CH, -A-B-Q- is -C(O)NH-, L is a direct bond, R¹ is substituted or unsubstituted aryl or substituted or unsubstituted heteroaryl, and R² is C₁₋₈alkyl substituted with substituted or unsubstituted aryl or substituted or unsubstituted heteroaryl.

The TOR kinase inhibitors of formula (I) may not include compounds wherein X and Z are both N and Y is CH, -A-B-Q- is -C(O)NH-, L is a direct bond, R¹ is phenyl, naphthyl, indanyl or biphenyl, each of which may be optionally substituted with one or more substituents independently selected from the group consisting substituted or unsubstituted C₁₋₈alkyl, substituted or unsubstituted C₂₋₈alkenyl, substituted or unsubstituted aryl, substituted or unsubstituted cycloalkyl or substituted or unsubstituted heterocyclylalkyl.

The TOR kinase inhibitors of formula (I) may not include compounds wherein X and Z are both N and Y is CH, -A-B-Q- is -C(O)NH-, L is a direct bond, R¹ is phenyl, naphthyl or biphenyl, each of which may be optionally substituted with one or more substituents each independently selected from the group consisting of C₁₋₄alkyl, amino, aminoC₁₋₁₂alkyl, halogen, hydroxy, hydroxyC₁₋₄alkyl, C₁₋₄alkyloxyC₁₋₄alkyl, -CF₃, C₁₋₁₂alkoxy, aryloxy, arylC₁₋₁₂alkoxy, -CN, -OCF₃, -COR_{g}, -COOR_{g}, -CONR_{g}Rₕ, -NR_{g}CORₕ, -SO₂R_{g}, -SO₃R_{g} or -SO₂NR_{g}Rₕ, wherein each R_{g} and Rₕ are independently selected from the group consisting of hydrogen, C₁₋₄alkyl, C₃₋₆cycloalkyl, aryl, arylC₁₋₆alkyl, heteroaryl or heteroarylC₁₋₆alkyl; or A is a 5- to 6-membered monocyclic heteroaromatic ring having from one, two, three or four heteroatoms independently selected from the group consisting of N, O and S, that monocyclic heteroaromatic ring may be optionally substituted with one or more substituents each independently selected from the group consisting of C₁₋₆alkyl, amino, aminoC₁₋₁₂alkyl, halogen, hydroxy, hydroxyC₁₋₄alkyl, C₁₋₄alkyloxyC₁₋₄alkyl, C₁₋₁₂alkoxy, aryloxy, aryl C₁₋₁₂alkoxy, -CN, -CF₃, -OCF₃, -CORᵢ, -COORᵢ, -CONRᵢRⱼ, -NRᵢCORⱼ, -NRᵢSO₂Rⱼ, -SO₂Rᵢ, -SO₃Rᵢ or -SO₂NRᵢRⱼ, wherein each Rᵢ and Rⱼ are independently selected from the group consisting of hydrogen, C₁₋₄ alkyl, C₃₋₆cycloalkyl, aryl, arylC₁₋₆alkyl, heteroaryl or heteroarylC₁₋₆alkyl; or A is a 8- to 10 membered bicyclic heteroaromatic ring from one, two, three or four heteroatoms selected from the group consisting of N, O and S, and may be optionally substituted with one, two or three substituents each independently selected from the group consisting of C₁₋₆alkyl, amino, aminoC₁₋₁₂alkyl, halogen, hydroxy, hydroxyC₁₋₄alkyl, C₁₋₄alkyloxyC₁₋₄alkyl, C₁₋₁₂alkoxy, aryloxy, aryl C₁₋₁₂alkoxy, -CN, -CF₃, -OCF₃, -CORₖ, -COORₖ, -CONRₖRₗ, -NRₖCORₗ, -NRₖSO₂Rₗ, -SO₂Rₖ, -SO₃Rₖ or -SO₂NRₖRₗ, wherein each Rₖ and Rₗ are independently selected from the group consisting of hydrogen, C₁₋₄ alkyl, C₃₋₆ cycloalkyl, aryl, arylC₁₋₆alkyl, heteroaryl or heteroarylC₁₋₆alkyl, and R² is C₁₋₈alkyl substituted with substituted or unsubstituted aryl or substituted or unsubstituted heteroaryl.

The TOR kinase inhibitors of formula (I) may not include compounds wherein X and Y are both N and Z is CH, -A-B-Q- is -C(O)NH-, L is a direct bond, R¹ is substituted or unsubstituted phenyl or substituted or unsubstituted heteroaryl, and R² is substituted or unsubstituted methyl, unsubstituted ethyl, unsubstituted propyl, or an acetamide.

The TOR kinase inhibitors of formula (I) may not include compounds wherein X and Y are both N and Z is CH, -A-B-Q- is -C(O)NH-, L is a direct bond, R¹ is substituted or unsubstituted phenyl or substituted or unsubstituted heteroaryl, and R² is an acetamide.

The TOR kinase inhibitors of formula (I) may not include compounds wherein X is N and Y and Z are both CH, -A-B-Q- is -C(O)NH-, L is a direct bond, R¹ is a (2,5'-Bi-1H-benzimidazole)-5-carboxamide, and R² is H.

The TOR kinase inhibitors of formula (I) may not include compounds wherein one of X and Z is CH and the other is N, Y is CH, -A-B-Q- is -C(O)NH-, L is a direct bond, R¹ is unsubstituted pyridine, and R² is H, methyl or substituted ethyl.

The TOR kinase inhibitors of formula (I) may not include compounds wherein X and Z are both N and Y is CH, -A-B-Q- is -C(O)NH-, R¹ is H, C₁₋₈alkyl, C₂₋₈alkenyl, aryl or cycloalkyl, and L is NH.

The TOR kinase inhibitors of formula (I) may not include compounds wherein X and Z are both N and Y is CH, -A-B-Q- is -C(O)NR³-, R² is H, substituted or unsubstituted C₁₋₈alkyl, substituted or unsubstituted phenyl, substituted or unsubstituted cycloalkyl, or substituted or unsubstituted heterocyclylalkyl, and L is NH.

The TOR kinase inhibitors of formula (I) may not include compounds wherein R¹ is a substituted or unsubstituted oxazolidinone.

The TOR kinase inhibitors of formula (I) may not include one or more of the following compounds: 1,7-dihydro-2-phenyl-8H-Purin-8-one, 1,2-dihydro-3-phenyl-6H-Imidazo[4,5-e]-1,2,4-triazin-6-one, 1,3-dihydro-6-(4-pyridinyl)-2H-Imidazo[4,5-b]pyridin-2-one, 6-(1,3-benzodioxol-5-yl)-1,3-dihydro-1-[(1S)-1-phenylethyl]- 2H-Imidazo[4,5-b]pyrazin-2-one, 3-[2,3-dihydro-2-oxo-3-(4-pyridinylmethyl)-1H-imidazo[4,5-b]pyrazin-5-yl]-Benzamide, 1-[2-(dimethylamino)ethyl]-1,3-dihydro-6-(3,4,5-trimethoxyphenyl)-2H-Imidazo[4,5-b]pyrazin-2-one, N-[5-(1,1-dimethylethyl)-2-methoxyphenyl]-N'-[4-(1,2,3,4-tetrahydro-2-oxopyrido[2,3-b]pyrazin-7-yl)-1-naphthalenyl]-Urea, N-[4-(2,3-dihydro-2-oxo-1H-imidazo[4,5-b]pyridin-6-yl)-1-naphthalenyl]-N'-[5-(1,1-dimethylethyl)-2-methoxyphenyl]-Urea, 1,3 -dihydro-5 -phenyl-2H-Imidazo[4,5-b]pyrazin-2-one, 1,3-dihydro-5-phenoxy-2H-Imidazo[4,5-b]pyridin-2-one, 1,3-dihydro-1-methyl-6-phenyl-2H-Imidazo[4,5-b]pyridin-2-one, 1,3-dihydro-5-(1H-imidazol-1-yl) 2H-Imidazo[4,5-b]pyridin-2-one, 6-(2,3-dihydro-2-oxo-1H-imidazo[4,5-b]pyridin-6-yl)-8-methyl-2(1H)-Quinolinone and 7,8-dihydro-8-oxo-2-phenyl-9H-purine-9-acetic acid.

As also described herein, the TOR kinase inhibitors may include compounds having the following formula (Ia): 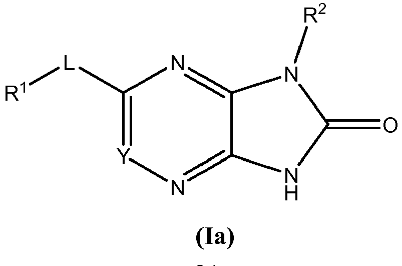 and pharmaceutically acceptable salts, clathrates, solvates, stereoisomers, tautomers, and prodrugs thereof, wherein:
L is a direct bond, NH or O;
Y is N or CR³;
R¹ is H, substituted or unsubstituted C₁₋₈alkyl, substituted or unsubstituted C₂₋₈alkenyl, substituted or unsubstituted aryl, substituted or unsubstituted heteroaryl, substituted or unsubstituted cycloalkyl or substituted or unsubstituted heterocyclylalkyl;
R² is H, substituted or unsubstituted C₁₋₈alkyl, substituted or unsubstituted aryl, substituted or unsubstituted heteroaryl, substituted or unsubstituted cycloalkyl, or substituted or unsubstituted heterocyclylalkyl;
R³ is H, substituted or unsubstituted C₁₋₈alkyl, substituted or unsubstituted aryl, substituted or unsubstituted heteroaryl, substituted or unsubstituted cycloalkyl, substituted or unsubstituted heterocyclylalkyl, -NHR⁴ or -N(R⁴)₂; and
R⁴ is at each occurrence independently substituted or unsubstituted C₁₋₈alkyl, substituted or unsubstituted aryl, substituted or unsubstituted heteroaryl, substituted or unsubstituted cycloalkyl, or substituted or unsubstituted heterocyclylalkyl.

The TOR kinase inhibitors of formula (Ia) may be those wherein R¹ is substituted aryl, such as substituted phenyl.

The TOR kinase inhibitors of formula (Ia) may be those wherein R¹ is substituted or unsubstituted aryl, such as substituted or unsubstituted phenyl or substituted or unsubstituted naphthyl.

The TOR kinase inhibitors of formula (Ia) may be those wherein R¹ is substituted or unsubstituted heteroaryl, such as substituted or unsubstituted quinoline, substituted or unsubstituted pyridine, substituted or unsubstituted pyrimidine, substituted or unsubstituted indole, or substituted or unsubstituted thiophene.

The TOR kinase inhibitors of formula (Ia) may be those wherein R¹ is H.

The TOR kinase inhibitors of formula (Ia) may be those wherein R² is substituted C₁₋₈alkyl.

The TOR kinase inhibitors of formula (Ia) may be those wherein R² is methyl or ethyl substituted with substituted or unsubstituted aryl, substituted or unsubstituted heteroaryl, substituted or unsubstituted cycloalkyl, or substituted or unsubstituted heterocyclylalkyl.

The TOR kinase inhibitors of formula (Ia) may be those wherein R² is substituted or unsubstituted cycloalkyl or substituted or unsubstituted heterocyclylalkyl.

The TOR kinase inhibitors of formula (Ia) may be those wherein R² is substituted or unsubstituted aryl, such as substituted or unsubstituted phenyl.

The TOR kinase inhibitors of formula (Ia) may be those wherein R² is H.

The TOR kinase inhibitors of formula (Ia) may be those wherein Y is CH.

The TOR kinase inhibitors of formula (Ia) may be those wherein L is a direct bond.

The TOR kinase inhibitors of formula (Ia) may be those wherein R¹ is substituted or unsubstituted aryl and R² is unsubstituted C₁₋₈alkyl.

The TOR kinase inhibitors of formula (Ia) may be those wherein R¹ is substituted or unsubstituted aryl and R² is C₁₋₈alkyl substituted with one or more substituents selected from alkoxy, amino, hydroxy, cycloalkyl, or heterocyclylalkyl.

The TOR kinase inhibitors of formula (Ia) may be those wherein R¹ is substituted or unsubstituted aryl and R² is substituted or unsubstituted cycloalkyl, or substituted or unsubstituted heterocyclylalkyl.

The TOR kinase inhibitors of formula (Ia) may not include compounds wherein Y is CH, L is a direct bond, R¹ is substituted or unsubstituted aryl or substituted or unsubstituted heteroaryl, and R² is C₁₋₈alkyl substituted with substituted or unsubstituted aryl or substituted or unsubstituted heteroaryl.

As also described herein, the TOR kinase inhibitors may include compounds having the following formula (Ib): 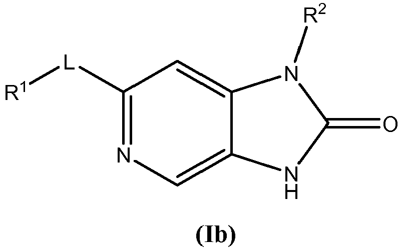 and pharmaceutically acceptable salts, clathrates, solvates, stereoisomers, tautomers, and prodrugs thereof, wherein:
L is a direct bond, NH or O;
R¹ is H, substituted or unsubstituted C₁₋₈alkyl, substituted or unsubstituted C₂₋₈alkenyl, substituted or unsubstituted aryl, substituted or unsubstituted heteroaryl, substituted or unsubstituted cycloalkyl or substituted or unsubstituted heterocyclylalkyl; and
R² is H, substituted or unsubstituted C₁₋₈alkyl, substituted or unsubstituted aryl, substituted or unsubstituted heteroaryl, substituted or unsubstituted cycloalkyl, or substituted or unsubstituted heterocyclylalkyl.

The TOR kinase inhibitors of formula (Ib) may be those wherein R¹ is substituted aryl, such as substituted phenyl.

The TOR kinase inhibitors of formula (Ib) may be those wherein R¹ is substituted or unsubstituted aryl, such as substituted or unsubstituted phenyl or substituted or unsubstituted naphthyl.

The TOR kinase inhibitors of formula (Ib) may be those wherein R¹ is substituted or unsubstituted heteroaryl, such as substituted or unsubstituted quinoline, substituted or unsubstituted pyridine, substituted or unsubstituted pyrimidine, substituted or unsubstituted indole, or substituted or unsubstituted thiophene.

The TOR kinase inhibitors of formula (Ib) may be those wherein R¹ is H.

The TOR kinase inhibitors of formula (Ib) may be those wherein R² is substituted C₁₋₈alkyl.

The TOR kinase inhibitors of formula (Ib) may be those wherein R² is methyl or ethyl substituted with substituted or unsubstituted aryl, substituted or unsubstituted heteroaryl, substituted or unsubstituted cycloalkyl, or substituted or unsubstituted heterocyclylalkyl.

The TOR kinase inhibitors of formula (Ib) may be those wherein R² is substituted or unsubstituted cycloalkyl or substituted or unsubstituted heterocyclylalkyl.

The TOR kinase inhibitors of formula (Ib) may be those wherein R² is substituted or unsubstituted aryl, such as substituted or unsubstituted phenyl.

The TOR kinase inhibitors of formula (Ib) may be those wherein R² is H.

The TOR kinase inhibitors of formula (Ib) may be those wherein L is a direct bond.

The TOR kinase inhibitors of formula (Ib) may be those wherein R¹ is substituted or unsubstituted aryl and R² is unsubstituted C₁₋₈alkyl.

The TOR kinase inhibitors of formula (Ib) may be those wherein R¹ is substituted or unsubstituted aryl and R² is C₁₋₈alkyl substituted with one or more substituents selected from alkoxy, amino, hydroxy, cycloalkyl, or heterocyclylalkyl.

The TOR kinase inhibitors of formula (Ib) may be those wherein R¹ is substituted or unsubstituted aryl and R² is substituted or unsubstituted cycloalkyl, or substituted or unsubstituted heterocyclylalkyl.

The TOR kinase inhibitors may include compounds having the following formula (Ic): 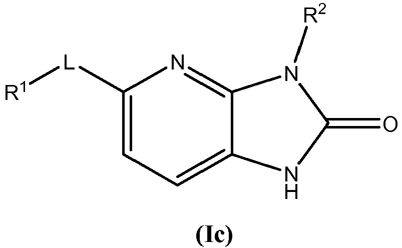 and pharmaceutically acceptable salts, clathrates, solvates, stereoisomers, tautomers, and prodrugs thereof, wherein:
L is a direct bond, NH or O;
R¹ is H, substituted or unsubstituted C₁₋₈alkyl, substituted or unsubstituted C₂₋₈alkenyl, substituted or unsubstituted aryl, substituted or unsubstituted heteroaryl, substituted or unsubstituted cycloalkyl or substituted or unsubstituted heterocyclylalkyl; and
R² is H, substituted or unsubstituted C₁₋₈alkyl, substituted or unsubstituted aryl, substituted or unsubstituted heteroaryl, substituted or unsubstituted cycloalkyl, or substituted or unsubstituted heterocyclylalkyl.

The TOR kinase inhibitors of formula (Ic) may be those wherein R¹ is substituted aryl, such as substituted phenyl.

The TOR kinase inhibitors of formula (Ic) may be those wherein R¹ is substituted or unsubstituted aryl, such as substituted or unsubstituted phenyl or substituted or unsubstituted naphthyl.

The TOR kinase inhibitors of formula (Ic) may be those wherein R¹ is substituted or unsubstituted heteroaryl, such as substituted or unsubstituted quinoline, substituted or unsubstituted pyridine, substituted or unsubstituted pyrimidine, substituted or unsubstituted indole, or substituted or unsubstituted thiophene.

The TOR kinase inhibitors of formula (Ic) may be those wherein R¹ is H.

The TOR kinase inhibitors of formula (Ic) may be those wherein R² is substituted C₁₋₈alkyl.

The TOR kinase inhibitors of formula (Ic) may be those wherein R² is methyl or ethyl substituted with substituted or unsubstituted aryl, substituted or unsubstituted heteroaryl, substituted or unsubstituted cycloalkyl, or substituted or unsubstituted heterocyclylalkyl.

The TOR kinase inhibitors of formula (Ic) may be those wherein R² is substituted or unsubstituted cycloalkyl or substituted or unsubstituted heterocyclylalkyl.

The TOR kinase inhibitors of formula (Ic) may be those wherein R² is substituted or unsubstituted aryl, such as substituted or unsubstituted phenyl.

The TOR kinase inhibitors of formula (Ic) are those wherein R² is H.

The TOR kinase inhibitors of formula (Ic) may be those wherein L is a direct bond.

The TOR kinase inhibitors of formula (Ic) may be those wherein R¹ is substituted or unsubstituted aryl and R² is unsubstituted C₁₋₈alkyl.

The TOR kinase inhibitors of formula (Ic) may be those wherein R¹ is substituted or unsubstituted aryl and R² is C₁₋₈alkyl substituted with one or more substituents selected from alkoxy, amino, hydroxy, cycloalkyl, or heterocyclylalkyl.

The TOR kinase inhibitors of formula (Ic) may be those wherein R¹ is substituted or unsubstituted aryl and R² is substituted or unsubstituted cycloalkyl, or substituted or unsubstituted heterocyclylalkyl.

As also described herein, the TOR kinase inhibitors may include compounds having the following formula (Id): 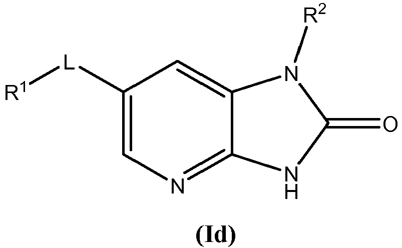 and pharmaceutically acceptable salts, clathrates, solvates, stereoisomers, tautomers, and prodrugs thereof, wherein:
L is a direct bond, NH or O;
R¹ is H, substituted or unsubstituted C₁₋₈alkyl, substituted or unsubstituted C₂₋₈alkenyl, substituted or unsubstituted aryl, substituted or unsubstituted heteroaryl, substituted or unsubstituted cycloalkyl or substituted or unsubstituted heterocyclylalkyl; and
R² is H, substituted or unsubstituted C₁₋₈alkyl, substituted or unsubstituted aryl, substituted or unsubstituted heteroaryl, substituted or unsubstituted cycloalkyl, or substituted or unsubstituted heterocyclylalkyl.

The TOR kinase inhibitors of formula (Id) may be those wherein R¹ is substituted aryl, such as substituted phenyl.

The TOR kinase inhibitors of formula (Id) may be those wherein R¹ is substituted or unsubstituted aryl, such as substituted or unsubstituted phenyl or substituted or unsubstituted naphthyl.

The TOR kinase inhibitors of formula (Id) may be those wherein R¹ is substituted or unsubstituted heteroaryl, such as substituted or unsubstituted quinoline, substituted or unsubstituted pyridine, substituted or unsubstituted pyrimidine, substituted or unsubstituted indole, or substituted or unsubstituted thiophene.

The TOR kinase inhibitors of formula (Id) may be those wherein R¹ is H.

The TOR kinase inhibitors of formula (Id) may be those wherein R² is substituted C₁₋₈alkyl.

The TOR kinase inhibitors of formula (Id) may be those wherein R² is methyl or ethyl substituted with substituted or unsubstituted aryl, substituted or unsubstituted heteroaryl, substituted or unsubstituted cycloalkyl, or substituted or unsubstituted heterocyclylalkyl.

The TOR kinase inhibitors of formula (Id) may those wherein R² is substituted or unsubstituted cycloalkyl or substituted or unsubstituted heterocyclylalkyl.

The TOR kinase inhibitors of formula (Id) may be those wherein R² is substituted or unsubstituted aryl, such as substituted or unsubstituted phenyl.

The Heteroaryl Compounds of formula (Id) may be those wherein R² is H.

The TOR kinase inhibitors of formula (Id) may be those wherein L is a direct bond.

The TOR kinase inhibitors of formula (Id) may be those wherein R¹ is substituted or unsubstituted aryl and R² is unsubstituted C₁₋₈alkyl.

The TOR kinase inhibitors of formula (Id) may be those wherein R¹ is substituted or unsubstituted aryl and R² is C₁₋₈alkyl substituted with one or more substituents selected from alkoxy, amino, hydroxy, cycloalkyl, or heterocyclylalkyl.

The TOR kinase inhibitors of formula (Id) may be wherein R¹ is substituted or unsubstituted aryl and R² is substituted or unsubstituted cycloalkyl, or substituted or unsubstituted heterocyclylalkyl.

As also described herein, the TOR kinase inhibitors may include compounds having the following formula (Ie): 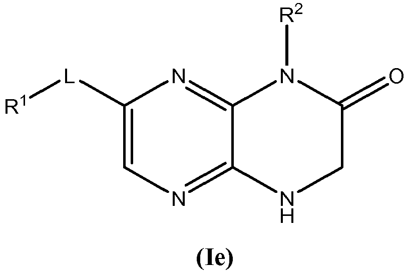 and pharmaceutically acceptable salts, clathrates, solvates, stereoisomers, tautomers, and prodrugs thereof, wherein:
L is a direct bond, NH or O;
R¹ is H, substituted or unsubstituted C₁₋₈alkyl, substituted or unsubstituted C₂₋₈alkenyl, substituted or unsubstituted aryl, substituted or unsubstituted heteroaryl, substituted or unsubstituted cycloalkyl or substituted or unsubstituted heterocyclylalkyl; and
R² is H, substituted or unsubstituted C₁₋₈alkyl, substituted or unsubstituted aryl, substituted or unsubstituted heteroaryl, substituted or unsubstituted cycloalkyl, or substituted or unsubstituted heterocyclylalkyl.

The TOR kinase inhibitors of formula (Ie) may be those wherein R¹ is substituted aryl, such as substituted phenyl.

The TOR kinase inhibitors of formula (Ie) may be those wherein R¹ is substituted or unsubstituted aryl, such as substituted or unsubstituted phenyl or substituted or unsubstituted naphthyl.

The TOR kinase inhibitors of formula (Ie) may be those wherein R¹ is substituted or unsubstituted heteroaryl, such as substituted or unsubstituted quinoline, substituted or unsubstituted pyridine, substituted or unsubstituted pyrimidine, substituted or unsubstituted indole, or substituted or unsubstituted thiophene.

The TOR kinase inhibitors of formula (Ie) may be those wherein R¹ is H.

The TOR kinase inhibitors of formula (Ie) may be those wherein R² is substituted C₁₋₈alkyl.

The TOR kinase inhibitors of formula (Ie) may be those wherein R² is methyl or ethyl substituted with substituted or unsubstituted aryl, substituted or unsubstituted heteroaryl, substituted or unsubstituted cycloalkyl, or substituted or unsubstituted heterocyclylalkyl.

The TOR kinase inhibitors of formula (Ie) may be those wherein R² is substituted or unsubstituted cycloalkyl or substituted or unsubstituted heterocyclylalkyl.

The TOR kinase inhibitors of formula (Ie) may be those wherein R² is substituted or unsubstituted aryl, such as substituted or unsubstituted phenyl.

The TOR kinase inhibitors of formula (Ie) may be those wherein R² is H.

The TOR kinase inhibitors of formula (Ie) may be those wherein L is a direct bond.

The TOR kinase inhibitors of formula (Ie) may be those wherein R¹ is substituted or unsubstituted aryl and R² is unsubstituted C₁₋₈alkyl.

The TOR kinase inhibitors of formula (Ie) may be those wherein R¹ is substituted or unsubstituted aryl and R² is C₁₋₈alkyl substituted with one or more substituents selected from alkoxy, amino, hydroxy, cycloalkyl, or heterocyclylalkyl.

The TOR kinase inhibitors of formula (Ie) may be those wherein R¹ is substituted or unsubstituted aryl and R² is substituted or unsubstituted cycloalkyl, or substituted or unsubstituted heterocyclylalkyl.

As also describe herein, the TOR kinase inhibitors may include compounds having the following formula (If): 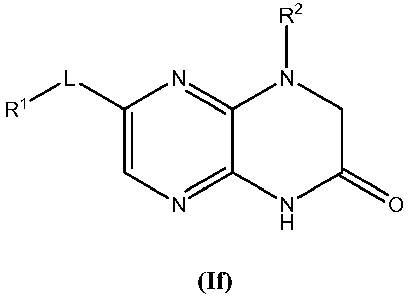 and pharmaceutically acceptable salts, clathrates, solvates, stereoisomers, tautomers, and prodrugs thereof, wherein:
L is a direct bond, NH or O;
R¹ is H, substituted or unsubstituted C₁₋₈alkyl, substituted or unsubstituted C₂₋₈alkenyl, substituted or unsubstituted aryl, substituted or unsubstituted heteroaryl, substituted or unsubstituted cycloalkyl or substituted or unsubstituted heterocyclylalkyl; and
R² is H, substituted or unsubstituted C₁₋₈alkyl, substituted or unsubstituted aryl, substituted or unsubstituted heteroaryl, substituted or unsubstituted cycloalkyl, or substituted or unsubstituted heterocyclylalkyl.

The TOR kinase inhibitors of formula (If) may be those wherein R¹ is substituted aryl, such as substituted phenyl.

The TOR kinase inhibitors of formula (If) may be those wherein R¹ is substituted or unsubstituted aryl, such as substituted or unsubstituted phenyl or substituted or unsubstituted naphthyl.

The TOR kinase inhibitors of formula (If) may be those wherein R¹ is substituted or unsubstituted heteroaryl, such as substituted or unsubstituted quinoline, substituted or unsubstituted pyridine, substituted or unsubstituted pyrimidine, substituted or unsubstituted indole, or substituted or unsubstituted thiophene.

The TOR kinase inhibitors of formula (If) may be those wherein R¹ is H.

The TOR kinase inhibitors of formula (If) may be those wherein R² is substituted C₁₋₈alkyl.

The TOR kinase inhibitors of formula (If) may be those wherein R² is methyl or ethyl substituted with substituted or unsubstituted aryl, substituted or unsubstituted heteroaryl, substituted or unsubstituted cycloalkyl, or substituted or unsubstituted heterocyclylalkyl.

The TOR kinase inhibitors of formula (If) may be those wherein R² is substituted or unsubstituted cycloalkyl or substituted or unsubstituted heterocyclylalkyl.

The TOR kinase inhibitors of formula (If) may be those wherein R² is substituted or unsubstituted aryl, such as substituted or unsubstituted phenyl.

The TOR kinase inhibitors of formula (If) may be those wherein R² is H.

The TOR kinase inhibitors of formula (If) may be those wherein L is a direct bond.

The TOR kinase inhibitors of formula (If) may be those wherein R¹ is substituted or unsubstituted aryl and R² is unsubstituted C₁₋₈alkyl.

The TOR kinase inhibitors of formula (If) may be those wherein R¹ is substituted or unsubstituted aryl and R² is C₁₋₈alkyl substituted with one or more substituents selected from alkoxy, amino, hydroxy, cycloalkyl, or heterocyclylalkyl.

The TOR kinase inhibitors of formula (If) may be those wherein R¹ is substituted or unsubstituted aryl and R² is substituted or unsubstituted cycloalkyl, or substituted or unsubstituted heterocyclylalkyl.

As also described herein, the TOR kinase inhibitors may include compounds having the following formula (Ig): 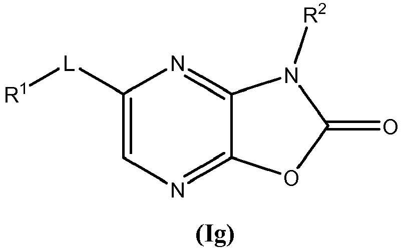 and pharmaceutically acceptable salts, clathrates, solvates, stereoisomers, tautomers, and prodrugs thereof, wherein:
L is a direct bond, NH or O;
R¹ is H, substituted or unsubstituted C₁₋₈alkyl, substituted or unsubstituted C₂₋₈alkenyl, substituted or unsubstituted aryl, substituted or unsubstituted heteroaryl, substituted or unsubstituted cycloalkyl or substituted or unsubstituted heterocyclylalkyl; and
R² is H, substituted or unsubstituted C₁₋₈alkyl, substituted or unsubstituted aryl, substituted or unsubstituted heteroaryl, substituted or unsubstituted cycloalkyl, or substituted or unsubstituted heterocyclylalkyl.

The TOR kinase inhibitors of formula (Ig) may be those wherein R¹ is substituted aryl, such as substituted phenyl.

The TOR kinase inhibitors of formula (Ig) may be those wherein R¹ is substituted or unsubstituted aryl, such as substituted or unsubstituted phenyl or substituted or unsubstituted naphthyl.

The TOR kinase inhibitors of formula (Ig) may be those wherein R¹ is substituted or unsubstituted heteroaryl, such as substituted or unsubstituted quinoline, substituted or unsubstituted pyridine, substituted or unsubstituted pyrimidine, substituted or unsubstituted indole, or substituted or unsubstituted thiophene.

The TOR kinase inhibitors of formula (Ig) may be those wherein R¹ is H.

The TOR kinase inhibitors of formula (Ig) may be those wherein R² is substituted C₁₋₈alkyl.

The TOR kinase inhibitors of formula (Ig) may be those wherein R² is methyl or ethyl substituted with substituted or unsubstituted aryl, substituted or unsubstituted heteroaryl, substituted or unsubstituted cycloalkyl, or substituted or unsubstituted heterocyclylalkyl.

The TOR kinase inhibitors of formula (Ig) may be those wherein R² is substituted or unsubstituted cycloalkyl or substituted or unsubstituted heterocyclylalkyl.

The TOR kinase inhibitors of formula (Ig) may be those wherein R² is substituted or unsubstituted aryl, such as substituted or unsubstituted phenyl.

The TOR kinase inhibitors of formula (Ig) may be those wherein R² is H.

The TOR kinase inhibitors of formula (Ig) may be those wherein L is a direct bond.

The TOR kinase inhibitors of formula (Ig) may be those wherein R¹ is substituted or unsubstituted aryl and R² is unsubstituted C₁₋₈alkyl.

The TOR kinase inhibitors of formula (Ig) may be those wherein R¹ is substituted or unsubstituted aryl and R² is C₁₋₈alkyl substituted with one or more substituents selected from alkoxy, amino, hydroxy, cycloalkyl, or heterocyclylalkyl.

The TOR kinase inhibitors of formula (Ig) may be those wherein R¹ is substituted or unsubstituted aryl and R² is substituted or unsubstituted cycloalkyl, or substituted or unsubstituted heterocyclylalkyl.

Representative TOR kinase inhibitors of formula (I) may include:
(S)-1 -(1 -hydroxy-3-methylbutan-2-yl)-6-phenyl-1H-imidazo[4,5-b]pyrazin-2(3H)-one;
1-((tetrahydro-2H-pyran-4-yl)methyl)-6-(3,4,5-trimethoxyphenyl)-1H-imidazo[4,5-b]pyrazin-2(3H)-one;
(R)-6-(naphthalen-1-yl)-1-(1-phenylethyl)-1H-imidazo[4,5-b]pyrazin-2(3H)-one;
1-(3-methoxybenzyl)-6-(4-(methylsulfonyl)phenyl)-1H-imidazo[4,5-b]pyrazin-2(3H)-one;
(S)-1-(1-phenylethyl)-6-(quinolin-5-yl)-1H-imidazo[4,5-b]pyrazin-2(3H)-one;
6-(4-hydroxyphenyl)-1-((tetrahydro-2H-pyran-4-yl)methyl)-1H-imidazo[4,5-b]pyrazin-2(3H)-one;
(S)-6-(naphthalen-1-yl)-1-(1-phenylethyl)-1H-imidazo[4,5-b]pyrazin-2(3H)-one;
(S)-1-(1-hydroxy-3-methylbutan-2-yl)-6-(5-isopropyl-2-methoxyphenyl)-1H-imidazo[4,5-b]pyrazin-2(3H)-one;
(R)-1-(1-hydroxy-3-methylbutan-2-yl)-6-phenyl-1H-imidazo[4,5-b]pyrazin-2(3H)-one;
(R)-1-(1-phenylethyl)-6-(quinolin-5-yl)-1H-imidazo[4,5-b]pyrazin-2(3H)-one;
(S)-1-(1-hydroxy-3-methylbutan-2-yl)-6-(quinolin-5-yl)-1H-imidazo[4,5-b]pyrazin-2(3H)-one;
(R)-1-(1-hydroxy-3-methylbutan-2-yl)-6-(quinolm-5-yl)-1H-imidazo[4,5-b]pyrazin-2(3H)-one;
(R)-1-(1-hydroxy-3-methylbutan-2-yl)-6-(5-isopropyl-2-methoxyphenyl)-1H-imidazo[4,5-b]pyrazin-2(3H)-one;
1-benzyl-6-(quinolin-5-yl)-1H-imidazo[4,5-b]pyrazin-2(3H)-one;
1-(4-methoxybenzyl)-6-(quinolin-5-yl)-1H-imidazo[4,5-b]pyrazin-2(3H)-one;
(R)-1 -(1 -phenylethyl)-1H-imidazo[4,5-b]pyrazin-2(3H)-one;
(S)-1 -(1 -phenylethyl)-1H-imidazo[4,5-b]pyrazin-2(3H)-one;
1-isopropyl-6-(5-isopropyl-2-methoxyphenyl)-1H-imidazo[4,5-b]pyrazin-2(3H)-one;
1-cyclohexyl-6-(5-isopropyl-2-methoxyphenyl)-1H-imidazo[4,5-b]pyrazin-2(3H)-one;
5-(quinolin-5-yl)-1H-imidazo[4,5-b]pyrazin-2(3H)-one;
1-isobutyl-6-(5-isopropyl-2-methoxyphenyl)-1H-imidazo[4,5-b]pyrazin-2(3H)-one;
1-(2-hydroxyethyl)-6-(5-isopropyl-2-methoxyphenyl)-1H-imidazo[4,5-b]pyrazin-2(3H)-one;
6-(5-isopropyl-2-methoxyphenyl)-1-(tetrahydro-2H-pyran-4-yl)-1H-imidazo [4,5-b]pyrazm-2(3H)-one;
(R)-1-(1-phenylethyl)-6-(quinolin-5-yl)-1H-imidazo[4,5-c]pyridin-2(3H)-one;
(S)-1-(1-phenylethyl)-6-(quinolin-5-yl)-1H-imidazo[4,5-c]pyridin-2(3H)-one;
3-(1-phenylethyl)-5-(quinolin-5-yl)-1H-imidazo[4,5-b]pyridin-2(3H)-one;
(R)-3-(1-phenylethyl)-5-(quinolin-5-yl)-1H-imidazo[4,5-b]pyridin-2(3H)-one;
(R)-6-(5-isopropyl-2-methoxyphenyl)-1-(3-methylbutan-2-yl)-1H-imidazo[4,5-b]pyrazin-2(3H)-one;
(S)-6-(5-isopropyl-2-methoxyphenyl)-1-(tetrahydrofuran-3-yl)-1H-imidazo[4,5-b]pyrazin-2(3H)-one;
(S)-6-(5-isopropyl-2-methoxyphenyl)-1-(3-methylbutan-2-yl)-1H-imidazo[4,5-b]pyrazin-2(3H)-one;
1-cyclopentyl-6-(5-isopropyl-2-methoxyphenyl)-1H-imidazo[4,5-b]pyrazin-2(3H)-one;
(R)-6-(5-isopropyl-2-methoxyphenyl)-1-(tetrahydrofuran-3-yl)-1H-imidazo[4,5-b]pyrazin-2(3H)-one;
1-(cyclopropylmethyl)-6-(5-isopropyl-2-methoxyphenyl)-1H-imidazo[4,5-b]pyrazin-2(3H)-one;
1-(cyclopentylmethyl)-6-(5-isopropyl-2-methoxyphenyl)-1H-imidazo[4,5-b]pyrazin-2(3H)-one;
1-(cyclohexylmethyl)-6-(5-isopropyl-2-methoxyphenyl)-1H-imidazo[4,5-b]pyrazin-2(3H)-one;
6-(5-isopropyl-2-methoxyphenyl)-1-neopentyl-1H-imidazo[4,5-b]pyrazin-2(3H)-one;
1-isopropyl-6-(3-isopropylphenyl)-1H-imidazo[4,5-b]pyrazin-2(3H)-one;
1-isopropyl-6-(2-methoxyphenyl)-1H-imidazo[4,5-b]pyrazin-2(3H)-one;
(S)-3-(1-hydroxy-3-methylbutan-2-yl)-5-(5-isopropyl-2-methoxyphenyl)-1H-imidazo[4,5-b]pyridin-2(3H)-one;
(R)-1-(2-hydroxy-1-phenylethyl)-6-(quinolin-5-yl)-1H-imidazo[4,5-b]pyrazin-2(3H)-one;
(S)-1-(2-hydroxy-1-phenylethyl)-6-(quinolin-5-yl)-1H-imidazo[4,5-b]pyrazin-2(3H)-one;
1-(1-phenylethyl)-6-(quinolin-5-yl)-1H-imidazo[4,5-b]pyrazin-2(3H)-one;
1-benzhydryl-6-(quinolin-5-yl)-1H-imidazo[4,5-b]pyrazin-2(3H)-one;
(S)-1-(1-phenylpropyl)-6-(quinolin-5-yl)-1H-imidazo[4,5-b]pyrazin-2(3H)-one;
(R)-1-(1-phenylpropyl)-6-(quinolin-5-yl)-1H-imidazo[4,5-b]pyrazin-2(3H)-one;
6-(5-isopropyl-2-methoxyphenyl)-1-(tetrahydro-2H-pyran-3-yl)-1H-imidazo[4,5-b]pyrazin-2(3H)-one;
1-(3-methoxybenzyl)-6-(quinolin-5-yl)-1H-imidazo[4,5-b]pyrazin-2(3H)-one;
(R)-1-methyl-3-(1-phenylethyl)-5-(quinolin-5-yl)-1H-imidazo[4,5-b]pyrazin-2(3H)-one;
(S)-1-methyl-3-(1-phenylethyl)-5-(quinolin-5-yl)-1H-imidazo[4,5-b]pyrazin-2(3H)-one;
1-(cyclopentylmethyl)-6-(quinolin-5-yl)-1H-imidazo[4,5-b]pyrazin-2(3H)-one;
1-(1-(2-fluorophenyl)ethyl)-6-(quinolin-5-yl)-1H-imidazo[4,5-b]pyrazin-2(3H)-one;
1-(1-(4-fluorophenyl)ethyl)-6-(quinolin-5-yl)-1H-imidazo[4,5-b]pyrazin-2(3H)-one;
1-cyclopentyl-6-(quinolin-5-yl)-1H-imidazo[4,5-b]pyrazin-2(3H)-one;
1-(1-(3-fluorophenyl)ethyl)-6-(quinolin-5-yl)-1H-imidazo[4,5-b]pyrazin-2(3H)-one;
1-(1-(3-methoxyphenyl)ethyl)-6-(quinolin-5-yl)-1H-imidazo[4,5-b]pyrazin-2(3H)-one;
1-(1-(4-methoxyphenyl)ethyl)-6-(quinolin-5-yl)-1H-imidazo[4,5-b]pyrazin-2(3H)-one;
6-(quinolin-5-yl)-1-(tetrahydro-2H-pyran-4-yl)-1H-imidazo[4,5-b]pyrazin-2(3H)-one;
6-(quinolin-5-yl)-1-(tetrahydro-2H-pyran-3-yl)-1H-imidazo[4,5-b]pyrazin-2(3H)-one;
1-((1s,4s)-4-hydroxycyclohexyl)-6-(quinolin-5-yl)-1H-imidazo[4,5-b]pyrazin-2(3H)-one;
1-((1r,4r)-4-hydroxycyclohexyl)-6-(quinolin-5-yl)-1H-imidazo[4,5-b]pyrazin-2(3H)-one;
6-(isoquinolin-5-yl)-1-(1-phenylethyl)-1H-imidazo[4,5-b]pyrazin-2(3H)-one;
(R)-1-(1-phenylethyl)-6-(quinolin-5-yl)-1H-imidazo[4,5-b]pyridin-2(3H)-one;
1-(1-phenylethyl)-6-(quinolin-5-yl)-1H-imidazo[4,5-b]pyridin-2(3H)-one;
1-isopropyl-6-(quinolin-5-yl)-1H-imidazo[4,5-b]pyrazin-2(3H)-one;
1-(1-(4-chlorophenyl)ethyl)-6-(quinolin-5-yl)-1H-imidazo[4,5-b]pyrazin-2(3H)-one;
1-(1-(4-(methylsulfonyl)phenyl)ethyl)-6-(quinolin-5-yl)-1H-imidazo[4,5-b]pyrazin-2(3H)-one;
1-(1-(pyridin-4-yl)ethyl)-6-(quinolin-5-yl)-1H-imidazo[4,5-b]pyrazin-2(3H)-one;
5-methyl-1-((S)-1-phenylethyl)-6-(quinolin-5-yl)-1H-imidazo[4,5-b]pyrazin-2(3H)-one;
5-methyl-1-((R)-1-phenylethyl)-6-(quinolin-5-yl)-1H-imidazo[4,5-b]pyrazin-2(3H)-one;
1-(1-phenylethyl)-6-(quinolin-4-yl)-1H-imidazo[4,5-b]pyrazin-2(3H)-one;
6-(3-fluorophenyl)-1-(1-phenylethyl)-1H-imidazo[4,5-b]pyrazin-2(3H)-one;
6-(2-fluorophenyl)-1-(1-phenylethyl)-1H-imidazo[4,5-b]pyrazin-2(3H)-one;
1-(1-phenylethyl)-6-(quinolin-6-yl)-1H-imidazo[4,5-b]pyrazin-2(3H)-one;
1-(piperidin-4-ylmethyl)-6-(quinolin-5-yl)-1H-imidazo[4,5-b]pyrazin-2(3H)-one;
1-(1-(pyridin-2-yl)ethyl)-6-(quinolin-5-yl)-1H-imidazo[4,5-b]pyrazin-2(3H)-one;
1-(1-(pyridin-3-yl)ethyl)-6-(quinolin-5-yl)-1H-imidazo[4,5-b]pyrazin-2(3H)-one;
1-((1s,4s)-4-(hydroxymethyl)cyclohexyl)-6-(quinolin-5-yl)-1H-imidazo[4,5-b]pyrazin-2(3H)-one;
N-(4-(2-oxo-3-(1-phenylethyl)-2,3-dihydro-1H-imidazo[4,5-b]pyrazm-5-yl)phenyl)methanesulfonamide;
6-(3-(methylsulfbnyl)phenyl)-1-(1-phenylethyl)-1H-imidazo[4,5-b]pyrazm-2(3H)-one;
6-(3-ammophenyl)-1-(1-phenylethyl)-1H-imidazo[4,5-b]pyrazin-2(3H)-one;
6-(3-(dimethylammo)phenyl)-1-(1-phenylethyl)-1H-imidazo[4,5-b]pyrazm-2(3H)-one;
1-phenyl-6-(qumolm-5-yl)-1H-imidazo[4,5-b]pyrazin-2(3H)-one;
1-(1-phenylethyl)-6-(4-(trifluoromethyl)phenyl)-1H-imidazo[4,5-b]pyrazin-2(3H)-one;
N-(3-(2-oxo-3-(1-phenylethyl)-2,3-dihydro-1H-imidazo[4,5-b]pyrazm-5-yl)phenyl)methanesulfonamide;
6-(4-(methylsulfbnyl)phenyl)-1-(1-phenylethyl)-1H-imidazo[4,5-b]pyrazin-2(3H)-one;
3-(1-phenylethyl)-5-(quinolin-5-yl)oxazolo[5,4-b]pyrazin-2(3H)-one;
l-(cyclopentylmethyl)-6-(4-hydroxyphenyl)-1H-imidazo[4,5-b]pyrazin-2(3H)-one
6-(4-hydroxyphenyl)-1-isopropyl-1H-imidazo[4,5-b]pyrazin-2(3H)-one;
6-(4-hydroxyphenyl)-1-isobutyl-1H-imidazo[4,5-b]pyrazin-2(3H)-one;
6-(4-hydroxyphenyl)-1-((tetrahydro-2H-pyran-3-yl)methyl)-1H-imidazo[4,5-b]pyrazin-2(3H)-one;
1-(cyclohexylmethyl)-6-(4-hydroxyphenyl)-1H-imidazo[4,5-b]pyrazin-2(3H)-one;
5-(3-Hydroxyphenyl)-3-(2-methoxyphenyl)-1H-imidazo[4,5-b]pyridin-2(3H)-one;
4-(3-(3-Methoxybenzyl)-2-oxo-2,3-dihydrooxazolo[5,4-b]pyrazin-5-yl)-N-methyl benzamide;
1-Cyclopentyl-6-(4-hydroxyphenyl)-1H-imidazo[4,5-b]pyrazin-2(3H)-one;
l-Cyclohexyl-6-(4-hydroxyphenyl)-1H-imidazo[4,5-b]pyrazin-2(3H)-one;
4-(3-(Cyclohexylmethyl)-2-oxo-2,3-dihydro-1H-imidazo[4,5-b]pyrazin-5-yl)benzamide;
Methyl 4-(3-(cyclohexylmethyl)-2-oxo-2,3-dihydro-1H-imidazo[4,5-b]pyrazin-5-yl)benzoate;
1-(Cyclohexylmethyl)-6-(pyridin-4-yl)-1H-imidazo[4,5-b]pyrazin-2(3H)-one;
4-(3-(Cyclohexylmethyl)-2-oxo-2,3-dihydro-1H-imidazo[4,5-b]pyrazin-5-yl)-N-methylbenzamide;
1-(Cyclohexylmethyl)-6-(4-(hydroxymethyl)phenyl)-1H-imidazo[4,5-b]pyrazin-2(3H)-one;
1-(Cyclohexylmethyl)-6-(pyridin-3-yl)-1H-imidazo[4,5-b]pyrazin-2(3H)-one;
3-(Cyclohexylmethyl)-2-oxo-2,3-dihydro-1H-imidazo[4,5-b]pyrazm-5-yl)benzonitrile;
1-(Cyclohexylmethyl)-6-(1H-indol-5-yl)-1H-imidazo[4,5-b]pyrazin-2(3H)-one;
4-(3-(Cyclohexylmethyl)-2-oxo-2,3-dihydro-1H-imidazo[4,5-b]pyrazin-5-yl)-N-isopropylbenzamide;
1-(2-Hydroxyethyl)-6-(4-hydroxyphenyl)-1H-imidazo[4,5-b]pyrazin-2(3H)-one;
l-(Cyclohexylmethyl)-6-(1H-indol-6-yl)-1H-imidazo[4,5-b]pyrazin-2(3H)-one;
3-(3-(Cyclohexylmethyl)-2-oxo-2,3-dihydro-1H-imidazo[4,5-b]pyrazin-5-yl)benzamide;
6-(4-(Aminomethyl)phenyl)-1-(cyclohexylmethyl)-1H-imidazo[4,5-b]pyrazin-2(3H)-one;
6-(4-Hydroxyphenyl)-1-((1-methylpiperidin-4-yl)methyl)-1H-imidazo[4,5-b]pyrazin-2(3H)-one;;
4-(3-(Cyclohexylmethyl)-2-oxo-2,3-dihydro-1H-imidazo[4,5-b]pyrazin-5-yl)benzonitrile;
1-((1s,4s)-4-Hydroxycyclohexyl)-6-(4-hydroxyphenyl)-1H-imidazo[4,5-b]pyrazin-2(3H)-one;
1-(Cyclohexylmethyl)-6-(pyridin-2-yl)-1H-imidazo[4,5-b]pyrazin-2(3H)-one;
4-(3-(Cyclohexylmethyl)-2-oxo-2,3-dihydro-1H-imidazo[4,5-b]pyrazin-5-yl)-N-ethylbenzamide;
1-(Cyclohexylmethyl)-6-(4-(2-hydroxypropan-2-yl)phenyl)-1H-imidazo[4,5-b]pyrazin-2(3H)-one;
1-(Cyclohexylmethyl)-6-(4-hydroxy-2-methylphenyl)-1H-imidazo[4,5-b]pyrazin-2(3H)-one;
4-(3-(Cyclohexylmethyl)-2-oxo-2,3-dihydro-1H-imidazo[4,5-b]pyrazin-5-yl)benzoic acid;
6-(4-Hydroxyphenyl)-1-(2-methoxyethyl)-1H-imidazo[4,5-b]pyrazin-2(3H)-one;
6-(4-Hydroxyphenyl)-1-(3-methoxypropyl)-1H-imidazo[4,5-b]pyrazin-2(3H)-one;
6-(4-Hydroxyphenyl)-4-(3-methoxybenzyl)-3,4-dihydropyrazino[2,3-b]pyrazin-2(1H)-one;
6-(4-Hydroxyphenyl)-1-(2-(tetrahydro-2H-pyran-4-yl)ethyl)-1H-imidazo[4,5-b]pyrazin-2(3H)-one;
6-(4-Hydroxyphenyl)-1-phenethyl-1H-imidazo[4,5-b]pyrazin-2(3H)-one;
1-((1r,4r)-4-Hydroxycyclohexyl)-6-(4-hydroxyphenyl)-1H-imidazo[4,5-b]pyrazin-2(3H)-one;
6-(4-(1H-1,2,4-Triazol-3-yl)phenyl)-1-(cyclohexylmethyl)-1H-imidazo[4,5-b]pyrazin-2(3H)-one;
1-(Cyclohexylmethyl)-6-phenyl-1H-imidazo[4,5-b]pyrazin-2(3H)-one;
1-(Cyclohexylmethyl)-6-(1H-pyrazol-5-yl)-1H-imidazo[4,5-b]pyrazin-2(3H)-one;
1-(Cyclohexylmethyl)-6-(1H-pyrazol-4-yl)-1H-imidazo[4,5-b]pyrazin-2(3H)-one;
1-(Cyclohexylmethyl)-6-(1-oxoisoindolin-S-yl)-1H-imidazo[4,5-b]pyrazin-2(3H)-one;
6-(3-(1H-Tetrazol-5-yl)phenyl)-1-(cyclohexylmethyl)-1H-imidazo[4,5-b]pyrazin-2(3H)-one;
1-(Cyclohexylmethyl)-6-(2-oxoindolin-5-yl)-1H-imidazo[4,5-b]pyrazin-2(3H)-one;
1-(Cyclohexylmethyl)-6-(1H-indazol-5-yl)-1H-imidazo[4,5-b]pyrazin-2(3H)-one;
1-(Cyclohexylmethyl)-6-(6-methoxypyridin-3-yl)-1H-imidazo[4,5-b]pyrazin-2(3H)-one;
6-(4-Hydroxyphenyl)-1-(tetrahydro-2H-pyran-4-yl)-1H-imidazo[4,5-b]pyrazm-2(3H)-one;
6-(4-Hydroxyphenyl)-1-(piperidm-4-ylmethyl)-1H-imidazo[4,5-b]pyrazm-2(3H)-one;
1-(((1r,4r)-4-Ammocyclohexyl)methyl)-6-(4-hydroxyphenyl)-1H-imidazo[4,5-b]pyrazm-2(3H)-one;
1-(Cyclohexylmethyl)-6-(6-hydroxypyridin-3-yl)-1H-imidazo[4,5-b]pyrazin-2(3H)-one;
1-(Cyclohexylmethyl)-6-(2-methoxypyridin-4-yl)-1H-imidazo[4,5-b]pyrazin-2(3H)-one;
4-(3-((1r,4r)-4-Hydroxycyclohexyl)-2-oxo-2,3-dihydro-1H-imidazo[4,5-b]pyrazin-5-yl)benzamide;
2-(4-(3-(Cyclohexylmethyl)-2-oxo-2,3-dihydro-1H-imidazo[4,5-b]pyrazin-5-yl)phenyl) acetic acid;
2-(4-(3-(Cyclohexylmethyl)-2-oxo-2,3-dihydro-1H-imidazo[4,5-b]pyrazin-5-yl)phenyl) acetamide;
1-(Cyclohexylmethyl)-6-(2-oxoindolin-6-yl)-1H-imidazo[4,5-b]pyrazin-2(3H)-one;
4-(3-(Cyclohexylmethyl)-2-oxo-2,3-dihydro-1H-imidazo[4,5-b]pyrazin-5-yl)-3-methyl benzoic acid;
N-Methyl-4-(2-oxo-3-((tetrahydro-2H-pyran-4-yl)methyl)-2,3-dihydro-1H-imidazo[4,5-b]pyrazin-5-yl)benzamide;
4-(2-oxo-3-((Tetrahydro-2H-pyran-4-yl)methyl)-2,3-dihydro-1H-imidazo[4,5-b]pyrazin-5-yl)benzamide;
7-(4-Hydroxyphenyl)-1-(3-methoxybenzyl)-3,4-dihydropyrazino[2,3-b]pyrazin-2(1H)-one;
6-(4-(2-Hydroxypropan-2-yl)phenyl)-1-((tetrahydro-2H-pyran-4-yl)methyl)-1H-imidazo[4,5-b]pyrazin-2(3H)-one;
6-(1H-Indol-5-yl)-1-((tetrahydro-2H-pyran-4-yl)methyl)-1H-imidazo[4,5-b]pyrazin-2(3H)-one;
6-(4-(4H-1,2,4-Triazol-3-yl)phenyl)-1-((tetrahydro-2H-pyran-4-yl)methyl)-1H-imidazo [4,5-b]pyrazin-2(3H)-one;
6-(1H-Benzo[d]imidazol-5-yl)-1-(cyclohexylmethyl)-1H-imidazo[4,5-b]pyrazin-2(3H)-one;
4-(2-oxo-3-(2-(Tetrahydro-2H-pyran-4-yl)ethyl)-2,3-dihydro-1H-imidazo[4,5-b]pyrazin-5-yl)benzamide;
6-(3-(2H-1,2,3-Triazol-4-yl)phenyl)-1-(cyclohexylmethyl)-1H-imidazo[4,5-b]pyrazin-2(3H)-one;
6-(4-(1H-Imidazol-1-yl)phenyl)-1-(cyclohexylmethyl)-1H-imidazo[4,5-b]pyrazin-2(3H)-one;
6-(4-(1H-1,2,4-Triazol-3-yl)phenyl)-1-((1r,4r)-4-hydroxycyclohexyl)-1H-imidazo[4,5-b]pyrazin-2(3H)-one;
6-(4-(2H-tetrazol-5-yl)phenyl)-1-(cyclohexylmethyl)-1H-imidazo[4,5-b]pyrazin-2(3H)-one;
1-(Cyclohexylmethyl)-6-(2-hydroxypyridin-4-yl)-1H-imidazo[4,5-b]pyrazin-2(3H)-one;
6-(4-(1H-1,2,4-Triazol-3-yl)phenyl)-1-(2-(tetrahydro-2H-pyran-4-yl)ethyl)-1H-imidazo [4,5-b]pyrazin-2(3H)-one;
6-(4-(1H-Imidazol-2-yl)phenyl)-1-(cyclohexylmethyl)-1H-imidazo[4,5-b]pyrazin-2(3H)-one;
6-(4-(1H-1,2,3-Triazol-1-yl)phenyl)-1-(cyclohexylmethyl)-1H-imidazo[4,5-b]pyrazin-2(3H)-one;
6-(4-(2-Hydroxypropan-2-yl)phenyl)-1-(2-(tetrahydro-2H-pyran-4-yl)ethyl)-1H-imidazo[4,5-b]pyrazin-2(3H)-one;
1-(Cyclohexylmethyl)-6-(4-(5-methyl-1H-1,2,4-triazol-3-yl)phenyl)-1H-imidazo[4,5-b]pyrazin-2(3H)-one;
6-(4-(1H-Pyrazol-3-yl)phenyl)-1-(cyclohexylmethyl)-1H-imidazo[4,5-b]pyrazin-2(3H)-one;
6-(4-(lH-Pyrazol-4-yl)phenyl)-1-(cyclohexylmethyl)-1H-imidazo[4,5-b]pyrazin-2(3H)-one;
6-(4-(5-(Aminomethyl)-1H-1,2,4-triazol-3-yl)phenyl)-1-(cyclohexylmethyl)-1H-imidazo[4,5-b]pyrazin-2(3H)-one hydrochloride;
1-(Cyclohexylmethyl)-6-(4-(5-(trifluoromethyl)-1H-1,2,4-triazol-3-yl)phenyl)-1H-imidazo[4,5-b]pyrazin-2(3H)-one;
6-(4-Hydroxyphenyl)-1-((1r,4r)-4-methoxycyclohexyl)-1H-imidazo[4,5-b]pyrazin-2(3H)-one;
6-(4-Hydroxyphenyl)-1-((tetrahydrofuran-2-yl)methyl)-1H-imidazo[4,5-b]pyrazin-2(3H)-one;
6-(3-(1H-1,2,4-Triazol-3-yl)phenyl)-1-(cyclohexylmethyl)-1H-imidazo[4,5-b]pyrazin-2(3H)-one;
1-((1r,4r)-4-(Hydroxymethyl)cyclohexyl)-6-(4-hydroxyphenyl)-1H-imidazo[4,5-b]pyrazin-2(3H)-one;
6-(4-Hydroxyphenyl)-1-((1s,4s)-4-methoxycyclohexyl)-1H-imidazo[4,5-b]pyrazin-2(3H)-one;
6-(4-Hydroxyphenyl)-1-((1r,4r)-4-(methoxymethyl)cyclohexyl)-1H-imidazo[4,5-b]pyrazin-2(3H)-one;
6-(1-Methyl-1H-pyrazol-4-yl)-1-((tetrahydro-2H-pyran-4-yl)methyl)-1H-imidazo[4,5-b]pyrazin-2(3H)-one;
1-(((1r,4r)-4-Hydroxycyclohexyl)methyl)-6-(4-hydroxyphenyl)-1H-imidazo[4,5-b]pyrazin-2(3H)-one;
6-(4-Hydroxyphenyl)-1-((tetrahydrofuran-3-yl)methyl)-1H-imidazo[4,5-b]pyrazin-2(3H)-one;
1-(((1s,4s)-4-Hydroxycyclohexyl)methyl)-6-(4-hydroxyphenyl)-1H-imidazo[4,5-b]pyrazin-2(3H)-one;
6-(1H-Benzo[d]imidazol-5-yl)-1-((tetrahydro-2H-pyran-4-yl)methyl)-1H-imidazo[4,5-b]pyrazin-2(3H)-onehydrochloride;
6-(4-(5-(Morpholinomethyl)-1H-1,2,4-triazol-3-yl)phenyl)-1-((tetrahydro-2H-pyran-4-yl)methyl)-1H-imidazo[4,5-b]pyrazin-2(3H)-one;
6-(4-Hydroxyphenyl)-1-(3-(2-oxopyrrolidin-1-yl)propyl)-1H-imidazo[4,5-b]pyrazin-2(3H)-one;
6-(4-Hydroxyphenyl)-1-(2-morpholinoethyl)-1H-imidazo[4,5-b]pyrazin-2(3H)-one hydrochloride;
1-(Cyclohexylmethyl)-6-(4-(oxazol-5-yl)phenyl)-1H-imidazo[4,5-b]pyrazin-2(3H)-one;
6-(2-Methyl-1H-benzo[d]imidazol-5-yl)-1-((tetrahydro-2H-pyran-4-yl)methyl)-1H-imidazo[4,5-b]pyrazin-2(3H)-one hydrocholoride;
6-(4-(5-(Methoxymethyl)-1H-1,2,4-triazol-3-yl)phenyl)-1-((tetrahydro-2H-pyran-4-yl)methyl)-1H-imidazo[4,5-b]pyrazin-2(3H)-one;
1-((1s,4s)-4-(Hydroxymethyl)cyclohexyl)-6-(4-hydroxyphenyl)-1H-imidazo[4,5-b]pyrazin-2(3H)-one;
6-(3-Methyl-1H-pyrazol-4-yl)-1-((tetrahydro-2H-pyran-4-yl)methyl)-1H-imidazo[4,5-b]pyrazin-2(3H)-one;
6-(1H-Pyrazol-4-yl)-1-((tetrahydro-2H-pyran-4-yl)methyl)-1H-imidazo[4,5-b]pyrazin-2(3H)-one;
6-(2-Amino-1H-benzo[d]imidazol-5-yl)-1-((tetrahydro-2H-pyran-4-yl)methyl)-1H-imidazo[4,5-b]pyrazin-2(3H)-one dihydrochloride;
6-(4-(5-(2-Hydroxypropan-2-yl)-1H-1,2,4-triazol-3-yl)phenyl)-1-((tetrahydro-2H-pyran-4-yl)methyl)-1H-imidazo[4,5-b]pyrazin-2(3H)-one;
6-(4-(5-Isopropyl-1H-1,2,4-triazol-3-yl)phenyl)-1-((tetrahydro-2H-pyran-4-yl)methyl)-1H-imidazo[4,5-b]pyrazin-2(3H)-one;
4-(2-Methoxy-l-(2-morpholinoethyl)-1H-imidazo[4,5-b]pyrazin-6-yl)benzamide hydrochloride;
4-(1-((1s,4s)-4-Hydroxycyclohexyl)-2-methoxy-1H-imidazo[4,5-b]pyrazin-6-yl) benzamide;
6-(4-Hydroxyphenyl)-1-((1s,4s)-4-(methoxymethyl)cyclohexyl)-1H-imidazo[4,5-b]pyrazin-2(3H)-one;
6-(3H-imidazo[4,5-b]pyridin-6-yl)-1-((tetrahydro-2H-pyran-4-yl)methyl)-1H-imidazo[4,5-b]pyrazin-2(3H)-one;
1-(2-(2,2-Dimethyltetrahydro-2H-pyran-4-yl)ethyl)-6-(4-hydroxyphenyl)-1H-imidazo[4,5-b]pyrazin-2(3H)-one;
6-(4-(1H-Pyrazol-1-yl)phenyl)-1-((tetrahydro-2H-pyran-4-yl)methyl)-1H-imidazo[4,5-b]pyrazin-2(3H)-one;
6-(4-(4H-1,2,4-Triazol-3-yl)phenyl)-1-(2-morpholinoethyl)-1H-imidazo[4,5-b]pyrazin-2(3H)-one;
6-(4-(1H-Benzo[d]imidazol-2-yl)phenyl)-1-((tetrahydro-2H-pyran-4-yl)methyl)-1H-imidazo[4,5-b]pyrazin-2(3H)-one;
6-(4-(1H-Imidazol-2-yl)phenyl)-1-((tetrahydro-2H-pyran-4-yl)methyl)-1H-imidazo[4,5-b]pyrazin-2(3H)-one hydrochloride;
6-(4-(5-(Hydroxymethyl)-1H-1,2,4-triazol-3-yl)phenyl)-1-((tetrahydro-2H-pyran-4-yl)methyl)-1H-imidazo[4,5-b]pyrazin-2(3H)-one;
6-(4-(1H-Imidazol-5-yl)phenyl)-1-((tetrahydro-2H-pyran-4-yl)methyl)-1H-imidazo[4,5-b]pyrazin-2(3H)-one hydrochloride;
6-(4-Hydroxyphenyl)-1-((5-oxopyrrolidin-2-yl)methyl)-1H-imidazo[4,5-b]pyrazin-2(3H)-one;
6-(4-(4,5-Dimethyl-1H-imidazol-2-yl)phenyl)-1-((tetrahydro-2H-pyran-4-yl)methyl)-1H-imidazo[4,5-b]pyrazin-2(3H)-one;
6-(4-(1H-1,2,4-Triazol-5-yl)phenyl)-1-(((1s,4s)-4-methoxycyclohexyl)methyl)-1H-imidazo [4,5-b]pyrazin-2(3H)-one;
6-(4-(1H-1,2,4-Triazol-5-yl)phenyl)-1-(((1r,4r)-4-methoxycyclohexyl)methyl)-1H-imidazo[4,5-b]pyrazin-2(3H)-one;
6-(6-(1H-1,2,4-Triazol-3-yl)pyridin-3-yl)-1-((tetrahydro-2H-pyran-4-yl)methyl)-1H-imidazo[4,5-b]pyrazin-2(3H)-one;
6-(4-(1H-1,2,4-Triazol-3-yl)phenyl)-1-(2-(2-oxopyrrolidin-1-yl)ethyl)-1H-imidazo[4,5-b]pyrazin-2(3H)-one;
6-(4-(5-((dimethylamino)methyl)-1H-1,2,4-triazol-3-yl)phenyl)-1-((tetrahydro-2H-pyran-4-yl)methyl)-1H-imidazo[4,5-b]pyrazin-2(3H)-one;
6-(4-Hydroxyphenyl)-1-(pyrrolidm-2-ylmethyl)-1H-imidazo[4,5-b]pyrazin-2(3H)-one hydrochloride;
6-(2-Aminobenzimidazol-5-yl)-1-(cyclohexylmethyl)-4-imidazolino[4,5-b]pyrazin-2-one di hydrochloride;
6-(2-(Dimethylamino)-1H-benzo[d]imidazol-5-yl)-1-((tetrahydro-2H-pyran-4-yl)methyl)-1H-imidazo[4,5-b]pyrazin-2(3H)-one;
6-(4-Hydroxyphenyl)-1-(piperidin-3-ylmethyl)-1H-imidazo[4,5-b]pyrazin-2(3H)-one;
6-(4-(4H-1,2,4-triazol-3-yl)phenyl)-1-(2-(piperidin-1-yl)ethyl)-1H-imidazo[4,5-b]pyrazin-2(3H)-one hydrochloride;
1-(Cyclohexylmethyl)-6-(2-(methylamino)pyrimidin-5-yl)-1H-imidazo[4,5-b]pyrazin-2(3H)-one;
6-(3-methyl-4-(1H-1,2,4-triazol-3-yl)phenyl)-1-((tetrahydro-2H-pyran-4-yl)methyl)-1H-imidazo[4,5-b]pyrazin-2(3H)-one;
1-(Cyclohexylmethyl)-6-(2-(2-methoxyethylamino)pyrimidin-5-yl)-1H-imidazo[4,5-b]pyrazin-2(3H)-one;
6-(4-(5-((methylamino)methyl)-1H-1,2,4-triazol-3-yl)phenyl)-1-((tetrahydro-2H-pyran-4-yl)methyl)-1H-imidazo[4,5-b]pyrazin-2(3H)-one;
6-(4-(5-Oxopyrrolidin-2-yl)phenyl)-1-(2-(tetrahydro-2H-pyran-4-yl)ethyl)-1H-imidazo[4,5-b]pyrazin-2(3H)-one;
6-(4-(5-methyl-1H-1,2,4-triazol-3-yl)phenyl)-1-(2-(tetrahydro-2H-pyran-4-yl)ethyl)-1H-imidazo[4,5-b]pyrazin-2(3H)-one;
6-(4-(1H-imidazol-2-yl)phenyl)-1-(2-(tetrahydro-2H-pyran-4-yl)ethyl)-1H-imidazo[4,5-b]pyrazin-2(3H)-one;
6-(4-(4H-1,2,4-triazol-3-yl)phenyl)-1-(2-methyl-2-morpholinopropyl)-1H-imidazo[4,5-b]pyrazin-2(3H)-one;
6-(4-(4H-1,2,4-Triazol-3-yl)phenyl)-1-(1-morpholinopropan-2-yl)-1H-imidazo[4,5-b]pyrazin-2(3H)-one;
6-(4-(Pyrrolidin-2-yl)phenyl)-1-(2-(tetrahydro-2H-pyran-4-yl)ethyl)-1H-imidazo[4,5-b]pyrazin-2(3H)-one;
6-(4-(5-(ammomethyl)-1H-1,2,4-triazol-3-yl)phenyl)-1-(2-(tetrahydro-2H-pyran-4-yl)ethyl)-1H-imidazo[4,5-b]pyrazin-2(3H)-one;
6-(5-(Hydroxymethyl)thiophen-2-yl)-1-((tetrahydro-2H-pyran-4-yl)methyl)-1H-imidazo[4,5-b]pyrazin-2(3H)-one;
(1r,4r)-4-(6-(4-Hydroxyphenyl)-2-oxo-2,3-dihydro-1H-imidazo[4,5-b]pyrazin-1-yl)cyclohexanecarboxamide;
(1s,4s)-4-(6-(4-Hydroxyphenyl)-2-oxo-2,3-dihydro-1H-imidazo[4,5-b]pyrazin-1-yl)cyclohexanecarboxamide;
6-(4-(5-methyl-1H-1,2,4-triazol-3-yl)phenyl)-1-(2-morpholinoethyl)-1H-imidazo[4,5-b]pyrazin-2(3H)-one;
6-(4-(5-Oxopyrrolidin-3-yl)phenyl)-1-(2-(tetrahydro-2H-pyran-4-yl)ethyl)-1H-imidazo[4,5-b]pyrazin-2(3H)-one;
6-(4-(Pyrrolidin-3-yl)phenyl)-1-(2-(tetrahydro-2H-pyran-4-yl)ethyl)-1H-imidazo[4,5-b]pyrazin-2(3H)-one;
6-(1H-benzo[d]imidazol-5-yl)-1-(2-(tetrahydro-2H-pyran-4-yl)ethyl)-1H-imidazo[4,5-b]pyrazin-2(3H)-one;
6-(3-(Hydroxymethyl)thiophen-2-yl)-1-((tetrahydro-2H-pyran-4-yl)methyl)-1H-imidazo[4,5-b]pyrazin-2(3H)-one;
6-(5-(2-Hydroxyethyl)thiophen-2-yl)-1-((tetrahydro-2H-pyran-4-yl)methyl)-1H-imidazo[4,5-b]pyrazin-2(3H)-one;
1-(Cyclohexylmethyl)-6-(pyrimidin-5-yl)-1H-imidazo[4,5-b]pyrazin-2(3H)-one;
6-(6-Fluoropyridin-3 -yl)-1 -((tetrahydro-2H-pyran-4-yl)methyl)- 1H-imidazo [4,5 -b]pyrazin-2(3H)-one;
6-(6-Aminopyridin-3-yl)-1-((tetrahydro-2H-pyran-4-yl)methyl)-1H-imidazo[4,5-b]pyrazin-2(3H)-one;
6-(4-(5-methyl-1H-imidazol-2-yl)phenyl)-1-((tetrahydro-2H-pyran-4-yl)methyl)-1H-imidazo[4,5-b]pyrazin-2(3H)-one;
6-(4-(5-Methyl-1H-1,2,4-triazol-3-yl)phenyl)-1-(2-(2-oxopyrrolidin-1-yl)ethyl)-1H-imidazo[4,5-b]pyrazin-2(3H)-one;
6-(6-(Methylamino)pyridin-3-yl)-1-((tetrahydro-2H-pyran-4-yl)methyl)-1H-imidazo[4,5-b]pyrazin-2(3H)-one;
6-(2-aminopyrimidin-5-yl)-1-(cyclohexylmethyl)-1H-imidazo[4,5-b]pyrazin-2(3H)-one;
6-(4-(2-hydroxypropan-2-yl)phenyl)-1-((1r,4r)-4-methoxycyclohexyl)methyl)-1H-imidazo[4,5-b]pyrazin-2(3H)-one;
6-(4-hydroxyphenyl)-1-((1-methylpiperidin-3-yl)methyl)-1H-imidazo[4,5-b]pyrazin-2(3H)-one;
6-(2-methyl-4-(1H-1,2,4-triazol-3-yl)phenyl)-1-(2-(tetrahydro-2H-pyran-4-yl)ethyl)-1H-imidazo[4,5-b]pyrazin-2(3H)-one;
1-(cyclohexylmethyl)-6-(6-(2-hydroxypropan-2-yl)pyridin-3-yl)-1H-imidazo[4,5-b]pyrazin-2(3H)-one;
6-(4-(hydroxymethyl)thiophen-2-yl)-1-((tetrahydro-2H-pyran-4-yl)methyl)-1H-imidazo[4,5-b]pyrazin-2(3H)-one;
6-(1H-benzo[d]imidazol-6-yl)-1-(((1r,4r)-4-methoxycyclohexyl)methyl)-1H-imidazo[4,5-b]pyrazin-2(3H)-one;
6-(4-(4,5-dimethyl-1H-imidazol-2-yl)phenyl)-1-(2-morpholinoethyl)-1H-imidazo[4,5-b]pyrazin-2(3H)-one;
6-(6-(2-hydroxypropan-2-yl)pyridin-3-yl)-1-((tetrahydro-2H-pyran-4-yl)methyl)-1H-imidazo[4,5-b]pyrazin-2(3H)-one;
6-(6-(2-hydroxypropan-2-yl)pyridin-3-yl)-1-(2-(tetrahydro-2H-pyran-4-yl)ethyl)-1H-imidazo[4,5-b]pyrazin-2(3H)-one;
6-(4-(4H-1,2,4-triazol-3-yl)phenyl)-1-(2-morpholino-2-oxoethyl)-1H-imidazo[4,5-b]pyrazin-2(3H)-one;
6-(4-(4H-1,2,4-triazol-3-yl)phenyl)-3-(cyclohexylmethyl)-3,4-dihydropyrazino[2,3-b]pyrazin-2(1H)-one;
6-(4-(1H-1,2,4-triazol-3-yl)phenyl)-1-(2-(tetrahydro-2H-pyran-4-yl)ethyl)-1H-imidazo[4,5-b]pyridin-2(3H)-one;
(R)-6-(4-(1H-1,2,4-triazol-3-yl)phenyl)-1-(1-phenylethyl)-1H-imidazo[4,5-b]pyrazin-2(3H)-one;
(S)-6-(4-(1H-1,2,4-triazol-3-yl)phenyl)-1-(1-phenylethyl)-1H-imidazo[4,5-b]pyrazin-2(3H)-one;
(1r,4r)-4-(6-(4-(2-hydroxypropan-2-yl)phenyl)-2-oxo-2,3-dihydro-1H-imidazo[4,5-b]pyrazin-1-yl)cyclohexanecarboxamide;
6-(3-Methyl-4-(1H-1,2,4-Triazol-3-yl)phenyl)-1-((tetrahydro-2H-pyran-4-yl)methyl)-1H-imidazo[4,5-B]pyrazin-2(3H)-one;
6-(4-(1H-imidazol-2-yl)phenyl)-1-(2-(tetrahydro-2H-pyran-4-yl)ethyl)-1H-imidazo[4,5-b]pyrazin-2(3H)-one;
6-(4-(5-(Ammomethyl)-1H-1,2,4-triazol-3-yl)phenyl)-1-(2-(tetrahydro-2H-pyran-4-yl)ethyl)-1H-imidazo[4,5-b]pyrazin-2(3H)-one;
6-(1H-benzo[d]imidazol-5-yl)-1-(2-(tetrahydro-2H-pyran-4-yl)ethyl)-1H-imidazo[4,5-b]pyrazin-2(3H)-one;
6-(2-Aminopyrimidin-5-yl)-1-(cyclohexylmethyl)-1H-imidazo[4,5-b]pyrazin-2(3H)-one;
6-(4-Hydroxyphenyl)-1-((1-methylpiperidin-2-yl)methyl)-1H-imidazo[4,5-b]pyrazin-2(3H)-one hydrochloride;
6-(3-Methyl-4-(1H-1,2,4-Triazol-3-yl)phenyl)-1-((tetrahydro-2H-pyran-4-yl)methyl)-1H-imidazo[4,5-B]pyrazin-2(3H)-one;
1-(Cyclohexylmethyl)-6-(6-(2-hydroxypropan-2-yl)pyridin-3-yl)-1H-imidazo[4,5-b]pyrazin-2(3H)-one;
6-(6-(2-Hydroxypropan-2-yl)pyridin-3-yl)-1-((tetrahydro-2H-pyran-4-yl)methyl)-1H-imidazo[4,5-b]pyrazin-2(3H)-one;
6-(6-(2-Hydroxypropan-2-yl)pyridin-3-yl)-1-(2-(tetrahydro-2H-pyran-4-yl)ethyl)-1H-imidazo[4,5-b]pyrazin-2(3H)-one;
6-(4-(4H-1,2,4-Triazol-3-yl)phenyl)-1-(2-morpholino-2-oxoethyl)-1H-imidazo[4,5-b]pyrazin-2(3H)-one;
(R)-6-(4-(4H-1,2,4-Triazol-3-yl)phenyl)-3-(cyclohexylmethyl)-3,4-dihydropyrazino[2,3-b]pyrazin-2(1H)-one;
(R)-6-(4-(1H-1,2,4-Triazol-3-yl)phenyl)-1-(1-phenylethyl)-1H-imidazo[4,5-B]pyrazin-2(3H)-one;
(S)-6-(4-(4H-1,2,4-Triazol-3-yl)phenyl)-1-(1-phenylethyl)-1H-imidazo[4,5-b]pyrazin-2(3H)-one;
(1r,4r)-4-(6-(4-(2-Hydroxypropan-2-yl)phenyl)-2-oxo-2,3-dihydro-1H-imidazo[4,5-b]pyrazin-1-yl)cyclohexanecarboxamide; and
6-(4-(5-Methyl-1H-1,2,4-triazol-3-yl)phenyl)-1-(2-(tetrahydro-2H-pyran-4-yl)ethyl)-1H-imidazo[4,5-b]pyrazin-2(3H)-one,
and pharmaceutically acceptable salts, clathrates, solvates, stereoisomers, tautomers, and prodrugs thereof.

As also described herein, the TOR kinase inhibitors may include compounds having the following formula (II): 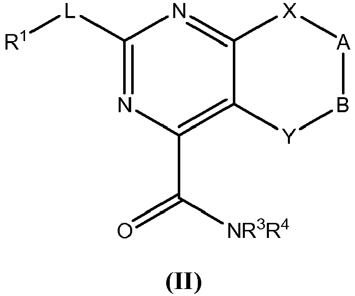 and pharmaceutically acceptable salts, clathrates, solvates, stereoisomers, tautomers, and prodrugs thereof, wherein:
R¹ is substituted or unsubstituted C₁₋₈alkyl, substituted or unsubstituted aryl, substituted or unsubstituted heteroaryl, substituted or unsubstituted cycloalkyl, or substituted or unsubstituted heterocyclylalkyl;
-X-A-B-Y- taken together form -N(R²)CH₂C(O)NH-, -N(R²)C(O)CH₂NH-, -N(R²)C(O)NH-, -N(R²)C=N-, or -C(R²)=CHNH-;
L is a direct bond, NH or O;
R² is substituted or unsubstituted C₁₋₈alkyl, substituted or unsubstituted aryl, substituted or unsubstituted heteroaryl, substituted or unsubstituted cycloalkyl, or substituted or unsubstituted heterocyclylalkyl; and
R³ and R⁴ are independently H or C₁₋₈alkyl.

The TOR kinase inhibitors of formula (II) may be those wherein -X-A-B-Y- taken together form -N(R²)CH₂C(O)NH-.

The TOR kinase inhibitors of formula (II) may be those wherein -X-A-B-Y- taken together form -N(R²)C(O)CH₂NH-.

The TOR kinase inhibitors of formula (II) may be those wherein -X-A-B-Y- taken together form -N(R²)C(O)NH-.

The TOR kinase inhibitors of formula (II) may be those wherein -X-A-B-Y- taken together form -N(R²)C=N-.

The TOR kinase inhibitors of formula (II) may be those wherein -X-A-B-Y- taken together form -C(R²)=CHNH-.

The TOR kinase inhibitors of formula (II) may be those wherein L is a direct bond.

The TOR kinase inhibitors of formula (II) may be those wherein R¹ is substituted aryl, such as substituted phenyl.

The TOR kinase inhibitors of formula (II) may be those wherein R¹ is substituted or unsubstituted heteroaryl, such as substituted or unsubstituted pyridine, substituted or unsubstituted indole or substituted or unsubstituted quinoline.

The TOR kinase inhibitors of formula (II) may be those wherein R¹ is substituted or unsubstituted cycloalkyl, such as substituted or unsubstituted cyclopentyl.

The TOR kinase inhibitors of formula (II) may be those wherein -X-A-B-Y- taken together form -N(R²)C(O)NH- and R¹ is substituted aryl, such as phenyl.

The TOR kinase inhibitors of formula (II) may be those wherein -X-A-B-Y- taken together form -N(R²)C(O)NH- and R¹ is substituted or unsubstituted heteroaryl, such as substituted or unsubstituted pyridine, substituted or unsubstituted indole or substituted or unsubstituted quinoline.

The TOR kinase inhibitors of formula (II) may be those wherein -X-A-B-Y- taken together form -N(R²)C(O)NH- and R¹ is substituted or unsubstituted cycloalkyl, such as substituted or unsubstituted cyclopentyl.

The TOR kinase inhibitors of formula (II) may be those wherein R² is substituted C₁₋₈alkyl, such as -CH₂C₆H₅.

The TOR kinase inhibitors of formula (II) may be those wherein R² is unsubstituted C₁₋₈alkyl, such as unsubstituted methyl.

The TOR kinase inhibitors of formula (II) may be those wherein R² is substituted or unsubstituted aryl, such as substituted or unsubstituted phenyl.

The TOR kinase inhibitors of formula (II) may be those wherein R² is substituted aryl, such as halo, haloalkyl or alkoxy substituted phenyl.

The TOR kinase inhibitors of formula (II) may be those wherein R² is substituted or unsubstituted cycloalkyl, such as substituted or unsubstituted cyclohexyl or substituted or unsubstituted cycloheptyl.

The TOR kinase inhibitors of formula (II) may be those wherein R² is substituted heterocyclylalkyl, such as substituted piperidine.

The TOR kinase inhibitors of formula (II) may be those wherein R³ and R⁴ are H.

The TOR kinase inhibitors of formula (II) may be those wherein -X-A-B-Y- taken together form -N(R²)C(O)NH- and R² is unsubstituted aryl, such as unsubstituted phenyl.

The TOR kinase inhibitors of formula (II) may be those wherein -X-A-B-Y- taken together form -N(R²)C(O)NH-, R¹ is substituted or unsubstituted heteroaryl, such as substituted or unsubstituted pyridine, and R² is substituted or unsubstituted aryl, such as substituted or unsubstituted phenyl.

The TOR kinase inhibitors of formula (II) may be those wherein -X-A-B-Y- taken together form -N(R²)C(O)NH-, R¹ is substituted or unsubstituted heteroaryl, such as substituted or unsubstituted pyridine, R² is substituted or unsubstituted aryl, such as substituted or unsubstituted phenyl, and R³ and R⁴ are H.

The TOR kinase inhibitors of formula (II) may be those wherein -X-A-B-Y- taken together form -N(R²)C(O)NH-, L is a direct bond, R¹ is substituted or unsubstituted heteroaryl, such as substituted or unsubstituted pyridine, R² is substituted or unsubstituted aryl, such as substituted or unsubstituted phenyl, and R³ and R⁴ are H.

The TOR kinase inhibitors of formula (II) may be those wherein -X-A-B-Y- taken together form -N(R²)C(O)NH-, R¹ is substituted or unsubstituted aryl, such as substituted or unsubstituted phenyl, and R² is substituted or unsubstituted aryl, such as substituted or unsubstituted phenyl.

The TOR kinase inhibitors of formula (II) may be those wherein -X-A-B-Y- taken together form -N(R²)C(O)NH-, R¹ is substituted or unsubstituted aryl, such as substituted or unsubstituted phenyl, R² is substituted or unsubstituted aryl, such as substituted or unsubstituted phenyl, and R³ and R⁴ are H.

The TOR kinase inhibitors of formula (II) may be those wherein -X-A-B-Y- taken together form -N(R²)C(O)NH-, L is a direct bond, R¹ is substituted or unsubstituted aryl, such as substituted or unsubstituted phenyl, R² is substituted or unsubstituted aryl, such as substituted or unsubstituted phenyl, and R³ and R⁴ are H.

The TOR kinase inhibitors of formula (II) may be those wherein -X-A-B-Y- taken together form -N(R²)C(O)NH-, R¹ is substituted or unsubstituted heteroaryl, L is a direct bond and R² is substituted or unsubstituted C₁₋₈alkyl or substituted or unsubstituted cycloalkyl.

The TOR kinase inhibitors of formula (II) may be those wherein -X-A-B-Y- taken together form -N(R²)C(O)NH-, R¹ is substituted or unsubstituted aryl, L is a direct bond and R² is substituted or unsubstituted C₁₋₈alkyl or substituted or unsubstituted cycloalkyl.

The TOR kinase inhibitors of formula (II) may not include 8,9-dihydro-8-oxo-9-phenyl-2-(3-pyridinyl)-7H-purine-6-carboxamide, 8,9-dihydro-8-oxo-9-phenyl-2-(3-pyridinyl)-7H-purine-6-carboxamide, 8,9-dihydro-8-oxo-9-phenyl-2-(3-pyridinyl)-7H-purine-6-carboxamide, 2-(4-cyanophenyl)-8-oxo-9-phenyl-8,9-dihydro-7H-purine-6-carboxamide, 2-(4-nitrophenyl)-8-oxo-9-phenyl-8,9-dihydro-7H-purine-6-carboxamide, 9-benzyl-2-(4-methoxyphenyl)-8-oxo-8,9-dihydro-7H-purine-6-carboxamide, 2-methyl-8-oxo-9-phenyl-8,9-dihydro-7H-purine-6-carboxamide, 9-benzyl-9H-purine-2,6-dicarboxamide, 9-[2,3-bis[(benzoyloxy)methyl]cyclobutyl]-2-methyl-9H-Purine-6-carboxamide, 9-benzyl-2-methyl-9H-purine-6-carboxamide, 9-(2-hydroxyethyl)-2-methyl-9H-purine-6-carboxamide, 9-(2-hydroxyethyl)-2-(trifluoromethyl)-9H-purine-6-carboxamide, 9-(2-hydroxyethyl)-2-(prop-1-enyl)-9H-purine-6-carboxamide, 9-(2-hydroxyethyl)-2-phenyl-9H-purine-6-carboxamide, 9-(3-hydroxypropyl)-2-methyl-9H-purine-6-carboxamide, 9-(3-hydroxypropyl)-2-(trifluoromethyl)-9H-purine-6-carboxamide, 2-methyl-9-phenylmethyl-9H-purine-6-carboxamide or 2-methyl-9-β-D-ribofuranosyl-9H-purine-6-carboxamide.

The TOR kinase inhibitors of formula (II) may not include compounds wherein R² is a substituted furanoside.

The TOR kinase inhibitors of formula (II) may not include compounds wherein R² is a substituted or unsubstituted furanoside.

The TOR kinase inhibitors of formula (II) may not include (2'R)-2'-deoxy-2'-fluoro-2'-*C*-methyl nucleosides.

As also described herein, the TOR kinase inhibitors may include compounds having the following formula (IIa): 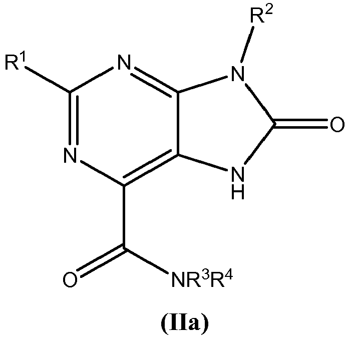 and pharmaceutically acceptable salts, clathrates, solvates, stereoisomers, tautomers, and prodrugs thereof, wherein:
R¹ is substituted or unsubstituted C₁₋₈alkyl, substituted or unsubstituted aryl, substituted or unsubstituted heteroaryl, substituted or unsubstituted cycloalkyl, or substituted or unsubstituted heterocyclylalkyl;
R² is substituted or unsubstituted C₁₋₈alkyl, substituted or unsubstituted aryl, substituted or unsubstituted heteroaryl, substituted or unsubstituted cycloalkyl, or substituted or unsubstituted heterocyclylalkyl; and
R³ and R⁴ are independently H or C₁₋₈alkyl.

The TOR kinase inhibitors of formula (IIa) may be those wherein R¹ is substituted aryl, substituted or unsubstituted heteroaryl, such as substituted phenyl.

The TOR kinase inhibitors of formula (IIa) may be those wherein R¹ is substituted or unsubstituted heteroaryl, such as substituted or unsubstituted pyridine, substituted or unsubstituted indole or substituted or unsubstituted quinoline.

The TOR kinase inhibitors of formula (IIa) may be those wherein R¹ is substituted or unsubstituted cycloalkyl, such as substituted or unsubstituted cyclopentyl.

The TOR kinase inhibitors of formula (IIa) may be those wherein R² is substituted C₁₋₈alkyl, such as -CH₂C₆H₅.

The TOR kinase inhibitors of formula (IIa) may be those wherein R² is unsubstituted C₁₋₈alkyl, such as unsubstituted methyl.

The TOR kinase inhibitors of formula (IIa) may be those wherein R² is substituted or unsubstituted aryl, such as substituted or unsubstituted phenyl.

The TOR kinase inhibitors of formula (IIa) may be those wherein R² is substituted aryl, such as halo, haloalkyl or alkoxy substituted phenyl.

The TOR kinase inhibitors of formula (IIa) may be wherein R² is substituted or unsubstituted cycloalkyl, such as substituted or unsubstituted cyclohexyl or substituted or unsubstituted cycloheptyl.

The TOR kinase inhibitors of formula (IIa) may be those wherein R² is substituted heterocyclylalkyl, such as substituted piperidine.

The TOR kinase inhibitors of formula (IIa) may be those wherein R³ and R⁴ are H.

The TOR kinase inhibitors of formula (IIa) may not include 8,9-dihydro-8-oxo-9-phenyl-2-(3-pyridinyl)-7H-Purine-6-carboxamide, 8,9-dihydro-8-oxo-9-phenyl-2-(3-pyridinyl)-7H-Purine-6-carboxamide, 8,9-dihydro-8-oxo-9-phenyl-2-(3-pyridinyl)-7H-Purine-6-carboxamide, 2-(4-cyanophenyl)-8-oxo-9-phenyl-8,9-dihydro-7H-purine-6-carboxamide, 2-(4-nitrophenyl)-8-oxo-9-phenyl-8,9-dihydro-7H-purine-6-carboxamide, 9-benzyl-2-(4-methoxyphenyl)-8-oxo-8,9-dihydro-7H-purine-6-carboxamide, 9-phenylmethyl-9H-purine-2,6-dicarboxamide, or 2-methyl-8-oxo-9-phenyl-8,9-dihydro-7H -purine-6-carboxamide.

The TOR kinase inhibitors of formula (IIa) may not include compounds wherein R² is a substituted furanoside.

The TOR kinase inhibitors of formula (IIa) may not include compounds wherein R² is a substituted or unsubstituted furanoside.

The TOR kinase inhibitors of formula (IIa) may not include (2'R)-2'-deoxy-2'-fluoro-2'-*C*-methyl nucleosides.

As also described, herein the TOR kinase inhibitory may include compounds having the following formula (IIb): 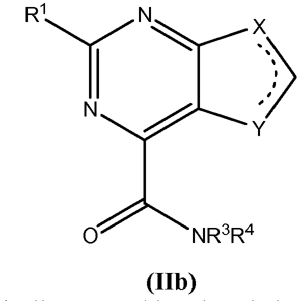 and pharmaceutically acceptable salts, clathrates, solvates, stereoisomers, tautomers, and prodrugs thereof, wherein: 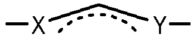 is -C(R²)=CH-NH- or -N(R²)-CH=N-;
R¹ is substituted or unsubstituted C₁₋₈alkyl, substituted or unsubstituted aryl, substituted or unsubstituted heteroaryl, substituted or unsubstituted cycloalkyl, or substituted or unsubstituted heterocyclylalkyl;
R² is substituted or unsubstituted C₁₋₈alkyl, substituted or unsubstituted aryl, substituted or unsubstituted heteroaryl, substituted or unsubstituted cycloalkyl, or substituted or unsubstituted heterocyclylalkyl; and
R³ and R⁴ are independently H or C₁₋₈alkyl.

The TOR kinase inhibitors of formula (IIb) may be those wherein R¹ is substituted aryl, such as substituted phenyl.

The TOR kinase inhibitors of formula (IIb) may be those wherein R¹ is substituted or unsubstituted heteroaryl, such as substituted or unsubstituted pyridine, substituted or unsubstituted indole or substituted or unsubstituted quinoline.

The TOR kinase inhibitors of formula (IIb) may be those wherein R¹ is substituted or unsubstituted cycloalkyl, such as substituted or unsubstituted cyclopentyl.

The TOR kinase inhibitors of formula (IIb) may be those wherein R² is substituted C₁₋₈alkyl, such as -CH₂C₆H₅.

The TOR kinase inhibitors of formula (IIb) may be those wherein R² is unsubstituted C₁₋₈alkyl, such as unsubstituted methyl.

The TOR kinase inhibitors of formula (IIb) may be those wherein R² is substituted or unsubstituted aryl, such as substituted or unsubstituted phenyl.

The TOR kinase inhibitors of formula (IIb) may be those wherein R² is substituted aryl, such as halo, haloalkyl or alkoxy substituted phenyl.

The TOR kinase inhibitors of formula (IIb) may be those wherein R² is substituted or unsubstituted cycloalkyl, such as substituted or unsubstituted cyclohexyl or substituted or unsubstituted cycloheptyl.

The TOR kinase inhibitors of formula (IIb) may be those wherein R² is substituted heterocyclylalkyl, such as substituted piperidine.

The TOR kinase inhibitors of formula (IIb) may be those wherein R³ and R⁴ are H.

The TOR kinase inhibitors of formula (IIb) may be those wherein 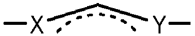 is -C(R²)=CH-NH- and R² is substituted aryl, such as substituted phenyl.

The TOR kinase inhibitors of formula (IIb) may be those wherein 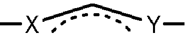 is -N(R²)-CH=N- and R² is substituted aryl, such as substituted phenyl.

The TOR kinase inhibitors of formula (IIb) may be those wherein R¹ is substituted aryl, such as phenyl, and R² is substituted aryl, such as substituted phenyl.

The TOR kinase inhibitors of formula (IIb) may not include 9-benzyl-9H-purine-2,6-dicarboxamide, 9-[2,3-bis[(benzoyloxy)methyl]cyclobutyl]-2-methyl-9H-Purine-6-carboxamide, 9-benzyl-2-methyl-9H-purine-6-carboxamide, 9-(2-hydroxyethyl)-2-methyl-9H-purine-6-carboxamide, 9-(2-hydroxyethyl)-2-(trifluoromethyl)-9H-purine-6-carboxamide, 9-(2-hydroxyethyl)-2-(prop-1-enyl)-9H-purine-6-carboxamide, 9-(2-hydroxyethyl)-2-phenyl-9H-purine-6-carboxamide, 9-(3-hydroxypropyl)-2-methyl-9H-purine-6-carboxamide, 9-(3-hydroxypropyl)-2-(trifluoromethyl)-9H-purine-6-carboxamide, 9-phenylmethyl-9H-purine-2,6-dicarboxamide, 2-methyl-9-phenylmethyl-9H-purine-6-carboxamide or 2-methyl-9-β-D-ribofuranosyl-9H-purine-6-carboxamide.

The TOR kinase inhibitors of formula (IIb) may not include compounds wherein R² is substituted cyclobutyl when 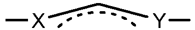 is -N(R²)-CH=N-.

The TOR kinase inhibitors of formula (IIb) may not include compounds wherein R² is a substituted furanoside when 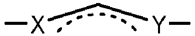 is -N(R²)-CH=N-.

The TOR kinase inhibitors of formula (IIb) may not include compounds wherein R² is substituted pyrimidine when 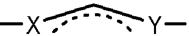 is -C(R²)=CH-NH-.

The TOR kinase inhibitors of formula (IIb) may not include compounds wherein R² is substituted oxetane when 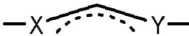 is -N(R²)-CH=N-.

The TOR kinase inhibitors of formula (IIb) may not include compounds wherein R² is substituted cyclopentyl or a heterocyclopentyl when 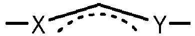 is -N(R²)-CH=N-.

As also described herein, the TOR kinase inhibitors may include compounds having the following formula (IIc): 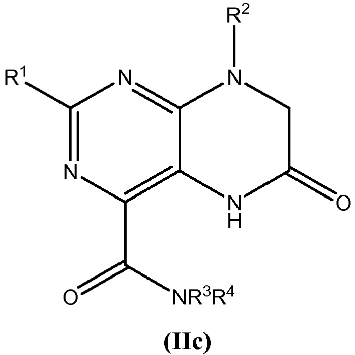 and pharmaceutically acceptable salts, clathrates, solvates, stereoisomers, tautomers, and prodrugs thereof, wherein:
R¹ is substituted or unsubstituted C₁₋₈alkyl, substituted or unsubstituted aryl, substituted or unsubstituted heteroaryl, substituted or unsubstituted cycloalkyl, or substituted or unsubstituted heterocyclylalkyl;
R² is substituted or unsubstituted C₁₋₈alkyl, substituted or unsubstituted aryl, substituted or unsubstituted heteroaryl, substituted or unsubstituted cycloalkyl, or substituted or unsubstituted heterocyclylalkyl; and
R³ and R⁴ are independently H or C₁₋₈alkyl.

The TOR kinase inhibitors of formula (IIc) may be those wherein R¹ is substituted aryl, such as substituted phenyl.

The TOR kinase inhibitors of formula (IIc) may be those wherein R¹ is substituted or unsubstituted heteroaryl, such as substituted or unsubstituted pyridine, substituted or unsubstituted indole or substituted or unsubstituted quinoline.

The TOR kinase inhibitors of formula (IIc) may be those wherein R¹ is substituted or unsubstituted cycloalkyl, such as substituted or unsubstituted cyclopentyl.

The TOR kinase inhibitors of formula (IIc) may be those wherein R² is substituted C₁₋₈alkyl, such as -CH₂C₆H₅.

The TOR kinase inhibitors of formula (IIc) may be those wherein R² is unsubstituted C₁₋₈alkyl, such as unsubstituted methyl.

The TOR kinase inhibitors of formula (IIc) may be those wherein R² is substituted or unsubstituted aryl, such as substituted or unsubstituted phenyl.

The TOR kinase inhibitors of formula (IIc) may be those wherein R² is substituted aryl, such as halo, haloalkyl or alkoxy substituted phenyl.

The TOR kinase inhibitors of formula (IIc) may be those wherein R² is substituted or unsubstituted cycloalkyl, such as substituted or unsubstituted cyclohexyl or substituted or unsubstituted cycloheptyl.

The TOR kinase inhibitors of formula (IIc) may be those wherein R² is substituted heterocyclylalkyl, such as substituted piperidine.

The TOR kinase inhibitors of formula (IIc) may be those wherein R³ and R⁴ are H.

As also described herein TOR kinase inhibitors may include compounds having the following formula (IId): 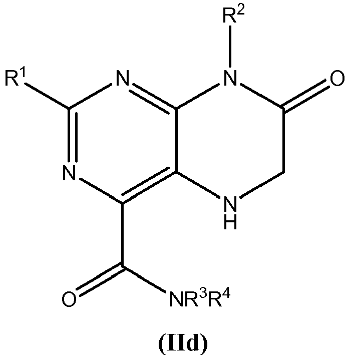 and pharmaceutically acceptable salts, clathrates, solvates, stereoisomers, tautomers, and prodrugs thereof, wherein:
R¹ is substituted or unsubstituted C₁₋₈alkyl, substituted or unsubstituted aryl, substituted or unsubstituted heteroaryl, substituted or unsubstituted cycloalkyl, or substituted or unsubstituted heterocyclylalkyl;
R² is substituted or unsubstituted C₁₋₈alkyl, substituted or unsubstituted aryl, substituted or unsubstituted heteroaryl, substituted or unsubstituted cycloalkyl, or substituted or unsubstituted heterocyclylalkyl; and
R³ and R⁴ are independently H or C₁₋₈alkyl.

The TOR kinase inhibitors of formula (IId) may be those wherein R¹ is substituted aryl, such as substituted phenyl.

The TOR kinase inhibitors of formula (IId) may be those wherein R¹ is substituted or unsubstituted heteroaryl, such as substituted or unsubstituted pyridine, substituted or unsubstituted indole or substituted or unsubstituted quinoline.

The TOR kinase inhibitors of formula (IId) may be those wherein R¹ is substituted or unsubstituted cycloalkyl, such as substituted or unsubstituted cyclopentyl.

The TOR kinase inhibitors of formula (IId) may be those wherein R² is substituted C₁₋₈alkyl, such as -CH₂C₆H₅.

The TOR kinase inhibitors of formula (IId) may be those wherein R² is unsubstituted C₁₋₈alkyl, such as unsubstituted methyl.

The TOR kinase inhibitors of formula (IId) may be those wherein R² is substituted or unsubstituted aryl, such as substituted or unsubstituted phenyl.

The TOR kinase inhibitors of formula (IId) may be those wherein R² is substituted aryl, such as halo, haloalkyl or alkoxy substituted phenyl.

The TOR kinase inhibitors of formula (IId) may be those wherein R² is substituted or unsubstituted cycloalkyl, such as substituted or unsubstituted cyclohexyl or substituted or unsubstituted cycloheptyl.

The TOR kinase inhibitors of formula (IId) may be those wherein R² is substituted heterocyclylalkyl, such as substituted piperidine.

The TOR kinase inhibitors of formula (IId) may be those wherein R³ and R⁴ are H.

Representative TOR kinase inhibitors of formula (IV) may include:
9-benzyl-8-oxo-2-(pyridin-3-yl)-8,9-dihydro-7H-purine-6-carboxamide;
N-methyl-8-oxo-9-phenyl-2-(pyridin-3-yl)-8,9-dihydro-7H-purine-6-carboxamide;
8-oxo-9-phenyl-2-(pyridin-2-yl)-8,9-dihydro-7H-purine-6-carboxamide;
2-(2-chloropyridin-3-yl)-8-oxo-9-phenyl-8,9-dihydro-7H-purine-6-carboxamide;
2-(2-methoxypyridin-3-yl)-8-oxo-9-phenyl-8,9-dihydro-7H-purine-6-carboxamide;
N,N-dimethyl-8-oxo-9-phenyl-2-(pyridin-3-yl)-8,9-dihydro-7H-purine-6-carboxamide;
9-methyl-8-oxo-2-(pyridin-3-yl)-8,9-dihydro-7H-purine-6-carboxamide;
2-(4-hydroxyphenyl)-9-(2-methoxyphenyl)-8-oxo-8,9-dihydro-7H-purine-6-carboxamide;
2-(3-hydroxyphenyl)-8-oxo-9-o-tolyl-8,9-dihydro-7H-purine-6-carboxamide;
2-(1H-indol-4-yl)-9-(2-methoxyphenyl)-8-oxo-8,9-dihydro-7H-purine-6-carboxamide;
2-(1H-indol-6-yl)-9-(2-methoxyphenyl)-8-oxo-8,9-dihydro-7H-purine-6-carboxamide;
2-(3-hydroxyphenyl)-9-(4-methoxyphenyl)-8-oxo-8,9-dihydro-7H-purine-6-carboxamide;
2-(2-hydroxypyridin-4-yl)-9-(2-methoxyphenyl)-8-oxo-8,9-dihydro-7H-purine-6-carboxamide;
9-(2-chlorophenyl)-2-(3-hydroxyphenyl)-8-oxo-8,9-dihydro-7H-purine-6-carboxamide;
9-(2-fluorophenyl)-2-(3-hydroxyphenyl)-8-oxo-8,9-dihydro-7H-purine-6-carboxamide;
9-(2,6-difluorophenyl)-2-(3-hydroxyphenyl)-8-oxo-8,9-dihydro-7H-purine-6-carboxamide;
9-cycloheptyl-8-oxo-2-(pyridin-3-yl)-8,9-dihydro-7H-purine-6-carboxamide;
9-(2-methoxyphenyl)-8-oxo-2-(quinolin-5-yl)-8,9-dihydro-7H-purine-6-carboxamide;
2-cyclopentyl-9-(2-methoxyphenyl)-8-oxo-8,9-dihydro-7H-purine-6-carboxamide;
9-(2-methoxyphenyl)-8-oxo-2-(3-(trifluoromethyl)phenyl)-8,9-dihydro-7H-purine-6-carboxamide;
9-(2-methoxyphenyl)-2-(6-methoxypyridin-3-yl)-8-oxo-8,9-dihydro-7H-purine-6-carboxamide;
2-(3-hydroxyphenyl)-8-oxo-9-(4-(trifluoromethyl)phenyl)-8,9-dihydro-7H-purine-6-carboxamide;
9-benzyl-2-(3-hydroxyphenyl)-8-oxo-8,9-dihydro-7H-purine-6-carboxamide;
2-(3-hydroxyphenyl)-8-oxo-9-(2-(trifluoromethoxy)phenyl)-8,9-dihydro-7H-purine-6-carboxamide;
9-(2,4-dichlorophenyl)-2-(3-hydroxyphenyl)-8-oxo-8,9-dihydro-7H-purine-6-carboxamide;
9-(2-methoxyphenyl)-2-(3-nitrophenyl)-8-oxo-8,9-dihydro-7H-purine-6-carboxamide;
2-(3-cyanophenyl)-8-oxo-9-phenyl-8,9-dihydro-7H-purine-6-carboxamide;
9-(3-fluorophenyl)-2-(3-hydroxyphenyl)-8-oxo-8,9-dihydro-7H-purine-6-carboxamide;
9-(2-methoxyphenyl)-8-oxo-2-(2-(trifluoromethyl)phenyl)-8,9-dihydro-7H-purine-6-carboxamide;
2-(5-fluoropyridin-3-yl)-9-(2-methoxyphenyl)-8-oxo-8,9-dihydro-7H-purine-6-carboxamide;
2-(1-benzylpiperidin-4-yl)-9-(2-methoxyphenyl)-8-oxo-8,9-dihydro-7H-purine-6-carboxamide;
benzyl 4-(6-carbamoyl-8-oxo-2-(pyridin-3-yl)-7H-purin-9(8H)-yl)piperidine-1-carboxylate;
9-cyclohexyl-2-(3-hydroxyphenyl)-8-oxo-8,9-dihydro-7H-purine-6-carboxamide;
9-(2-methoxyphenyl)-8-oxo-2-(3-(trifluoromethoxy)phenyl)-8,9-dihydro-7H-purine-6-carboxamide;
9-phenyl-2-(pyridin-3-yl)-9H-purine-6-carboxamide;
6-oxo-8-phenyl-2-(pyridin-3-yl)-5,6,7,8-tetrahydropteridine-4-carboxamide;
6-oxo-8-phenyl-2-(pyridin-4-yl)-5,6,7,8-tetrahydropteridine-4-carboxamide;
2-(3-aminophenyl)-9-(2-methoxyphenyl)-8-oxo-8,9-dihydro-7H-purine-6-carboxamide;
2-(3-hydroxyphenyl)-9-(2-methoxyphenyl)-9H-purine-6-carboxamide;
9-Cyclopentyl-2-(3-hydroxyphenyl)-8-oxo-8,9-dihydro-7H-purine-6-carboxamide;
9-tert-Butyl-2-(3-hydroxy-phenyl)-8-oxo-8,9-dihydo-7H-purine-6-carboxamide;
[2-(3-Hydroxyphenyl)-9-(2-methoxyphenyl)-8-oxo(7-hydropurin-6-yl)]-N-methylcarbox-amide;
2-phenyl-5H-pyrrolo[3,2-d]pyrimidine-4-carboxamide;
[2-(3-Hydroxyphenyl)-9-(2-methoxyphenyl)-8-oxo(7-hydropurin-6-yl)]-N,N-dimethyl carboxamide;
2-(3-Hydroxyphenylamino)-9-(2-methoxyphenyl)-8-oxo-8,9-dihydro-7H-purine-6-carboxamide;
2-(4-Hydroxyphenylamino)-9-(2-methoxyphenyl)-8-oxo-8,9-dihydro-7H-purine-6-carboxamide;
9-(trans-4-Hydroxycyclohexyl)-2-(3-hydroxyphenyl)-8-oxo-8,9-dihydro-7H-purine-6-carboxamide;
9-(trans-4-Hydroxycyclohexyl)-8-oxo-2-(pyridin-3-yl)-8,9-dihydro-7H-purine-6-carboxamide;
9-(trans-4-Hydroxycyclohexyl)-2-(3-hydroxyphenyl)-8-oxo-8,9-dihydro-7H-purine-6-carboxamide;
9-(trans-4-Hydroxycyclohexyl)-8-oxo-2-(pyridin-3-yl)-8,9-dihydro-7H-purine-6-carboxamide;
2-(3-Hydroxyphenylamino)-9-(2-methoxyphenyl)-9H-purine-6-carboxamide;
9-Isopropyl-2-(3-hydroxy-phenyl)-8-oxo-8,9-dihydo-7H-purine-6-carboxamide;
Methyl 4-(6-carbamoyl-9-(2-methoxyphenyl)-8-oxo-8,9-dihydro-7H-purin-2-yl) benzoate;
2-(2-Chloro-3-hydroxyphenyl)-9-(2-methoxyphenyl)-8-oxo-7-hydropurine-6-carbox amide;
2-(3-Cyanophenyl)-9-(2-methoxyphenyl)-8-oxo-8,9-dihydro-7H-purine-6-carboxamide;
2-(2-Hydroxyphenylamino)-9-(2-methoxyphenyl)-8-oxo-8,9-dihydro-7H-purine-6-carboxamide;
2-(3-Hydroxyphenyl)-9-(4-methoxy-2-methylphenyl)-8-oxo-8,9-dihydro-7H-purine-6-carboxamide;
2-(3-Hydroxyphenyl)-8-oxo-9-(2-(trifluoromethyl)phenyl)-8,9-dihydro-7H-purine-6-carboxamide;
2-(4-Cyano-phenyl)-9-(2-methoxy-phenyl)-8-oxo-8,9-dihydro-7H-purine-6-carboxamide;
4-[6-Carbamoyl-9-(2-methoxy-phenyl)-8-oxo-8,9-dihydro-7H-purin-2-yl]-benzoic acid;
Methyl 3-(6-carbamoyl-9-(2-methoxyphenyl)-8-oxo-8,9-dihydro-7H-purin-2-yl)benzoate;
3-(6-Carbamoyl-9-(2-methoxyphenyl)-8-oxo-8,9-dihydro-7H-purin-2-yl)benzoic acid;
2-(3-Hydroxyphenyl)-9-(2-isopropylphenyl)-8-oxo-8,9-dihydro-7H-purine-6-carboxamide;
2-(1H-Indazol-6-yl)-9-(2-methoxyphenyl)-8-oxo-7-hydropurine-6-carboxamide;
2-(4-Carbamoylphenyl)-9-(2-methoxyphenyl)-8-oxo-8,9-dihydro-7H-purine-6-carboxamide;
9-(2-Ethylphenyl)-2-(3-hydroxyphenyl)-8-oxo-8,9-dihydro-7H-purine-6-carboxamide;
9-(2,5-Dichlorophenyl)-2-(3-hydroxyphenyl)-8-oxo-7-hydropurine-6-carboxamide;
2-(3-Carbamoylphenyl)-9-(2-methoxyphenyl)-8-oxo-8,9-dihydro-7H-purine-6-carbox amide;
9-(2,6-Dichlorophenyl)-2-(3-hydroxyphenyl)-8-oxo-7-hydropurine-6-carboxamide;
2-(2-Hydroxyphenyl)-9-(2-methoxyphenyl)purine-6-carboxamide;
2-(1H-Indazol-5-yl)-9-(2-methoxyphenyl)-8-oxo-7-hydropurine-6-carboxamide;
9-(2,3-Dichlorophenyl)-2-(3-hydroxyphenyl)-8-oxo-7-hydropurine-6-carboxamide;
2-[4-(Hydroxymethyl)phenyl]-9-(2-methoxyphenyl)-8-oxo-7-hydropurine-6-carbox-amide;
2-[3-(Hydroxymethyl)phenyl]-9-(2-methoxyphenyl)-8-oxo-7-hydropurine-6-carbox-amide;
9-(2-Methoxyphenyl)-8-oxo-2-(pyridin-4-yl)-8,9-dihydro-7H-purine-6-carboxamide;
2-(4-Fluoro-3-hydroxyphenyl)-9-(2-methoxyphenyl)-8-oxo-7-hydropurine-6-carbox-amide;
2-(2-Fluoro-3-hydroxyphenyl)-9-(2-methoxyphenyl)-8-oxo-7-hydropurine-6-carbox-amide;
2-[4-(1-Hydroxy-isopropyl)phenyl]-9-(2-methoxyphenyl)-8-oxo-7-hydropurine-6-carboxamide;
2-[3-(1-Hydroxy-isopropyl)phenyl]-9-(2-methoxyphenyl)-8-oxo-7-hydropurine-6-carboxamide;
9-(2-Methoxyphenyl)-2-(2-nitrophenyl)-8-oxo-7-hydropurine-6-carboxamide;
9-(2-Methoxyphenyl)-2-(4-nitrophenyl)-8-oxo-7-hydropurine-6-carboxamide;
9-(2-Methoxyphenyl)-2-(2-nitrophenyl)-8-oxo-7-hydropurine-6-carboxamide;
9-(2,4-Difluorophenyl)-2-(3-hydroxyphenyl)-8-oxo-7-hydropurine-6-carboxamide;
9-(2-Methoxyphenyl)-2-{3-[(methylsulfonyl)amino]phenyl}-8-oxo-7-hydropurine-6-carboxamide;
9-(4-Chloro-2-fluorophenyl)-2-(3-hydroxyphenyl)-8-oxo-7-hydropurine-6-carboxamide;
9-(2-Chlorophenyl)-8-oxo-2-(3-pyridyl)-7-hydropurine-6-carboxamide;
8-Oxo-2-(3-pyridyl)-9-[2-(trifluoromethyl)phenyl]-7-hydropurine-6-carboxamide;
9-(3-Chloro-2-fluorophenyl)-2-(3-hydroxyphenyl)-8-oxo-7-hydropurine-6-carboxamide;
9-(2-Fluoro-3-trifluoromethylphenyl)-2-(3-hydroxyphenyl)-8-oxo-7-hydropurine-6-carboxamide;
9-(2,3,4-Trifluorophenyl)-2-(3-hydroxyphenyl)-8-oxo-7-hydropurine-6-carboxamide;
2-(1H-Benzo[d]imidazol-6-yl)-9-(2-methoxyphenyl)-8-oxo-8,9-dihydro-7H-purine-6-carboxamide;
2-[3-(Acetylamino)phenyl]-9-(2-methoxyphenyl)-8-oxo-7-hydropurine-6-carboxamide;
2-(3-hydroxyphenyl)-8-(2-methoxyphenyl)-6-oxo-5,6,7,8-tetrahydropteridine-4-carbox-amide;
9-(2-Methoxyphenyl)-8-oxo-2-pyrazol-4-yl-7-hydropurine-6-carboxamide;
9-(2-Methoxyphenyl)-8-oxo-2-pyrazol-3-yl-7-hydropurine-6-carboxamide;
9-(4-Aminocyclohexyl)-2-(3-hydroxyphenyl)-8-oxo-7-hydropurine-6-carboxamide;
2-[3-(Difluoromethyl)phenyl]-9-(2-methoxyphenyl)-8-oxo-7-hydropurine-6-carbox-amide;
2-[5-(Difluoromethyl)-2-fluorophenyl]-9-(2-methoxyphenyl)-8-oxo-7-hydropurine-6-carboxamide;
2-(1H-benzo[d]imidazol-4-yl)-9-(2-methoxyphenyl)-8-oxo-8,9-dihydro-7H-purine-6-carboxamide;
2-(6-Hydroxypyridin-3-yl)-8-oxo-9-(2-(trifluoromethyl)phenyl)-8,9-dihydro-7H-purine-6-carboxamide;
2-(1H-benzo[d]imidazol-6-yl)-9-(2-fluorophenyl)-8-oxo-8,9-dihydro-7H-purine-6-carboxamide;
2-Benzimidazol-6-yl-8-oxo-9-[2-(trifluoromethyl)phenyl]-7-hydropurine-6-carboxamide;
2-(5-Chloropyridin-3-yl)-8-oxo-9-(2-(trifluoromethyl)phenyl)-8,9-dihydro-7H-purine-6-carboxamide;
trans-4-(6-Carbamoyl-9-(2-methoxyphenyl)-8-oxo-8,9-dihydro-7H-purin-2-ylamino) cyclohexyl carbamate;
(R)-9-(2-Methoxyphenyl)-8-oxo-2-(pyrrolidin-3-ylamino)-8,9-dihydro-7H-purine-6-carboxamide;
(S)-9-(2-Methoxyphenyl)-8-oxo-2-(pyrrolidin-3-ylamino)-8,9-dihydro-7H-purine-6-carboxamide;
(cis)-4-(6-Carbamoyl-9-(2-methoxyphenyl)-8-oxo-8,9-dihydro-7H-purin-2-ylamino) cyclohexyl carbamate;
2-(trans-4-Hydroxycyclohexylamino)-9-(2-methoxyphenyl)-8-oxo-8,9-dihydro-7H-purine-6-carboxamide;
2-(4-Chloropyridin-3-yl)-8-oxo-9-(2-(trifluoromethyl)phenyl)-8,9-dihydro-7H-purine-6-carboxamide;
2-(cis-4-Hydroxycyclohexylamino)-9-(2-methoxyphenyl)-8-oxo-8,9-dihydro-7H-purine-6-carboxamide;
2-(4-((1H-Imidazol-1-yl)methyl)phenylammo)-9-(2-methoxyphenyl)-8-oxo-8,9-dihydro-7H-purine-6-carboxamide;
2-(4-Hydroxypyridin-3-yl)-8-oxo-9-(2-(trifluoromethyl)phenyl)-8,9-dihydro-7H-purine-6-carboxamide;
(R)-9-(2-Methoxyphenyl)-8-oxo-2-(pyrrolidin-2-ylmethylamino)-8,9-dihydro-7H-purine-6-carboxamide;
(S)-9-(2-Methoxyphenyl)-8-oxo-2-(pyrrolidin-2-ylmethylamino)-8,9-dihydro-7H-purine-6-carboxamide;
2-(4-(1H-1,2,4-Triazol-3-yl)phenyl)-9-(2-methoxyphenyl)-8-oxo-7-hydropurine-6-carboxamide;
2-(2-Hydroxyethylamino)-9-(2-methoxyphenyl)-8-oxo-8,9-dihydro-7H-purine-6-carboxamide ;
9-(2-Methoxyphenyl)-8-oxo-2-(2-(trifluoromethyl)-1H-benzo[d]imidazol-6-yl)-8,9-dihydro-7H-purine-6-carboxamide;
2-(3-(1H-1,2,4-Triazol-3-yl)phenyl)-9-(2-methoxyphenyl)-8-oxo-7-hydropurine-6-carboxamide;
9-(Biphenyl-2-yl)-2-(3-hydroxyphenyl)-8-oxo-8,9-dihydro-7H-purine-6-carboxamide;
2-(4-(1H-1,2,4-Triazol-3-yl)phenyl)-9-(2-fluorophenyl)-8-oxo-7-hydropurine-6-carboxamide;
2-(4-(1H-1,2,4-Triazol-3-yl)phenyl)-9-(2-isopropylphenyl)-8-oxo-8,9-dihydro-7H-purine-6-carboxamide;
9-(2-Methoxyphenyl)-2-(2-methyl-1H-benzo[d]imidazol-6-yl)-8-oxo-8,9-dihydro-7H-purine-6-carboxamide;
2-(3-(Hydroxymethyl)phenylamino)-9-(2-methoxyphenyl)-8-oxo-8,9-dihydro-7H-purine-6-carboxamide;
2-(2-(Hydroxymethyl)phenylamino)-9-(2-methoxyphenyl)-8-oxo-8,9-dihydro-7H-purine-6-carboxamide;
9-(2-tert-Butylphenyl)-2-(3-hydroxyphenyl)-8-oxo-8,9-dihydro-7H-purine-6-carboxamide;
2-(3-Hydroxyphenyl)-8-oxo-9-(2-phenoxyphenyl)-8,9-dihydro-7H-purine-6-carboxamide;
2-(1H-Benzo[d]imidazol-6-yl)-9-(2-isopropylphenyl)-8-oxo-8,9-dihydro-7H-purine-6-carboxamide;
2-(1H-Indazol-4-yl)-9-(2-methoxyphenyl)-8-oxo-8,9-dihydro-7H-purine-6-carboxamide;
2-(2-Hydroxypyridin-3-yl)-8-oxo-9-(2-(trifluoromethyl)phenyl)-8,9-dihydro-7H-purine-6-carboxamide;
2-(1H-Imidazo[4,5-b]pyridin-6-yl)-9-(2-methoxyphenyl)-8-oxo-8,9-dihydro-7H-purine-6-carboxamide;
2-(4-(1H-Imidazol-1-yl)phenyl)-9-(2-isopropylphenyl)-8-oxo-8,9-dihydro-7H-purine-6-carboxamide;
9-(2-Cyclohexylphenyl)-2-(3-hydroxyphenyl)-8-oxo-8,9-dihydro-7H-purine-6-carboxamide;
2-(4-(1H-Imidazol-2-yl)phenyl)-9-(2-isopropylphenyl)-8-oxo-8,9-dihydro-7H-purine-6-carboxamide;
2-(1H-Benzo[d]imidazol-1-yl)-9-(2-methoxyphenyl)-8-oxo-8,9-dihydro-7H-purine-6-carboxamide;
2-(1H-Imidazo[4,5-b]pyridin-6-yl)-9-(2-isopropylphenyl)-8-oxo-8,9-dihydro-7H-purine-6-carboxamide;
9-(2-Isopropylphenyl)-8-oxo-2-(1H-pyrrolo[2,3-b]pyridin-5-yl)-8,9-dihydro-7H-purine-6-carboxamide;
2-(1H-Imidazo[4,5-b]pyridin-6-yl)-8-oxo-9-(2-(trifluoromethyl)phenyl)-8,9-dihydro-7H-purine-6-carboxamide;
9-(2-Methoxyphenyl)-2-(2-(methylthio)-1H-benzo[d]imidazol-5-yl)-8-oxo-8,9-dihydro-7H-purine-6-carboxamide;
2-(1H-Indol-5-yl)-9-(2-isopropylphenyl)-8-oxo-8,9-dihydro-7H-purine-6-carboxamide;
9-(Cyclohexylmethyl)-2-(3-hydroxyphenyl)-8-oxo-8,9-dihydro-7H-purine-6-carboxamide;
9-(2,3-Dihydro-1H-inden-1-yl)-2-(3-hydroxyphenyl)-8-oxo-8,9-dihydro-7H-purine-6-carboxamide;
2-(3-Hydroxyphenyl)-9-isobutyl-8-oxo-8,9-dihydro-7H-purine-6-carboxamide;
9-(trans-4-Methoxycyclohexyl)-2-(3-hydroxyphenyl)-8-oxo-8,9-dihydro-7H-purine-6-carboxamide;
9-(cis-4-Methoxycyclohexyl)-2-(3-hydroxyphenyl)-8-oxo-8,9-dihydro-7H-purine-6-carboxamide;
2-(3-Hydroxyphenyl)-8-oxo-9-(5,6,7,8-tetrahydronaphthalen-1-yl)-8,9-dihydro-7H-purine-6-carboxamide;
2-(4-(1H-1,2,4-Triazol-3-yl)phenyl)-9-cyclohexyl-8-oxo-8,9-dihydro-7H-purine-6-carboxamide;
2-(3-Hydroxyphenyl)-9-(1H-indol-4-yl)-8-oxo-8,9-dihydro-7H-purine-6-carboxamide;
9-(2-Fluoro-3-methoxyphenyl)-2-(3-hydroxyphenyl)-8-oxo-8,9-dihydro-7H-purine-6-carboxamide;
9-(2-Fluoro-5-methoxyphenyl)-2-(3-hydroxyphenyl)-8-oxo-8,9-dihydro-7H-purine-6-carboxamide;
9-Cyclohexyl-2-(1H-imidazo[4,5-b]pyridin-6-yl)-8-oxo-8,9-dihydro-7H-purine-6-carboxamide;
2-(3-Hydroxyphenyl)-8-oxo-9-(tetrahydro-2H-pyran-4-yl)-8,9-dihydro-7H-purine-6-carboxamide;
2-(3-Hydroxyphenyl)-8-oxo-9-((tetrahydro-2H-pyran-4-yl)methyl)-8,9-dihydro-7H-purine-6-carboxamide;
9-(2-Cyclopentylphenyl)-2-(3-hydroxyphenyl)-8-oxo-8,9-dihydro-7H-purine-6-carboxamide;
2-(3-Hydroxyphenyl)-8-oxo-9-(piperidin-4-yl)-8,9-dihydro-7H-purine-6-carboxamide;
9-(2-Fluoro-4-methoxyphenyl)-2-(3-hydroxyphenyl)-8-oxo-8,9-dihydro-7H-purine-6-carboxamide;
2-(1H-benzo[d]imidazol-6-yl)-9-cyclohexyl-8-oxo-8,9-dihydro-7H-purine-6-carboxamide;
2-Benzimidazol-6-yl-9-(trans-4-methoxycyclohexyl)-8-oxo-7-hydropurine-6-carboxamide;
2-(4-(Aminomethyl)phenyl)-9-(2-methoxyphenyl)-8-oxo-8,9-dihydro-7H-purine-6-carboxamide;
2-(3-Hydroxyphenyl)-9-(cis-4-(methoxymethyl)cyclohexyl)-8-oxo-8,9-dihydro-7H-purine-6-carboxamide;
9-(trans-4-Aminocyclohexyl)-2-(3-hydroxyphenyl)-8-oxo-8,9-dihydro-7H-purine-6-carboxamide;
2-(3-Hydroxyphenyl)-9-(2-isobutylphenyl)-8-oxo-8,9-dihydro-7H-purine-6-carboxamide;
(R)-2-(3-Hydroxyphenyl)-8-oxo-9-(tetrahydrofuran-3-yl)-8,9-dihydro-7H-purine-6-carboxamide;
(S)-2-(3-Hydroxyphenyl)-8-oxo-9-(tetrahydrofuran-3-yl)-8,9-dihydro-7H-purine-6-carboxamide;
2-(3-(Aminomethyl)phenyl)-9-(2-methoxyphenyl)-8-oxo-8,9-dihydro-7H-purine-6-carboxamide;
2-(4-(1H-1,2,3-Triazol-5-yl)phenyl)-9-(2-isopropylphenyl)-8-oxo-8,9-dihydro-7H-purine-6-carboxamide;
2-(4-(1H-1,2,4-Triazol-3-yl)phenyl)-9-(cis-4-methoxycyclohexyl)-8-oxo-8,9-dihydro-7H-purine-6-carboxamide;
2-(1H-Benzo[d]imidazol-6-yl)-9-(cis-4-methoxycyclohexyl)-8-oxo-8,9-dihydro-7H-purine-6-carboxamide;
2-(1H-Imidazo[4,5-b]pyridin-6-yl)-9-(cis-4-methoxycyclohexyl)-8-oxo-8,9-dihydro-7H-purine-6-carboxamide;
2-(3-Hydroxyphenyl)-9-((1r,4r)-4-(methoxymethyl)cyclohexyl)-8-oxo-8,9-dihydro-7H-purine-6-carboxamide; and
9-(2-Isopropylphenyl)-2-(4-(5-methyl-4H-1,2,4-triazol-3-yl)phenyl)-8-oxo-8,9-dihydro-7H-purine-6-carboxamide,
and pharmaceutically acceptable salts, clathrates, solvates, stereoisomers, tautomers, and prodrugs thereof.

As also described herein, the TOR kinase inhibitors may include compounds having the following formula (III): 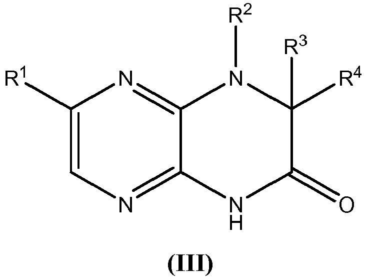 and pharmaceutically acceptable salts, clathrates, solvates, stereoisomers, tautomers, and prodrugs thereof, wherein:
R¹ is substituted or unsubstituted C₁₋₈ alkyl, substituted or unsubstituted aryl, substituted or unsubstituted cycloalkyl, substituted or unsubstituted heterocyclyl, or substituted or unsubstituted heterocyclylalkyl;
R² is H, substituted or unsubstituted C₁₋₈ alkyl, substituted or unsubstituted cycloalkyl, substituted or unsubstituted heterocyclyl, substituted or unsubstituted heterocyclylalkyl, substituted or unsubstituted aralkyl, or substituted or unsubstituted cycloalkylalkyl;
R³ and R⁴ are each independently H, substituted or unsubstituted C₁₋₈ alkyl, substituted or unsubstituted aryl, substituted or unsubstituted cycloalkyl, substituted or unsubstituted heterocyclyl, substituted or unsubstituted heterocyclylalkyl, substituted or unsubstituted aralkyl, substituted or unsubstituted cycloalkylalkyl, or R³ and R⁴, together with the atoms to which they are attached, form a substituted or unsubstituted cycloalkyl or substituted or unsubstituted heterocyclyl;
or R² and one of R³ and R⁴, together with the atoms to which they are attached, form a substituted or unsubstituted heterocyclyl,
wherein, the TOR kinase inhibitors may not include the compounds depicted below, namely: 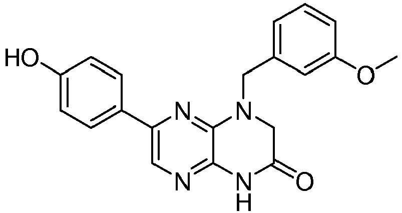 6-(4-hydroxyphenyl)-4-(3-methoxybenzyl)-3,4-dihydropyrazino[2,3-b]pyrazin-2(1H)-one; 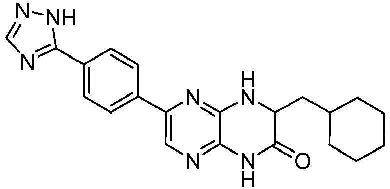 6-(4-(1H-1,2,4-triazol-5-yl)phenyl)-3-(cyclohexylmethyl)-3,4-dihydropyrazino[2,3-b]pyrazin-2(1H)-one;
or, 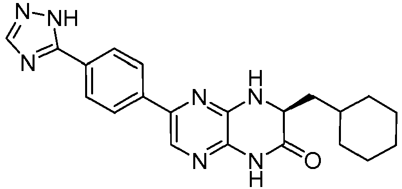 (R)-6-(4-(1H-1,2,4-triazol-5-yl)phenyl)-3-(cyclohexylmethyl)-3,4-dihydropyrazino[2,3-b]pyrazin-2(1H)-one.

In compounds of formula (III), R¹ may be substituted or unsubstituted aryl or substituted or unsubstituted heteroaryl. In one embodiment, R¹ is phenyl, pyridyl, pyrimidyl, benzimidazolyl, indolyl, indazolyl, 1H-pyrrolo[2,3-b]pyridyl, 1H-imidazo[4,5-b]pyridyl, 1H-imidazo[4,5-b]pyridin-2(3H)-onyl, 3H-imidazo[4,5-b]pyridyl, or pyrazolyl, each optionally substituted. R¹ may be phenyl substituted with one or more substituents independently selected from the group consisting of substituted or unsubstituted C₁₋₈ alkyl (for example, methyl), substituted or unsubstituted heterocyclyl (for example, substituted or unsubstituted triazolyl or pyrazolyl), halogen (for example, fluorine), aminocarbonyl, cyano, hydroxyalkyl (for example, hydroxypropyl), and hydroxy. R¹ may be pyridyl substituted with one or more substituents independently selected from the group consisting of substituted or unsubstituted C₁₋₈ alkyl, substituted or unsubstituted heterocyclyl (for example, substituted or unsubstituted triazolyl), halogen, aminocarbonyl, cyano, hydroxyalkyl, -OR, and -NR₂, wherein each R is independently H, or a substituted or unsubstituted C₁₋₄ alkyl. R¹ may be 1H-pyrrolo[2,3-b]pyridyl or benzimidazolyl, each optionally substituted with one or more substituents independently selected from the group consisting of substituted or unsubstituted C₁₋₈ alkyl, and -NR₂, wherein each R is independently H, or a substituted or unsubstituted C₁₋₄ alkyl.

In compounds of formula (III), R¹ may be 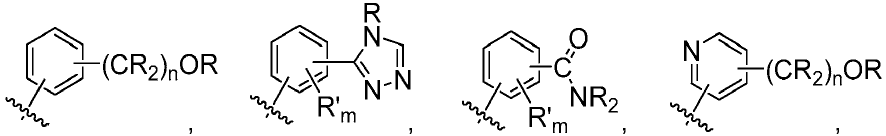 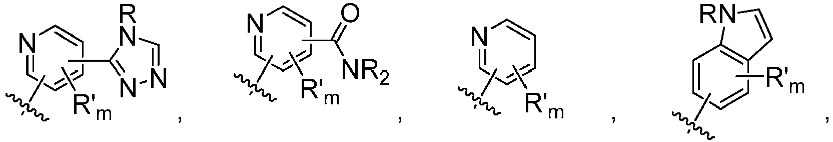 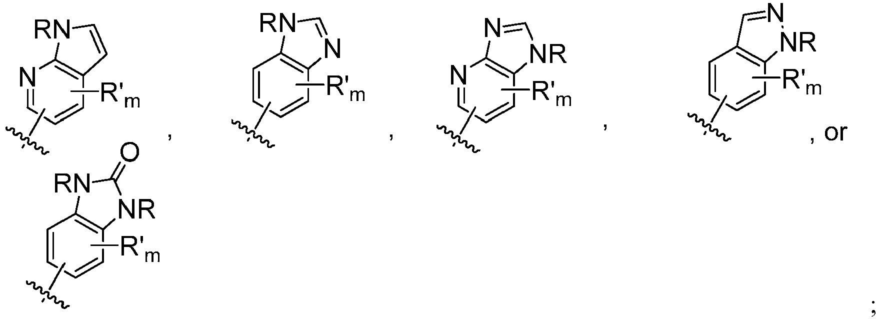 wherein R may be at each occurrence independently H, or a substituted or unsubstituted C₁₋₄ alkyl (for example, methyl); R' may be at each occurrence independently a substituted or unsubstituted C₁₋₄ alkyl, halogen (for example, fluorine), cyano, -OR, or -NR₂; m is 0-3; and n is 0-3. It will be understood by those skilled in the art that any of the subsitutuents R' may be attached to any suitable atom of any of the rings in the fused ring systems. It will also be understood by those skilled in the art that the connecting bond of R¹ (designated by the bisecting wavy line) may be attached to any of the atoms in any of the rings in the fused ring systems.

In compounds of formula (III), R¹ may be 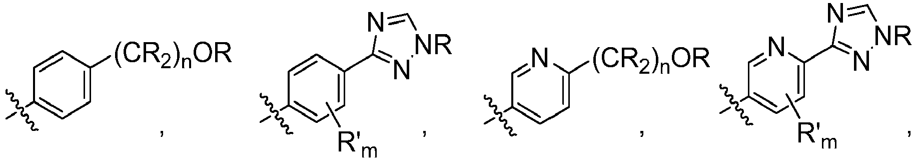 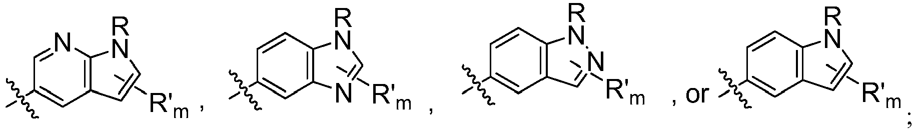 wherein R may be at each occurrence independently H, or a substituted or unsubstituted C₁₋₄ alkyl; R' may be at each occurrence independently a substituted or unsubstituted C₁₋₄ alkyl, halogen, cyano, -OR, or -NR₂; m may be 0-3; and n may be 0-3.

In compounds of formula (III), R² may be H, substituted or unsubstituted C₁₋₈ alkyl, substituted or unsubstituted cycloalkyl, substituted or unsubstituted heterocyclyl, substituted or unsubstituted C₁₋₄ alkyl-heterocyclyl, substituted or unsubstituted C₁₋₄ alkyl-aryl, or substituted or unsubstituted C₁₋₄ alkyl-cycloalkyl. For example, R² may be H, methyl, ethyl, n-propyl, isopropyl, n-butyl, *sec*-butyl, isobutyl, *tert*-butyl, n-pentyl, isopentyl, cyclopentyl, cyclohexyl, tetrahydrofuranyl, tetrahydropyranyl, (C₁₋₄ alkyl)-phenyl, (C₁₋₄ alkyl)-cyclopropyl, (C₁₋₄ alkyl)-cyclobutyl, (C₁₋₄ alkyl)-cyclopentyl, (C₁₋₄ alkyl)-cyclohexyl, (C₁₋₄ alkyl)-pyrrolidyl, (C₁₋₄ alkyl)-piperidyl, (C₁₋₄ alkyl)-piperazinyl, (C₁₋₄ alkyl)-morpholinyl, (C₁₋₄ alkyl)-tetrahydrofuranyl, or (C₁₋₄ alkyl)-tetrahydropyranyl, each optionally substituted.

R² may be H, C₁₋₄ alkyl, (C₁₋₄alkyl)(OR), 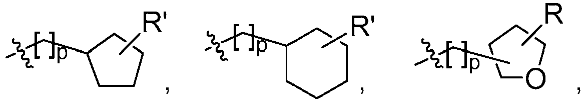 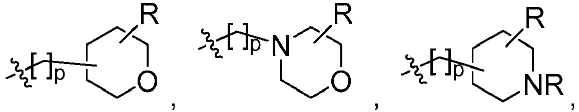 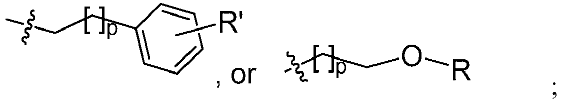 wherein R at each occurrence independently H, or a substituted or unsubstituted C₁₋₄ alkyl (for example, methyl); R' may be at each occurrence independently H, -OR, cyano, or a substituted or unsubstituted C₁₋₄ alkyl (for example, methyl); and p may be 0-3.

R² may be H, C₁₋₄ alkyl, (C₁₋₄alkyl)(OR), 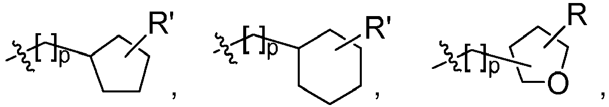 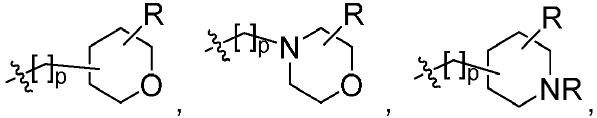 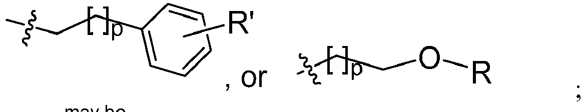 wherein R may be at each occurrence independently H, or a substituted or unsubstituted C₁₋₂ alkyl; R' may be at each occurrence independently H, -OR, cyano, or a substituted or unsubstituted C₁₋₂ alkyl; and p may be 0-1.

In some compounds of formula (III), R² and one of R³ and R⁴ together with the atoms to which they are attached may form a substituted or unsubstituted heterocyclyl. For example, the compound of formula (III) may be 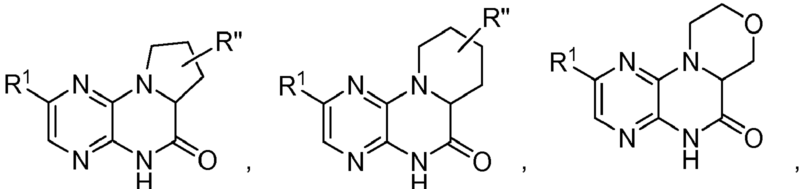 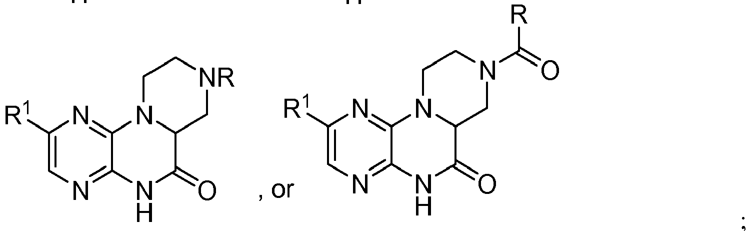 wherein R may be at each occurrence independently H, or a substituted or unsubstituted C₁₋₄ alkyl; R" may be H, OR, or a substituted or unsubstituted C₁₋₄ alkyl; and R¹ may be as defined herein.

In compounds of formula (III), R³ and R⁴ may be both H. In others, one of R³ and R⁴ may be and the other other than H. In still others, one of R³ and R⁴ may be C₁₋₄ alkyl (for example, methyl) and the other may be. In still others, both of R³ and R4 may be are C₁₋₄ alkyl (for example, methyl).

R¹ may be substituted or unsubstituted aryl, or substituted or unsubstituted heteroaryl. For example, R¹ may be phenyl, pyridyl, pyrimidyl, benzimidazolyl, indolyl, indazolyl, 1H-pyrrolo[2,3-b]pyridyl, 1H-imidazo[4,5-b]pyridyl, 1H-imidazo[4,5-b]pyridin-2(3H)-onyl, 3H-imidazo[4,5-b]pyridyl, or pyrazolyl, each optionally substituted. R¹ may be phenyl substituted with one or more substituents independently selected from the group consisting of substituted or unsubstituted C₁₋₈ alkyl, substituted or unsubstituted heterocyclyl, halogen, aminocarbonyl, cyano, hydroxyalkyl and hydroxy. In others, R¹ may be pyridyl substituted with one or more substituents independently selected from the group consisting of cyano, substituted or unsubstituted C₁₋₈ alkyl, substituted or unsubstituted heterocyclyl, hydroxyalkyl, halogen, aminocarbonyl, -OR, and -NR₂, wherein each R may be independently H, or a substituted or unsubstituted C₁₋₄ alkyl. In others, R¹ may be 1H-pyrrolo[2,3-b]pyridyl or benzimidazolyl, optionally substituted with one or more substituents independently selected from the group consisting of substituted or unsubstituted C₁₋₈ alkyl, and -NR₂, wherein R may be independently H, or a substituted or unsubstituted C₁₋₄ alkyl

In compounds of formula (III) may have an R¹ group set forth herein and an R² group set forth herein.

In compounds of formula (III), the compound at a concentration of 10 µM may inhibit mTOR, DNA-PK, or PI3K or a combination thereof, by at least about 50%. Compounds of formula (III) may be shown to be inhibitors of the kinases above in any suitable assay system.

Representative TOR kinase inhibitors of formula (III) may include:
6-(1H-pyrrolo[2,3-b]pyridin-3-yl)-4-(2-(tetrahydro-2H-pyran-4-yl)ethyl)-3,4-dihydropyrazino[2,3-b]pyrazin-2(1H)-one;
6-(4-methyl-6-(1H-1,2,4-triazol-3-yl)pyridin-3-yl)-4-((tetrahydro-2H-pyran-4-yl)methyl)-3 ,4-dihydropyrazino[2,3-b]pyrazin-2(1H)-one;
6-(5-fluoro-2-methyl-4-(1H-1,2,4-triazol-3-yl)phenyl)-4-((*trans*-4-methoxycyclohexyl)methyl)-3,4-dihydropyrazino[2,3-b]pyrazin-2(1H)-one;
6-(5-fluoro-2-methyl-4-(1H-1,2,4-triazol-3-yl)phenyl)-4-((*cis*-4-methoxycyclohexyl)methyl)-3,4-dihydropyrazino[2,3-b]pyrazin-2(1H)-one;
6-(6-(1H-1,2,4-triazol-3-yl)pyridin-3-yl)-4-((*trans*-4-methoxycyclohexyl)methyl)-3,4-dihydropyrazino[2,3-b]pyrazin-2(1H)-one;
6-(5-fluoro-2-methyl-4-(1H-1,2,4-triazol-3-yl)phenyl)-4-((*trans*-4-hydroxycyclohexyl)methyl)-3,4-dihydropyrazino[2,3-b]pyrazin-2(1H)-one;
6-(6-(1H-1,2,4-triazol-3-yl)pyridin-3-yl)-4-((*cis*-4-methoxycyclohexyl)methyl)-3,4-dihydropyrazino[2,3-b]pyrazin-2(1H)-one;
6-(6-(1H-1,2,4-triazol-3-yl)pyridin-3-yl)-4-((*trans*-4-hydroxycyclohexyl)methyl)-3,4-dihydropyrazino[2,3-b]pyrazin-2(1H)-one;
6-(6-(1H-1,2,4-triazol-3-yl)pyridin-3-yl)-4-(cis-4-hydroxycyclohexyl)-3,4-dihydropyrazino[2,3-b]pyrazin-2(1H)-one;
6-(6-(1H-1,2,4-triazol-3-yl)pyridin-3-yl)-4-((*cis*-4-hydroxycyclohexyl)methyl)-3,4-dihydropyrazino[2,3-b]pyrazin-2(1H)-one;
6-(5-fluoro-2-methyl-4-(1H-1,2,4-triazol-3-yl)phenyl)-4-(*trans*-4-methoxycyclohexyl)-3,4-dihydropyrazino[2,3-b]pyrazin-2(1H)-one;
6-(6-(1H-1,2,4-triazol-3-yl)pyridin-3-yl)-4-(*trans*-4-methoxycyclohexyl)-3,4-dihydropyrazino[2,3-b]pyrazin-2(1H)-one;
6-(6-(1H-1,2,4-triazol-3-yl)pyridin-3-yl)-4-(*trans*-4-hydroxycyclohexyl)-3,4-dihydropyrazino[2,3-b]pyrazin-2(1H)-one;
6-(5-fluoro-2-methyl-4-(1H-1,2,4-triazol-3-yl)phenyl)-4-((*cis*-4-hydroxycyclohexyl)methyl)-3,4-dihydropyrazino[2,3-b]pyrazin-2(1H)-one;
6-(6-(1H-1,2,4-triazol-3-yl)pyridin-3-yl)-4-(*cis*-4-methoxycyclohexyl)-3,4-dihydropyrazino[2,3-b]pyrazin-2(1H)-one;
6-(6-(1H-1,2,4-triazol-3-yl)pyridin-3-yl)-4-(2-methoxyethyl)-3,4-dihydropyrazino[2,3-b]pyrazin-2(1H)-one;
6-(6-(1H-1,2,4-triazol-3-yl)pyridin-3-yl)-4-isopropyl-3,4-dihydropyrazino[2,3-b]pyrazin-2(1H)-one;
6-(5-fluoro-2-methyl-4-(1H-1,2,4-triazol-3-yl)phenyl)-4-(*cis*-4-hydroxycyclohexyl)-3,4-dihydropyrazino[2,3-b]pyrazin-2(1H)-one;
6-(5-fluoro-2-methyl-4-(1H-1,2,4-triazol-3-yl)phenyl)-4-(*cis*-4-methoxycyclohexyl)-3,4-dihydropyrazino[2,3-b]pyrazin-2(1H)-one;
6-(5-fluoro-2-methyl-4-(1H-1,2,4-triazol-3-yl)phenyl)-4-(2-methoxyethyl)-3,4-dihydropyrazino[2,3-b]pyrazin-2(1H)-one;
6-(6-(1H-1,2,4-triazol-3-yl)pyridin-3-yl)-4-(tetrahydro-2H-pyran-4-yl)-3,4-dihydropyrazino[2,3-b]pyrazin-2(1H)-one;
6-(6-(1H-1,2,4-triazol-3-yl)pyridin-3-yl)-4-ethyl-3,4-dihydropyrazino[2,3-b]pyrazin-2(1H)-one;
6-(5-fluoro-2-methyl-4-(1H-1,2,4-triazol-3-yl)phenyl)-4-(*trans*-4-hydroxycyclohexyl)-3,4-dihydropyrazino[2,3-b]pyrazin-2(1H)-one;
6-(5-fluoro-2-methyl-4-(1H-1,2,4-triazol-3-yl)phenyl)-4-(tetrahydro-2H-pyran-4-yl)-3,4-dihydropyrazino[2,3-b]pyrazin-2(1H)-one;
6-(5-fluoro-2-methyl-4-(1H-1,2,4-triazol-3-yl)phenyl)-4-isopropyl-3,4-dihydropyrazino[2,3-b]pyrazin-2(1H)-one;
4-ethyl-6-(5-fluoro-2-methyl-4-(1H-1,2,4-triazol-3-yl)phenyl)-3,4-dihydropyrazino[2,3-b]pyrazin-2(1H)-one;
6-(3-fluoro-2-methyl-4-(1H-1,2,4-triazol-3-yl)phenyl)-4-(tetrahydro-2H-pyran-4-yl)-3,4-dihydropyrazino[2,3-b]pyrazin-2(1H)-one;
6-(3-fluoro-2-methyl-4-(1H-1,2,4-triazol-3-yl)phenyl)-4-(cis-4-methoxycyclohexyl)-3,4-dihydropyrazino[2,3-b]pyrazin-2(1H)-one;
6-(3-fluoro-2-methyl-4-(1H-1,2,4-triazol-3-yl)phenyl)-4-(*trans*-4-methoxycyclohexyl)-3,4-dihydropyrazino[2,3-b]pyrazin-2(1H)-one;
4-(2-methoxyethyl)-6-(4-methyl-6-(1H-1,2,4-triazol-3-yl)pyridin-3-yl)-3,4-dihydropyrazino[2,3-b]pyrazin-2(1H)-one;
6-(3-(1H-1,2,4-triazol-5-yl)phenyl)-4-(2-(tetrahydro-2H-pyran-4-yl)ethyl)-3,4-dihydropyrazino[2,3-b]pyrazin-2(1H)-one;
5-(8-(2-methoxyethyl)-6-oxo-5,6,7,8-tetrahydropyrazino[2,3-b]pyrazin-2-yl)-4-methylpicolinamide;
3-(6-oxo-8-(2-(tetrahydro-2H-pyran-4-yl)ethyl)-5,6,7,8-tetrahydropyrazino[2,3-b]pyrazin-2-yl)benzamide;
3-(6-oxo-8-(2-(tetrahydro-2H-pyran-4-yl)ethyl)-5,6,7,8-tetrahydropyrazino[2,3-b]pyrazin-2-yl)benzonitrile;
5-(8-(*trans*-4-methoxycyclohexyl)-6-oxo-5,6,7,8-tetrahydropyrazino[2,3-b]pyrazin-2-yl)-4-methylpicolinamide;
6-(1H-imidazo[4,5-b]pyridin-6-yl)-4-(2-(tetrahydro-2H-pyran-4-yl)ethyl)-3,4-dihydropyrazino[2,3-b]pyrazin-2(1H)-one;
6-(1H-indazol-6-yl)-4-(2-(tetrahydro-2H-pyran-4-yl)ethyl)-3,4-dihydropyrazmo[2,3-b]pyrazin-2(1H)-one;
4-((1R,3S)-3-methoxycyclopentyl)-6-(2-methyl-6-(4H-1,2,4-triazol-3-yl)pyridin-3-yl)-3,4-dihydropyrazino[2,3-b]pyrazin-2(1H)-one;
4-((1S,3R)-3-methoxycyclopentyl)-6-(2-methyl-6-(4H-1,2,4-triazol-3-yl)pyridin-3-yl)-3,4-dihydropyrazino[2,3-b]pyrazin-2(1H)-one;
4-((1R,3R)-3-methoxycyclopentyl)-6-(2-methyl-6-(4H-1,2,4-triazol-3-yl)pyridin-3-yl)-3,4-dihydropyrazino[2,3-b]pyrazin-2(1H)-one;
4-((1S,3S)-3-methoxycyclopentyl)-6-(2-methyl-6-(4H-1,2,4-triazol-3-yl)pyridin-3-yl)-3,4-dihydropyrazino[2,3-b]pyrazin-2(1H)-one;
4-ethyl-6-(2-methyl-6-(4H-1,2,4-triazol-3-yl)pyridin-3-yl)-3,4-dihydropyrazino[2,3-b]pyrazin-2(1H)-one;
6-(1H-pyrrolo[2,3-b]pyridin-5-yl)-4-(2-(tetrahydro-2H-pyran-4-yl)ethyl)-3,4-dihydropyrazino[2,3-b]pyrazin-2(1H)-one;
6-(1H-indol-6-yl)-4-(2-(tetrahydro-2H-pyran-4-yl)ethyl)-3,4-dihydropyrazino[2,3-b]pyrazin-2(1H)-one;
6-(1H-indol-5-yl)-4-(2-(tetrahydro-2H-pyran-4-yl)ethyl)-3,4-dihydropyrazino[2,3-b]pyrazin-2(1H)-one;
4-(((1R,3S)-3-methoxycyclopentyl)methyl)-6-(2-methyl-6-(4H-1,2,4-triazol-3-yl)pyridin-3-yl)-3,4-dihydropyrazino[2,3-b]pyrazin-2(1H)-one;
4-(((1S,3R)-3-methoxycyclopentyl)methyl)-6-(2-methyl-6-(4H-1,2,4-triazol-3-yl)pyridin-3-yl)-3,4-dihydropyrazino[2,3-b]pyrazin-2(1H)-one;
6-(3-fluoro-2-methyl-4-(4H-1,2,4-triazol-3-yl)phenyl)-4-(2-(tetrahydro-2H-pyran-4-yl)ethyl)-3,4-dihydropyrazino[2,3-b]pyrazin-2(1H)-one;
6-(3-fluoro-2-methyl-4-(4H-1,2,4-triazol-3-yl)phenyl)-4-(2-methoxyethyl)-3,4-dihydropyrazino[2,3-b]pyrazin-2(1H)-one;
3,3-dimethyl-6-(4-methyl-6-(4H-1,2,4-triazol-3-yl)pyridin-3-yl)-4-((tetrahydro-2H-pyran-4-yl)methyl)-3,4-dihydropyrazino[2,3-b]pyrazin-2(1H)-one;
6-(6-(2-hydroxypropan-2-yl)pyridin-3-yl)-4-((1R,3S)-3-methoxycyclopentyl)-3,4-dihydropyrazino[2,3-b]pyrazin-2(1H)-one;
6-(6-(2-hydroxypropan-2-yl)pyridin-3-yl)-4-((1S,3R)-3-methoxycyclopentyl)-3,4-dihydropyrazino[2,3-b]pyrazin-2(1H)-one;
6-(6-(2-hydroxypropan-2-yl)pyridin-3-yl)-4-(((1S,3S)-3-methoxycyclopentyl)methyl)-3,4-dihydropyrazino[2,3-b]pyrazin-2(1H)-one;
6-(6-(2-hydroxypropan-2-yl)pyridin-3-yl)-4-(((1R,3R)-3-methoxycyclopentyl)methyl)-3,4-dihydropyrazino[2,3-b]pyrazin-2(1H)-one;
6-(6-(2-hydroxypropan-2-yl)pyridin-3-yl)-4-((1S,3S)-3-methoxycyclopentyl)-3,4-dihydropyrazino[2,3-b]pyrazin-2(1H)-one;
6-(6-(2-hydroxypropan-2-yl)pyridin-3-yl)-4-((1R,3R)-3-methoxycyclopentyl)-3,4-dihydropyrazino[2,3-b]pyrazin-2(1H)-one;
6-(6-(2-hydroxypropan-2-yl)pyridin-3-yl)-4-(((1R,3S)-3-methoxycyclopentyl)methyl)-3,4-dihydropyrazino[2,3-b]pyrazin-2(1H)-one;
6-(6-(2-hydroxypropan-2-yl)pyridin-3-yl)-4-(((1S,3R)-3-methoxycyclopentyl)methyl)-3,4-dihydropyrazino[2,3-b]pyrazin-2(1H)-one;
6-(3-fluoro-4-(4H-1,2,4-triazol-3-yl)phenyl)-4-(2-methoxyethyl)-3,4-dihydropyrazino[2,3-b]pyrazin-2(1H)-one;
6-(3-fluoro-4-(4H-1,2,4-triazol-3-yl)phenyl)-4-(2-(tetrahydro-2H-pyran-4-yl)ethyl)-3,4-dihydropyrazino[2,3-b]pyrazin-2(1H)-one;
7'-(2-methyl-4-(4H-1,2,4-triazol-3-yl)phenyl)-1'-((tetrahydro-2H-pyran-4-yl)methyl)-1'H-spiro[cyclopentane-1,2'-pyrazino[2,3-b]pyrazin]-3'(4'H)-one;
7'-(2-methyl-4-(4H-1,2,4-triazol-3-yl)phenyl)-1'-((tetrahydro-2H-pyran-4-yl)methyl)-1'H-spiro[cyclobutane-1,2'-pyrazino[2,3-b]pyrazin]-3'(4'H)-one;
4-(cyclopropylmethyl)-6-(6-(2-hydroxypropan-2-yl)pyridin-3-yl)-3,4-dihydropyrazino[2,3-b]pyrazin-2(1H)-one;
7'-(2-methyl-4-(4H-1,2,4-triazol-3-yl)phenyl)-1'H-spiro[cyclopentane-1,2'-pyrazino[2,3-b]pyrazin]-3'(4'H)-one;
7'-(2-methyl-4-(4H-1,2,4-triazol-3-yl)phenyl)-1'H-spiro[cyclobutane-1,2'-pyrazino[2,3-b]pyrazin]-3'(4'H)-one;
7'-(2-methyl-4-(4H-1,2,4-triazol-3-yl)phenyl)-1'H-spiro[cyclopropane-1,2'-pyrazino[2,3-b]pyrazin] -3 '(4'H)-one;
(R)-6-(4-(4H-1,2,4-triazol-3-yl)phenyl)-4-((tetrahydrofuran-2-yl)methyl)-3,4-dihydropyrazino[2,3-b]pyrazin-2(1H)-one;
(S)-6-(4-(4H-1,2,4-triazol-3-yl)phenyl)-4-((tetrahydrofuran-2-yl)methyl)-3,4-dihydropyrazino[2,3-b]pyrazin-2(1H)-one;
6-(1H-indazol-5-yl)-4-(2-(tetrahydro-2H-pyran-4-yl)ethyl)-3,4-dihydropyrazino[2,3-b]pyrazin-2(1H)-one;
4-(6-oxo-8-(2-(tetrahydro-2H-pyran-4-yl)ethyl)-5,6,7,8-tetrahydropyrazino[2,3-b]pyrazin-2-yl)benzamide;
4-(2-methoxyethyl)-3,3-dimethyl-6-(2-methyl-4-(4H-1,2,4-triazol-3-yl)phenyl)-3,4-dihydropyrazino[2,3-b]pyrazin-2(1H)-one;
4-ethyl-3,3-dimethyl-6-(2-methyl-4-(4H-1,2,4-triazol-3-yl)phenyl)-3,4-dihydropyrazino[2,3-b]pyrazin-2(1H)-one;
6-(2-methyl-4-(4H-1,2,4-triazol-3-yl)phenyl)-3,4-dihydropyrazino[2,3-b]pyrazin-2(1H)-one;
3,3-dimethyl-6-(2-methyl-6-(4H-1,2,4-triazol-3-yl)pyridin-3-yl)-4-((tetrahydro-2H-pyran-4-yl)methyl)-3,4-dihydropyrazino[2,3-b]pyrazin-2(1H)-one;
(R)-6-(6-(1-hydroxyethyl)pyridin-3-yl)-4-(2-(tetrahydro-2H-pyran-4-yl)ethyl)-3,4-dihydropyrazino[2,3-b]pyrazin-2(1H)-one;
3,3-dimethyl-6-(2-methyl-4-(4H-1,2,4-triazol-3-yl)phenyl)-4-((tetrahydro-2H-pyran-4-yl)methyl)-3,4-dihydropyrazino[2,3-b]pyrazin-2(1H)-one;
6-(6-(2-hydroxypropan-2-yl)-4-methylpyridin-3-yl)-4-(*trans*-4-methoxycyclohexyl)-3,4-dihydropyrazino[2,3-b]pyrazin-2(1H)-one;
6-(6-(2-hydroxypropan-2-yl)-4-methylpyridin-3-yl)-4-((tetrahydro-2H-pyran-4-yl)methyl)-3,4-dihydropyrazino[2,3-b]pyrazin-2(1H)-one;
3,3-dimethyl-6-(2-methyl-4-(4H-1,2,4-triazol-3-yl)phenyl)-3,4-dihydropyrazino[2,3-b]pyrazin-2(1H)-one;
3,3-dimethyl-6-(2-methyl-6-(4H-1,2,4-triazol-3-yl)pyridin-3-yl)-4-(2-(tetrahydro-2H-pyran-4-yl)ethyl)-3,4-dihydropyrazino[2,3-b]pyrazin-2(1H)-one;
6-(6-(2-hydroxypropan-2-yl)-2-methylpyridin-3-yl)-4-((tetrahydro-2H-pyran-4-yl)methyl)-3,4-dihydropyrazino[2,3-b]pyrazin-2(1H)-one;
6-(6-(2-hydroxypropan-2-yl)-2-methylpyridin-3-yl)-4-(*trans*-4-methoxycyclohexyl)-3,4-dihydropyrazino[2,3-b]pyrazin-2(1H)-one;
(S)-6-(6-(1-hydroxyethyl)pyridin-3-yl)-4-(2-(tetrahydro-2H-pyran-4-yl)ethyl)-3,4-dihydropyrazino[2,3-b]pyrazin-2(1H)-one;
3,3-dimethyl-6-(2-methyl-4-(4H-1,2,4-triazol-3-yl)phenyl)-4-(2-(tetrahydro-2H-pyran-4-yl)ethyl)-3,4-dihydropyrazino[2,3-b]pyrazin-2(1H)-one;
6-(6-(2-hydroxypropan-2-yl)pyridin-3-yl)-3,3-dimethyl-4-(2-(tetrahydro-2H-pyran-4-yl)ethyl)-3,4-dihydropyrazino[2,3-b]pyrazin-2(1H)-one;
6-(4-(2-hydroxypropan-2-yl)phenyl)-4-(*trans*-4-methoxycyclohexyl)-3,4-dihydropyrazino[2,3-b]pyrazin-2(1H)-one;
6-(4-(2-hydroxypropan-2-yl)phenyl)-4-((*trans*-4-methoxycyclohexyl)methyl)-3,4-dihydropyrazino[2,3-b]pyrazin-2(1H)-one;
4-(*cis*-4-methoxycyclohexyl)-6-(2-methyl-6-(4H-1,2,4-triazol-3-yl)pyridin-3-yl)-3,4-dihydropyrazino[2,3-b]pyrazin-2(1H)-one;
4-(*trans*-4-methoxycyclohexyl)-6-(2-methyl-6-(4H-1,2,4-triazol-3-yl)pyridin-3-yl)-3,4-dihydropyrazino[2,3-b]pyrazin-2(1H)-one;
6-(4-(2-hydroxypropan-2-yl)phenyl)-4-((tetrahydro-2H-pyran-4-yl)methyl)-3,4-dihydropyrazino[2,3-b]pyrazin-2(1H)-one;
4-(2-methoxyethyl)-6-(2-methyl-6-(4H-1,2,4-triazol-3-yl)pyridin-3-yl)-3,4-dihydropyrazino[2,3-b]pyrazin-2(1H)-one;
9-(6-(4H-1,2,4-triazol-3-yl)-3-pyridyl)-6,11,4a-trihydromorpholino[4,3-e]pyrazino[2,3-b]pyrazin-5-one;
6-(2-methyl-6-(4H-1,2,4-triazol-3-yl)pyridin-3-yl)-4-((tetrahydro-2H-pyran-4-yl)methyl)-3,4-dihydropyrazino[2,3-b]pyrazin-2(1H)-one;
5-(8-(*cis*-4-methoxycyclohexyl)-6-oxo-5,6,7,8-tetrahydropyrazino[2,3-b]pyrazin-2-yl)-6-methylpicolinonitrile;
6-(6-(4H-1,2,4-triazol-3-yl)pyridin-3-yl)-4-(2-(tetrahydro-2H-pyran-4-yl)ethyl)-3,4-dihydropyrazino[2,3-b]pyrazin-2(1H)-one;
9-(4-(4H-1,2,4-triazol-3-yl)-2-methylphenyl)-3-(2-methoxyacetyl)-6,11,4a-trihydropiperazino[1,2-e]pyrazino[2,3-b]pyrazin-5-one;
9-(4-(4H-1,2,4-triazol-3-yl)-2-methylphenyl)-6,11,4a-trihydropiperazino[1,2-e]pyrazino[2,3 -b]pyrazin-5 -one;
9-(4-(4H-1,2,4-triazol-3-yl)-2-methylphenyl)-3-(2-methoxyethyl)-6,11,4a-trihydropiperazino[1,2-e]pyrazino[2,3-b]pyrazin-5-one;
4-(cyclopentylmethyl)-6-(2-methyl-6-(4H-1,2,4-triazol-3-yl)pyridin-3-yl)-3,4-dihydropyrazino[2,3-b]pyrazin-2(1H)-one;
9-(6-(4H-1,2,4-triazol-3-yl)-2-methyl-3-pyridyl)-6,11,4a-trihydromorpholino[4,3-e]pyrazino[2,3 -b]pyrazin-5 -one;
4-(*trans*-4-hydroxycyclohexyl)-6-(6-(2-hydroxypropan-2-yl)pyridin-3-yl)-3,4-dihydropyrazino[2,3-b]pyrazin-2(1H)-one;
4-(*cis*-4-hydroxycyclohexyl)-6-(6-(2-hydroxypropan-2-yl)pyridin-3-yl)-3,4-dihydropyrazino[2,3-b]pyrazin-2(1H)-one;
6-(6-(2-hydroxypropan-2-yl)pyridin-3-yl)-4-((tetrahydrofuran-3-yl)methyl)-3,4-dihydropyrazino[2,3-b]pyrazin-2(1H)-one;
4-(cyclopentylmethyl)-6-(6-(2-hydroxypropan-2-yl)pyridin-3-yl)-3,4-dihydropyrazino[2,3-b]pyrazin-2(1H)-one;
6-(6-(2-hydroxypropan-2-yl)pyridin-3-yl)-4-neopentyl-3,4-dihydropyrazino[2,3-b]pyrazin-2(1H)-one;
6-(6-(2-hydroxypropan-2-yl)pyridin-3-yl)-4-isobutyl-3,4-dihydropyrazino[2,3-b]pyrazin-2(1H)-one;
3-methyl-6-(2-methyl-4-(4H-1,2,4-triazol-3-yl)phenyl)-4-(2-(tetrahydro-2H-pyran-4-yl)ethyl)-3,4-dihydropyrazino[2,3-b]pyrazin-2(1H)-one;
6-(6-(2-hydroxypropan-2-yl)pyridin-3-yl)-4-(piperidin-4-yl)-3,4-dihydropyrazino[2,3-b]pyrazin-2(1H)-one;
6-(6-(2-hydroxypropan-2-yl)pyridin-3-yl)-4-(2-(tetrahydro-2H-pyran-3-yl)ethyl)-3,4-dihydropyrazino[2,3-b]pyrazin-2(1H)-one;
8-(4-(4H-1,2,4-triazol-3-yl)-2-methylphenyl)(3aS,2R)-2-methoxy-5,10,3a-trihydropyrazino[2,3-b]pyrrolidino[1,2-e]pyrazin-4-one;
8-(4-(4H-1,2,4-triazol-3-yl)-2-methylphenyl)(2R,3aR)-2-methoxy-5,10,3a-trihydropyrazino[2,3-b]pyrrolidino[1,2-e]pyrazin-4-one;
8-(4-(4H-1,2,4-triazol-3-yl)-2-methylphenyl)(2S,3aR)-2-methoxy-5,10,3a-trihydropyrazino[2,3-b]pyrrolidino[1,2-e]pyrazin-4-one;
8-(4-(4H-1,2,4-triazol-3-yl)-2-methylphenyl)(2S,3aS)-2-methoxy-5,10,3a-trihydropyrazino[2,3-b]pyrrolidino[1,2-e]pyrazin-4-one;
6-(6-(2-hydroxypropan-2-yl)pyridin-3-yl)-4-(3-methoxypropyl)-3,4-dihydropyrazino[2,3-b]pyrazin-2(1H)-one;
(S)-6-(6-(2-hydroxypropan-2-yl)pyridin-3-yl)-4-((tetrahydrofuran-2-yl)methyl)-3,4-dihydropyrazino[2,3-b]pyrazin-2(1H)-one;
(R)-6-(6-(2-hydroxypropan-2-yl)pyridin-3-yl)-4-((tetrahydrofuran-2-yl)methyl)-3,4-dihydropyrazino[2,3-b]pyrazin-2(1H)-one;
6-(2-methyl-6-(4H-1,2,4-triazol-3-yl)pyridin-3-yl)-4-(2-(tetrahydro-2H-pyran-4-yl)ethyl)-3,4-dihydropyrazino[2,3-b]pyrazin-2(1H)-one;
9-(4-(4H-1,2,4-triazol-3-yl)-2-methylphenyl)-3-methyl-6,11,4a-trihydropiperazino[1,2-e]pyrazino[2,3 -b]pyrazin-5 -one;
9-(4-(4H-1,2,4-triazol-3-yl)phenyl)-6,11,4a-trihydromorpholino[4,3-e]pyrazino[2,3-b]pyrazin-5-one;
9-(4-(4H-1,2,4-triazol-3-yl)-2-methylphenyl)-6,11,4a-trihydropiperidino[1,2-e]pyrazino[2,3-b]pyrazin-5-one;
6-(6-(2-hydroxypropan-2-yl)pyridin-3-yl)-4-(*trans*-4-methoxycyclohexyl)-3,4-dihydropyrazino[2,3-b]pyrazin-2(1H)-one;
6-(6-(2-hydroxypropan-2-yl)pyridin-3-yl)-4-(*cis*-4-methoxycyclohexyl)-3,4-dihydropyrazino[2,3-b]pyrazin-2(1H)-one;
6-(6-(2-hydroxypropan-2-yl)pyridin-3-yl)-4-(2-morpholinoethyl)-3,4-dihydropyrazino[2,3-b]pyrazin-2(1H)-one;
6-(6-(2-hydroxypropan-2-yl)pyridin-3-yl)-4-phenethyl-3,4-dihydropyrazino[2,3-b]pyrazin-2(1H)-one;
6-(6-(2-hydroxypropan-2-yl)pyridin-3-yl)-4-(tetrahydro-2H-pyran-4-yl)-3,4-dihydropyrazino[2,3-b]pyrazin-2(1H)-one;
4-(cyclohexylmethyl)-6-(6-(2-hydroxypropan-2-yl)pyridin-3-yl)-3,4-dihydropyrazino[2,3-b]pyrazin-2(1H)-one;
6-(6-(2-hydroxypropan-2-yl)pyridin-3-yl)-4-((*trans*-4-methoxycyclohexyl)methyl)-3,4-dihydropyrazino[2,3-b]pyrazin-2(1H)-one;
6-(6-(2-hydroxypropan-2-yl)pyridin-3-yl)-4-((*cis*-4-methoxycyclohexyl)methyl)-3,4-dihydropyrazino[2,3-b]pyrazin-2(1H)-one;
(R)-6-(6-(2-hydroxypropan-2-yl)pyridin-3-yl)-4-(tetrahydrofuran-3-yl)-3,4-dihydropyrazino[2,3-b]pyrazin-2(1H)-one;
(S)-6-(6-(2-hydroxypropan-2-yl)pyridin-3-yl)-4-(tetrahydrofuran-3-yl)-3,4-dihydropyrazino[2,3-b]pyrazin-2(1H)-one;
6-(6-(2-hydroxypropan-2-yl)pyridin-3-yl)-4-phenyl-3,4-dihydropyrazino[2,3-b]pyrazin-2(1H)-one;
(S)-6-(6-(2-hydroxypropan-2-yl)pyridin-3-yl)-3-methyl-4-(2-(tetrahydro-2H-pyran-4-yl)ethyl)-3,4-dihydropyrazino[2,3-b]pyrazin-2(1H)-one;
9-[6-(1-hydroxy-isopropyl)-3-pyridyl]-6,11,4a-trihydromorpholino[4,3-e]pyrazino[2,3-b]pyrazin-5-one;
6-(6-(2-hydroxypropan-2-yl)pyridin-3-yl)-4-((tetrahydro-2H-pyran-4-yl)methyl)-3,4-dihydropyrazino[2,3-b]pyrazin-2(1H)-one;
6-(6-(2-hydroxypropan-2-yl)pyridin-3-yl)-4-(2-methoxyethyl)-3,4-dihydropyrazino[2,3-b]pyrazin-2(1H)-one;
6-(2-amino-7-methyl-1H-benzo[d]imidazol-5-yl)-4-(3-(trifluoromethyl)benzyl)-3,4-dihydropyrazino[2,3-b]pyrazin-2(1H)-one;
6-(6-(2-hydroxypropan-2-yl)pyridin-3-yl)-4-(3-(trifluoromethyl)benzyl)-3,4-dihydropyrazino[2,3-b]pyrazin-2(1H)-one;
9-(4-(4H-1,2,4-triazol-3-yl)-2-methylphenyl)-6,11,4a-trihydromorpholino[4,3-e]pyrazino[2,3-b]pyrazin-5-one;
6-(4-methyl-2-(methylamino)-1H-benzo[d]imidazol-6-yl)-4-(2-(tetrahydro-2H-pyran-4-yl)ethyl)-3,4-dihydropyrazino[2,3-b]pyrazin-2(1H)-one;
8-(4-(4H-1,2,4-triazol-3-yl)-2-methylphenyl)-5,10,3a-trihydropyrazino[2,3-b]pyrrolidino[1,2-e]pyrazin-4-one;
6-(4-(4H-1,2,4-triazol-3-yl)phenyl)-4-ethyl-3,4-dihydropyrazino[2,3-b]pyrazin-2(1H)-one;
6-(4-(4H-1,2,4-triazol-3-yl)phenyl)-4-((tetrahydro-2H-pyran-4-yl)methyl)-3,4-dihydropyrazino[2,3-b]pyrazin-2(1H)-one;
6-(6-(2-hydroxypropan-2-yl)pyridin-3-yl)-4-(2-(tetrahydro-2H-pyran-4-yl)ethyl)-3,4-dihydropyrazino[2,3-b]pyrazin-2(1H)-one;
6-(4-(4H-1,2,4-triazol-3-yl)phenyl)-4-(2-methoxyethyl)-3,4-dihydropyrazino[2,3-b]pyrazin-2(1H)-one;
6-(4-(4H-1,2,4-triazol-3-yl)phenyl)-4-(3-(trifluoromethyl)benzyl)-3,4-dihydropyrazino[2,3-b]pyrazin-2(1H)-one;
6-(2-methyl-4-(4H-1,2,4-triazol-3-yl)phenyl)-4-(2-(tetrahydro-2H-pyran-4-yl)ethyl)-3,4-dihydropyrazino[2,3-b]pyrazin-2(1H)-one;
6-(4-methyl-1H-benzo[d]imidazol-6-yl)-4-(2-(tetrahydro-2H-pyran-4-yl)ethyl)-3,4-dihydropyrazino[2,3-b]pyrazin-2(1H)-one;
6-(4-(2-hydroxypropan-2-yl)phenyl)-4-(2-(tetrahydro-2H-pyran-4-yl)ethyl)-3,4-dihydropyrazino[2,3-b]pyrazin-2(1H)-one; and
6-(4-(1H-1,2,4-triazol-5-yl)phenyl)-4-(2-(tetrahydro-2H-pyran-4-yl)ethyl)-3,4-dihydropyrazino[2,3-b]pyrazin-2(1H)-one,
and pharmaceutically acceptable salts, clathrates, solvates, stereoisomers, tautomers, and prodrugs thereof.

As also described herein, the TOR kinase inhibitors may include compounds having the following formula (IV): 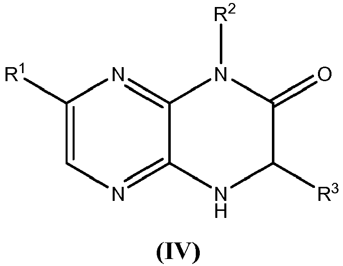 and pharmaceutically acceptable salts, clathrates, solvates, stereoisomers, tautomers, and prodrugs thereof, wherein:
R¹ is substituted or unsubstituted C₁₋₈ alkyl, substituted or unsubstituted aryl, substituted or unsubstituted cycloalkyl, substituted or unsubstituted heterocyclyl, or substituted or unsubstituted heterocyclylalkyl;
R² is H, substituted or unsubstituted C₁₋₈ alkyl, substituted or unsubstituted cycloalkyl, substituted or unsubstituted heterocyclyl, substituted or unsubstituted heterocyclylalkyl, substituted or unsubstituted aralkyl, or substituted or unsubstituted cycloalkylalkyl;
R³ is H, or a substituted or unsubstituted C₁₋₈ alkyl,
wherein in certain embodiments, the TOR kinase inhibitors de not include 7-(4-hydroxyphenyl)-1-(3-methoxybenzyl)-3,4-dihydropyrazino[2,3-b]pyrazin-2(1H)-one, depicted below:

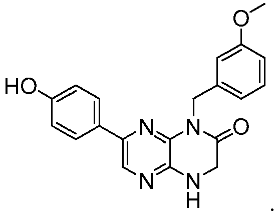

In compounds of formula (IV), R¹ may be substituted or unsubstituted aryl or substituted or unsubstituted heteroaryl. For example, R¹ may be phenyl, pyridyl, pyrimidyl, benzimidazolyl, 1H-pyrrolo[2,3-b]pyridyl, indazolyl, indolyl, 1H-imidazo[4,5-b]pyridyl, 1H-imidazo[4,5-b]pyridin-2(3H)-onyl, 3H-imidazo[4,5-b]pyridyl, or pyrazolyl, each optionally substituted. R¹ may be is phenyl substituted with one or more substituents independently selected from the group consisting of substituted or unsubstituted C₁₋₈ alkyl (for example, methyl), substituted or unsubstituted heterocyclyl (for example, a substituted or unsubstituted triazolyl or pyrazolyl), aminocarbonyl, halogen (for example, fluorine), cyano, hydroxyalkyl and hydroxy. R¹ may be is pyridyl substituted with one or more substituents independently selected from the group consisting of substituted or unsubstituted C₁₋₈ alkyl (for example, methyl), substituted or unsubstituted heterocyclyl (for example, a substituted or unsubstituted triazolyl), halogen, aminocarbonyl , cyano, hydroxyalkyl (for example, hydroxypropyl), -OR, and -NR₂, wherein each R may be independently H, or a substituted or unsubstituted C₁₋₄ alkyl. R¹ may be 1H-pyrrolo[2,3-b]pyridyl or benzimidazolyl, optionally substituted with one or more substituents independently selected may be from the group consisting of substituted or unsubstituted C₁₋₈ alkyl, and -NR₂, wherein R is independently H, or a substituted or unsubstituted C₁₋₄ alkyl.

In R¹ may be 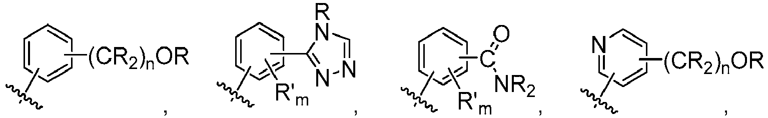 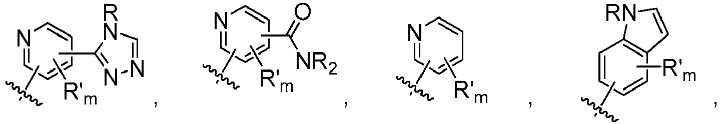 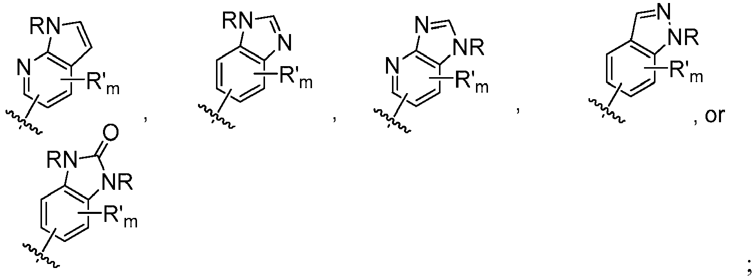 wherein R is at each occurrence independently H, or a substituted or unsubstituted C₁₋₄ alkyl (for example, methyl); R' may be at each occurrence independently a substituted or unsubstituted C₁₋₄ alkyl (for example, methyl), halogen (for example, fluoro), cyano, -OR, or -NR₂; m is 0-3; and n is 0-3. It will be understood by those skilled in the art that any of the subsitutuents R' may be attached to any suitable atom of any of the rings in the fused ring systems.

In compounds of formula (IV), R¹ may be 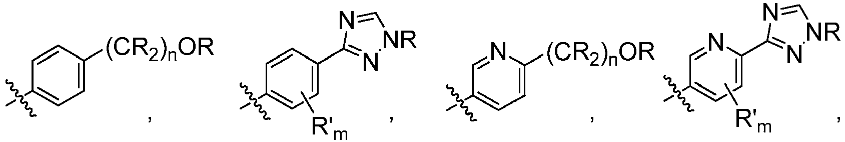 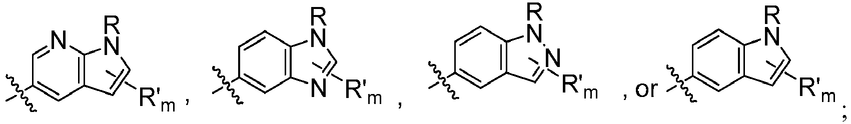 wherein R may be at each occurrence independently H, or a substituted or unsubstituted C₁₋₄ alkyl; R' may be at each occurrence independently a substituted or unsubstituted C₁₋₄ alkyl, halogen, cyano, -OR or -NR₂; m may be 0-3; and n may be 0-3.

In compounds of formula (IV), R² may be H, substituted or unsubstituted C₁₋₈ alkyl, substituted or unsubstituted cycloalkyl, substituted or unsubstituted heterocyclyl, substituted or unsubstituted C₁₋₄ alkyl-heterocyclyl, substituted or unsubstituted C₁₋₄ alkyl-aryl, or substituted or unsubstituted C₁₋₄ alkyl-cycloalkyl. For example, R² may be H, methyl, ethyl, n-propyl, isopropyl, n-butyl, *sec*-butyl, isobutyl, *tert-*butyl, n-pentyl, isopentyl, cyclopentyl, cyclohexyl, tetrahydrofuranyl, tetrahydropyranyl, (C₁₋₄ alkyl)-phenyl, (C₁₋₄ alkyl)-cyclopropyl, (C₁₋₄ alkyl)-cyclobutyl, (C₁₋₄ alkyl)-cyclopentyl, (C₁₋₄ alkyl)-cyclohexyl, (C₁₋₄ alkyl)-pyrrolidyl, (C₁₋₄ alkyl)-piperidyl, (C₁₋₄ alkyl)-piperazinyl, (C₁₋₄ alkyl)-morpholinyl, (C₁₋₄ alkyl)-tetrahydrofuranyl, or (C₁₋₄ alkyl)-tetrahydropyranyl, each optionally substituted.

R² may be H, C₁₋₄ alkyl, (C₁₋₄alkyl)(OR), 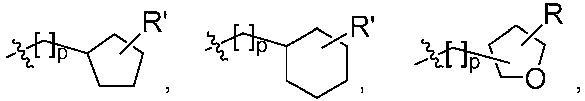 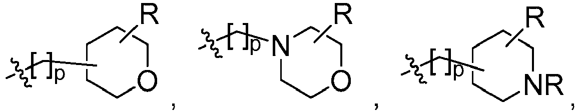 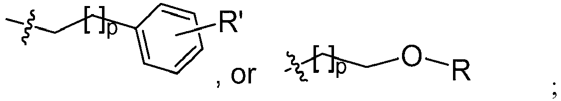 wherein R may be at each occurrence independently H, or a substituted or unsubstituted C₁₋₄ alkyl (for example, methyl); R' may be at each occurrence independently H, -OR, cyano,or a substituted or unsubstituted C₁₋₄ alkyl (for example, methyl); and p may be 0-3.

In may be compounds of formula (IV), R² may be H, C₁₋₄ alkyl, (C₁₋₄alkyl)(OR), 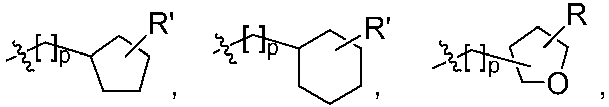 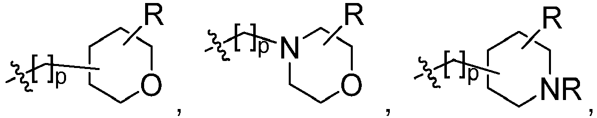 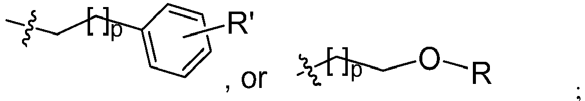 wherein R may be at each occurrence independently H, or a substituted or unsubstituted C₁₋₂ alkyl; R' may be at each occurrence independently H, -OR, cyano, or a substituted or unsubstituted C₁₋₂ alkyl; and p may be 0-1.

In compounds of formula (IV), R³ may be H.

R¹ may be substituted or unsubstituted aryl, or substituted or unsubstituted heteroaryl. For example, R¹ may be phenyl, pyridyl, pyrimidyl, benzimidazolyl, 1H-pyrrolo[2,3-b]pyridyl, indazolyl, indolyl, 1H-imidazo[4,5-b]pyridine, pyridyl, 1H-imidazo[4,5-b]pyridin-2(3H)-onyl, 3H-imidazo[4,5-b]pyridyl, or pyrazolyl, each optionally substituted. R¹ may be phenyl substituted with one or more substituents independently selected from the group consisting of substituted or unsubstituted C₁₋₈ alkyl, substituted or unsubstituted heterocyclyl, aminocarbonyl, halogen, cyano, hydroxyalkyl and hydroxy. In others, R¹ may be pyridyl substituted with one or more substituents independently selected from the group consisting of C₁₋₈ alkyl, substituted or unsubstituted heterocyclyl, halogen, aminocarbonyl, cyano, hydroxyalkyl, -OR, and -NR₂, wherein each R may be independently H, or a substituted or unsubstituted C₁₋₄ alkyl. In still others, R¹ may be 1H-pyrrolo[2,3-b]pyridyl or benzimidazolyl, optionally substituted with one or more substituents independently selected from the group consisting of substituted or unsubstituted C₁₋₈ alkyl, and -NR₂, wherein R may be independently H, or a substituted or unsubstituted C₁₋₄ alkyl.

The compounds of formula (IV) may have an R¹ group set forth herein and an R² group set forth herein.

In compounds of formula (IV), the compound at a concentration of 10 µM may inhibit mTOR, DNA-PK, PI3K, or a combination thereof by at least about 50%. Compounds of formula (IV) may be shown to be inhibitors of the kinases above in any suitable assay system.

Representative TOR kinase inhibitors of formula (IV) may include:
7-(5-fluoro-2-methyl-4-(1H-1,2,4-triazol-3-yl)phenyl)-1-((*trans*-4-methoxycyclohexyl)methyl)-3,4-dihydropyrazino[2,3-b]pyrazin-2(1H)-one;
7-(6-(1H-1,2,4-triazol-3-yl)pyridin-3-yl)-1-(*cis*-4-methoxycyclohexyl)-3,4-dihydropyrazino[2,3-b]pyrazin-2(1H)-one;
7-(1H-pyrrolo[2,3-b]pyridin-3-yl)-1-(2-(tetrahydro-2H-pyran-4-yl)ethyl)-3,4-dihydropyrazino[2,3-b]pyrazin-2(1H)-one;
7-(5-fluoro-2-methyl-4-(1H-1,2,4-triazol-3-yl)phenyl)-1-((*cis*-4-methoxycyclohexyl)methyl)-3,4-dihydropyrazino[2,3-b]pyrazin-2(1H)-one;
1-ethyl-7-(1H-pyrrolo[3,2-b]pyridin-5-yl)-3,4-dihydropyrazino[2,3-b]pyrazin-2(1H)-one;
7-(6-(1H-1,2,4-triazol-3-yl)pyridin-3-yl)-1-((*cis*-4-methoxycyclohexyl)methyl)-3,4-dihydropyrazino[2,3-b]pyrazin-2(1H)-one;
7-(1H-benzo[d]imidazol-4-yl)-1-(2-(tetrahydro-2H-pyran-4-yl)ethyl)-3,4-dihydropyrazino[2,3-b]pyrazin-2(1H)-one;
7-(1H-pyrrolo[2,3-b]pyridin-4-yl)-1-(2-(tetrahydro-2H-pyran-4-yl)ethyl)-3,4-dihydropyrazino[2,3-b]pyrazin-2(1H)-one;
7-(6-(1H-1,2,4-triazol-3-yl)pyridin-3-yl)-1-((*trans*-4-methoxycyclohexyl)methyl)-3,4-dihydropyrazino[2,3-b]pyrazin-2(1H)-one;
7-(6-(1H-1,2,4-triazol-3-yl)pyridin-3-yl)-1-((*trans*-4-hydroxycyclohexyl)methyl)-3,4-dihydropyrazino[2,3-b]pyrazin-2(1H)-one;
7-(6-(1H-1,2,4-triazol-3-yl)pyridin-3-yl)-1-(*cis*-4-hydroxycyclohexyl)-3,4-dihydropyrazino[2,3-b]pyrazin-2(1H)-one;
7-(5-fluoro-2-methyl-4-(1H-1,2,4-triazol-3-yl)phenyl)-1-(*cis*-4-hydroxycyclohexyl)-3,4-dihydropyrazino[2,3-b]pyrazin-2(1H)-one;
7-(6-(1H-1,2,4-triazol-3-yl)pyridin-3-yl)-1-(tetrahydro-2H-pyran-4-yl)-3,4-dihydropyrazino[2,3-b]pyrazin-2(1H)-one;
7-(6-(1H-1,2,4-triazol-3-yl)pyridin-3-yl)-1-(2-methoxyethyl)-3,4-dihydropyrazino[2,3-b]pyrazin-2(1H)-one;
7-(6-(1H-1,2,4-triazol-3-yl)pyridin-3-yl)-1-ethyl-3,4-dihydropyrazino[2,3-b]pyrazin-2(1H)-one;
7-(5-fluoro-2-methyl-4-(1H-1,2,4-triazol-3-yl)phenyl)-1-((*cis*-4-hydroxycyclohexyl)methyl)-3,4-dihydropyrazino[2,3-b]pyrazin-2(1H)-one;
7-(5-fluoro-2-methyl-4-(1H-1,2,4-triazol-3-yl)phenyl)-1-(tetrahydro-2H-pyran-4-yl)-3,4-dihydropyrazino[2,3-b]pyrazin-2(1H)-one;
7-(1H-indol-4-yl)-1-(2-(tetrahydro-2H-pyran-4-yl)ethyl)-3,4-dihydropyrazino[2,3-b]pyrazin-2(1H)-one;
7-(5-fluoro-2-methyl-4-(1H-1,2,4-triazol-3-yl)phenyl)-1-((*trans*-4-hydroxycyclohexyl)methyl)-3,4-dihydropyrazino[2,3-b]pyrazin-2(1H)-one;
7-(6-(1H-1,2,4-triazol-3-yl)pyridin-3-yl)-1-((*cis*-4-hydroxycyclohexyl)methyl)-3,4-dihydropyrazino[2,3-b]pyrazin-2(1H)-one;
7-(6-(1H-1,2,4-triazol-3-yl)pyridin-3-yl)-1-(*trans*-4-hydroxycyclohexyl)-3,4-dihydropyrazino[2,3-b]pyrazin-2(1H)-one;
7-(6-(1H-1,2,4-triazol-3-yl)pyridin-3-yl)-1-(*trans*-4-methoxycyclohexyl)-3,4-dihydropyrazino[2,3-b]pyrazin-2(1H)-one;
7-(6-(1H-1,2,4-triazol-3-yl)pyridin-3-yl)-1-isopropyl-3,4-dihydropyrazino[2,3-b]pyrazin-2(1H)-one;
7-(5-fluoro-2-methyl-4-(1H-1,2,4-triazol-3-yl)phenyl)-1-(*trans*-4-methoxycyclohexyl)-3,4-dihydropyrazino[2,3-b]pyrazin-2(1H)-one;
7-(5-fluoro-2-methyl-4-(1H-1,2,4-triazol-3-yl)phenyl)-1-(*trans*-4-hydroxycyclohexyl)-3,4-dihydropyrazino[2,3-b]pyrazin-2(1H)-one;
7-(5-fluoro-2-methyl-4-(1H-1,2,4-triazol-3-yl)phenyl)-1-(2-methoxyethyl)-3,4-dihydropyrazino[2,3-b]pyrazin-2(1H)-one;
7-(5-fluoro-2-methyl-4-(1H-1,2,4-triazol-3-yl)phenyl)-1-isopropyl-3,4-dihydropyrazino[2,3-b]pyrazin-2(1H)-one;
1-ethyl-7-(5-fluoro-2-methyl-4-(1H-1,2,4-triazol-3-yl)phenyl)-3,4-dihydropyrazino[2,3-b]pyrazin-2(1H)-one;
7-(2-hydroxypyridin-4-yl)-1-(2-(tetrahydro-2H-pyran-4-yl)ethyl)-3,4-dihydropyrazino[2,3-b]pyrazin-2(1H)-one;
1-isopropyl-7-(4-methyl-6-(1H-1,2,4-triazol-3-yl)pyridin-3-yl)-3,4-dihydropyrazino[2,3-b]pyrazin-2(1H)-one;
5-(8-isopropyl-7-oxo-5,6,7,8-tetrahydropyrazino[2,3-b]pyrazin-2-yl)-4-methylpicolinamide; 7-(1H-indazol-4-yl)-1-(2-(tetrahydro-2H-pyran-4-yl)ethyl)-3,4-dihydropyrazino[2,3-b]pyrazin-2(1H)-one;
7-(2-aminopyrimidin-5-yl)-1-(2-(tetrahydro-2H-pyran-4-yl)ethyl)-3,4-dihydropyrazino[2,3-b]pyrazin-2(1H)-one;
7-(2-aminopyridin-4-yl)-1-(2-(tetrahydro-2H-pyran-4-yl)ethyl)-3,4-dihydropyrazino[2,3-b]pyrazin-2(1H)-one;
7-(6-(methylamino)pyridin-3-yl)-1-(2-(tetrahydro-2H-pyran-4-yl)ethyl)-3,4-dihydropyrazino[2,3-b]pyrazin-2(1H)-one;
7-(6-hydroxypyridin-3-yl)-1-(2-(tetrahydro-2H-pyran-4-yl)ethyl)-3,4-dihydropyrazino[2,3-b]pyrazin-2(1H)-one;
7-(4-(1H-pyrazol-3-yl)phenyl)-1-(2-methoxyethyl)-3,4-dihydropyrazino[2,3-b]pyrazin-2(1H)-one;
7-(pyridin-3-yl)-1-(2-(tetrahydro-2H-pyran-4-yl)ethyl)-3,4-dihydropyrazino[2,3-b]pyrazin-2(1H)-one;
7-(1H-indazol-4-yl)-1-(2-methoxyethyl)-3,4-dihydropyrazino[2,3-b]pyrazin-2(1H)-one;
7-(1H-indazol-6-yl)-1-(2-methoxyethyl)-3,4-dihydropyrazino[2,3-b]pyrazin-2(1H)-one;
7-(pyrimidin-5-yl)-1-(2-(tetrahydro-2H-pyran-4-yl)ethyl)-3,4-dihydropyrazino[2,3-b]pyrazin-2(1H)-one;
7-(6-methoxypyridin-3-yl)-1-(2-(tetrahydro-2H-pyran-4-yl)ethyl)-3,4-dihydropyrazino[2,3-b]pyrazin-2(1H)-one;
1-(2-methoxyethyl)-7-(1H-pyrrolo[2,3-b]pyridin-5-yl)-3,4-dihydropyrazino[2,3-b]pyrazin-2(1H)-one;
1-ethyl-7-(1H-pyrrolo[2,3-b]pyridin-5-yl)-3,4-dihydropyrazino[2,3-b]pyrazin-2(1H)-one;
1-ethyl-7-(1H-indazol-4-yl)-3,4-dihydropyrazino[2,3-b]pyrazin-2(1H)-one;
7-(pyridin-4-yl)-1-(2-(tetrahydro-2H-pyran-4-yl)ethyl)-3,4-dihydropyrazino[2,3-b]pyrazin-2(1H)-one;
7-(6-aminopyridin-3-yl)-1-(2-(tetrahydro-2H-pyran-4-yl)ethyl)-3,4-dihydropyrazino[2,3-b]pyrazin-2(1H)-one;
1-methyl-7-(2-methyl-6-(4H-1,2,4-triazol-3-yl)pyridin-3-yl)-3,4-dihydropyrazino[2,3-b]pyrazin-2(1H)-one;
2-(2-hydroxypropan-2-yl)-5-(8-(*trans*-4-methoxycyclohexyl)-7-oxo-5 ,6,7,8-tetrahydropyrazino[2,3-b]pyrazin-2-yl)pyridine 1-oxide;
4-methyl-5-(7-oxo-8-((tetrahydro-2H-pyran-4-yl)methyl)-5,6,7,8-tetrahydropyrazino[2,3-b]pyrazin-2-yl)picolinamide;
5-(8-((*cis*-4-methoxycyclohexyl)methyl)-7-oxo-5,6,7,8-tetrahydropyrazino[2,3-b]pyrazin-2-yl)-4-methylpicolinamide;
7-(1H-pyrazol-4-yl)-1-(2-(tetrahydro-2H-pyran-4-yl)ethyl)-3,4-dihydropyrazino[2,3-b]pyrazin-2(1H)-one;
1-(*trans*-4-methoxycyclohexyl)-7-(4-methyl-6-(1H-1,2,4-triazol-3-yl)pyridin-3-yl)-3,4-dihydropyrazino[2,3-b]pyrazin-2(1H)-one;
3-((7-(2-methyl-6-(4H-1,2,4-triazol-3-yl)pyridin-3-yl)-2-oxo-3,4-dihydropyrazino[2,3-b]pyrazin-1(2H)-yl)methyl)benzonitrile;
1-((*trans*-4-methoxycyclohexyl)methyl)-7-(4-methyl-6-(1H-1,2,4-triazol-3-yl)pyridin-3-yl)-3,4-dihydropyrazino[2,3-b]pyrazin-2(1H)-one;
3-(7-oxo-8-(2-(tetrahydro-2H-pyran-4-yl)ethyl)-5,6,7,8-tetrahydropyrazino[2,3-b]pyrazin-2-yl)benzamide;
5-(8-((*trans*-4-methoxycyclohexyl)methyl)-7-oxo-5,6,7,8-tetrahydropyrazino[2,3-b]pyrazin-2-yl)-4-methylpicolinamide;
3-((7-(6-(2-hydroxypropan-2-yl)pyridin-3-yl)-2-oxo-3,4-dihydropyrazino[2,3-b]pyrazin-1(2H)-yl)methyl)benzonitrile;
7-(6-(2-hydroxypropan-2-yl)pyridin-3-yl)-1-((1R,3R)-3-methoxycyclopentyl)-3,4-dihydropyrazino[2,3-b]pyrazin-2(1H)-one;
7-(6-(2-hydroxypropan-2-yl)pyridin-3-yl)-1-((1S,3R)-3-methoxycyclopentyl)-3,4-dihydropyrazino[2,3-b]pyrazin-2(1H)-one;
7-(6-(2-hydroxypropan-2-yl)pyridin-3-yl)-1-((1S,3S)-3-methoxycyclopentyl)-3,4-dihydropyrazino[2,3-b]pyrazin-2(1H)-one;
7-(6-(2-hydroxypropan-2-yl)pyridin-3-yl)-1-((1R,3S)-3-methoxycyclopentyl)-3,4-dihydropyrazino[2,3-b]pyrazin-2(1H)-one;
7-(1H-indazol-6-yl)-1-(2-(tetrahydro-2H-pyran-4-yl)ethyl)-3,4-dihydropyrazino[2,3-b]pyrazin-2(1H)-one;
7-(2-methyl-6-(4H-1,2,4-triazol-3-yl)pyridin-3-yl)-1-(2-morpholinoethyl)-3,4-dihydropyrazino[2,3-b]pyrazin-2(1H)-one;
1-(*trans*-4-hydroxycyclohexyl)-7-(2-methyl-6-(4H-1,2,4-triazol-3-yl)pyridin-3-yl)-3,4-dihydropyrazino[2,3-b]pyrazin-2(1H)-one;
1-(*cis*-4-hydroxycyclohexyl)-7-(2-methyl-6-(4H-1,2,4-triazol-3-yl)pyridin-3-yl)-3,4-dihydropyrazino[2,3-b]pyrazin-2(1H)-one;
7-(6-(2-hydroxypropan-2-yl)pyridin-3-yl)-1-(2-morpholinoethyl)-3,4-dihydropyrazino[2,3-b]pyrazin-2(1H)-one;
1-isopropyl-7-(2-methyl-6-(4H-1,2,4-triazol-3-yl)pyridin-3-yl)-3,4-dihydropyrazino[2,3-b]pyrazin-2(1H)-one;
7-(1H-imidazo[4,5-b]pyridin-6-yl)-1-(2-(tetrahydro-2H-pyran-4-yl)ethyl)-3,4-dihydropyrazino[2,3-b]pyrazin-2(1H)-one;
1-((*cis*-4-methoxycyclohexyl)methyl)-7-(2-methyl-6-(1H-1,2,4-triazol-3-yl)pyridin-3-yl)-3,4-dihydropyrazino[2,3-b]pyrazin-2(1H)-one;
1-(*trans*-4-hydroxycyclohexyl)-7-(6-(2-hydroxypropan-2-yl)pyridin-3-yl)-3,4-dihydropyrazino[2,3-b]pyrazin-2(1H)-one;
1-(*cis*-4-hydroxycyclohexyl)-7-(6-(2-hydroxypropan-2-yl)pyridin-3-yl)-3,4-dihydropyrazino[2,3-b]pyrazin-2(1H)-one;
4-(7-oxo-8-(2-(tetrahydro-2H-pyran-4-yl)ethyl)-5,6,7,8-tetrahydropyrazino[2,3-b]pyrazin-2-yl)benzamide;
7-(1H-mdazol-5-yl)-1-(2-(tetrahydro-2H-pyran-4-yl)ethyl)-3,4-dihydropyrazino[2,3-b]pyrazin-2(1H)-one;
7-(1H-pyrrolo[2,3-b]pyridin-5-yl)-1-(2-(tetrahydro-2H-pyran-4-yl)ethyl)-3,4-dihydropyrazino[2,3-b]pyrazin-2(1H)-one;
7-(2-methyl-6-(4H-1,2,4-triazol-3-yl)pyridin-3-yl)-1-(tetrahydro-2H-pyran-4-yl)-3,4-dihydropyrazino[2,3-b]pyrazin-2(1H)-one;
1-((1S,3R)-3-methoxycyclopentyl)-7-(2-methyl-6-(4H-1,2,4-triazol-3-yl)pyridin-3-yl)-3,4-dihydropyrazino[2,3-b]pyrazin-2(1H)-one;
1-((1R,3R)-3-methoxycyclopentyl)-7-(2-methyl-6-(4H-1,2,4-triazol-3-yl)pyridin-3-yl)-3,4-dihydropyrazino[2,3-b]pyrazin-2(1H)-one;
1-((1R,3S)-3-methoxycyclopentyl)-7-(2-methyl-6-(4H-1,2,4-triazol-3-yl)pyridmin-3-yl)-3,4-dihydropyrazino[2,3-b]pyrazin-2(1H)-one;
1-((1S,3S)-3-methoxycyclopentyl)-7-(2-methyl-6-(4H-1,2,4-triazol-3-yl)pyridin-3-yl)-3,4-dihydropyrazino[2,3-b]pyrazin-2(1H)-one;
7-(1H-indol-5-yl)-1-(2-(tetrahydro-2H-pyran-4-yl)ethyl)-3,4-dihydropyrazino[2,3-b]pyrazin-2(1H)-one;
1-ethyl-7-(2-methyl-6-(4H-1,2,4-triazol-3-yl)pyridin-3-yl)-3,4-dihydropyrazino[2,3-b]pyrazin-2(1H)-one;
7-(1H-mdol-6-yl)-1-(2-(tetrahydro-2H-pyran-4-yl)ethyl)-3,4-dihydropyrazino[2,3-b]pyrazin-2(1H)-one;
7-(4-(2-hydroxypropan-2-yl)phenyl)-1-(*trans*-4-methoxycyclohexyl)-3,4-dihydropyrazino[2,3-b]pyrazin-2(1H)-one;
7-(6-(2-hydroxypropan-2-yl)pyridin-3-yl)-1-(tetrahydro-2H-pyran-4-yl)-3,4-dihydropyrazino[2,3-b]pyrazin-2(1H)-one;
1-((*trans*-4-methoxycyclohexyl)methyl)-7-(2-methyl-6-(1H-1,2,4-triazol-3-yl)pyridin-3-yl)-3,4-dihydropyrazino[2,3-b]pyrazin-2(1H)-one;
7-(6-(2-hydroxypropan-2-yl)pyridin-3-yl)-1-((*cis*-4-methoxycyclohexyl)methyl)-3,4-dihydropyrazino[2,3-b]pyrazin-2(1H)-one;
1-(2-methoxyethyl)-7-(4-methyl-2-(methylamino)-1H-benzo [d]imidazol-6-yl)-3,4-dihydropyrazino[2,3-b]pyrazin-2(1H)-one;
7-(7-methyl-2-oxo-2,3-dihydro-1H-benzo[d]imidazol-5-yl)-1-((tetrahydro-2H-pyran-4-yl)methyl)-3,4-dihydropyrazino[2,3-b]pyrazin-2(1H)-one;
7-(2-methyl-4-(4H-1,2,4-triazol-3-yl)phenyl)-3,4-dihydropyrazino[2,3-b]pyrazin-2(1H)-one; 1-(2-methoxyethyl)-7-(4-methyl-6-(1H-1,2,4-triazol-3-yl)pyridin-3-yl)-3,4-dihydropyrazino[2,3-b]pyrazin-2(1H)-one;
1-benzyl-7-(2-methyl-4-(4H-1,2,4-triazol-3-yl)phenyl)-3,4-dihydropyrazino[2,3-b]pyrazin-2(1H)-one;
7-(3-fluoro-4-(4H-1,2,4-triazol-3-yl)phenyl)-1-(2-methoxyethyl)-3,4-dihydropyrazino[2,3-b]pyrazin-2(1H)-one;
7-(3-fluoro-4-(4H-1,2,4-triazol-3-yl)phenyl)-1-(2-(tetrahydro-2H-pyran-4-yl)ethyl)-3,4-dihydropyrazino[2,3-b]pyrazin-2(1H)-one;
7-(3-fluoro-2-methyl-4-(1H-1,2,4-triazol-3-yl)phenyl)-1-(2-methoxyethyl)-3,4-dihydropyrazino[2,3-b]pyrazin-2(1H)-one;
1-(*trans*-4-methoxycyclohexyl)-7-(2-methyl-6-(4H-1,2,4-triazol-3-yl)pyridin-3-yl)-3,4-dihydropyrazino[2,3-b]pyrazin-2(1H)-one;
7-(6-(2-hydroxypropan-2-yl)pyridin-3-yl)-1-(*trans*-4-methoxycyclohexyl)-3,4-dihydropyrazino[2,3-b]pyrazin-2(1H)-one;
7-(5-fluoro-2-methyl-4-(4H-1,2,4-triazol-3-yl)phenyl)-1-(2-(tetrahydro-2H-pyran-4-yl)ethyl)-3,4-dihydropyrazino[2,3-b]pyrazin-2(1H)-one;
7-(3-fluoro-2-methyl-4-(1H-1,2,4-triazol-3-yl)phenyl)-1-(2-(tetrahydro-2H-pyran-4-yl)ethyl)-3,4-dihydropyrazino[2,3-b]pyrazin-2(1H)-one;
1-(2-methoxyethyl)-7-(2-methyl-6-(4H-1,2,4-triazol-3-yl)pyridin-3-yl)-3,4-dihydropyrazino[2,3-b]pyrazin-2(1H)-one;
7-(6-(2-hydroxypropan-2-yl)pyridin-3-yl)-1-((*trans*-4-methoxycyclohexyl)methyl)-3,4-dihydropyrazino[2,3-b]pyrazin-2(1H)-one;
1-(cyclopentylmethyl)-7-(6-(2-hydroxypropan-2-yl)pyridin-3-yl)-3,4-dihydropyrazino[2,3-b]pyrazin-2(1H)-one;
7-(4-(2-hydroxypropan-2-yl)phenyl)-1-(2-methoxyethyl)-3,4-dihydropyrazino[2,3-b]pyrazin-2(1H)-one;
(S)-7-(6-(1-hydroxyethyl)pyridin-3-yl)-1-(2-(tetrahydro-2H-pyran-4-yl)ethyl)-3,4-dihydropyrazino[2,3-b]pyrazin-2(1H)-one;
(R)-7-(6-(1-hydroxyethyl)pyridin-3-yl)-1-(2-(tetrahydro-2H-pyran-4-yl)ethyl)-3,4-dihydropyrazino[2,3-b]pyrazin-2(1H)-one;
7-(2-methyl-6-(4H-1,2,4-triazol-3-yl)pyridin-3-yl)-1-((tetrahydro-2H-pyran-4-yl)methyl)-3,4-dihydropyrazino[2,3-b]pyrazin-2(1H)-one;
7-(4-(2-hydroxypropan-2-yl)phenyl)-1-(2-(tetrahydro-2H-pyran-4-yl)ethyl)-3,4-dihydropyrazino[2,3-b]pyrazin-2(1H)-one;
7-(6-(2-hydroxypropan-2-yl)pyridin-3-yl)-1-(4-(trifluoromethyl)benzyl)-3,4-dihydropyrazino[2,3-b]pyrazin-2(1H)-one;
7-(6-(2-hydroxypropan-2-yl)pyridm-3-yl)-1-(3-(trifluoromethyl)benzyl)-3,4-dihydropyrazino[2,3-b]pyrazin-2(1H)-one;
7-(6-(2-hydroxypropan-2-yl)pyridin-3-yl)-1-(3-methoxypropyl)-3,4-dihydropyrazino[2,3-b]pyrazin-2(1H)-one;
7-(4-methyl-6-(1H-1,2,4-triazol-3-yl)pyridin-3-yl)-1-(2-(tetrahydro-2H-pyran-4-yl)ethyl)-3,4-dihydropyrazino[2,3-b]pyrazin-2(1H)-one;
7-(6-(2-hydroxypropan-2-yl)pyridin-3-yl)-1-(2-methoxyethyl)-3,4-dihydropyrazino[2,3-b]pyrazin-2(1H)-one;
7-(6-(2-hydroxypropan-2-yl)pyridin-3-yl)-1-((tetrahydro-2H-pyran-4-yl)methyl)-3,4-dihydropyrazino[2,3-b]pyrazin-2(1H)-one;
7-(4-methyl-2-(methylamino)-1H-benzo[d]imidazol-6-yl)-1-((tetrahydro-2H-pyran-4-yl)methyl)-3,4-dihydropyrazino[2,3-b]pyrazin-2(1H)-one;
7-(2-amino-4-methyl-1H-benzo[d]imidazol-6-yl)-1-((tetrahydro-2H-pyran-4-yl)methyl)-3,4-dihydropyrazino[2,3-b]pyrazin-2(1H)-one;
7-(2-methyl-6-(4H-1,2,4-triazol-3-yl)pyridin-3-yl)-1-(2-(tetrahydro-2H-pyran-4-yl)ethyl)-3,4-dihydropyrazino[2,3-b]pyrazin-2(1H)-one;
(R)-7-(6-(2-hydroxypropan-2-yl)pyridin-3-yl)-3-methyl-1-(2-(tetrahydro-2H-pyran-4-yl)ethyl)-3,4-dihydropyrazino[2,3-b]pyrazin-2(1H)-one;
(S)-7-(6-(2-hydroxypropan-2-yl)pyridin-3-yl)-3-methyl-1-(2-(tetrahydro-2H-pyran-4-yl)ethyl)-3,4-dihydropyrazino[2,3-b]pyrazin-2(1H)-one;
7-(6-(2-hydroxypropan-2-yl)pyridin-3-yl)-3,3-dimethyl-1-(2-(tetrahydro-2H-pyran-4-yl)ethyl)-3,4-dihydropyrazino[2,3-b]pyrazin-2(1H)-one;
7-(2-amino-4-methyl-1H-benzo[d]imidazol-6-yl)-1-(2-(tetrahydro-2H-pyran-4-yl)ethyl)-3,4-dihydropyrazino[2,3-b]pyrazin-2(1H)-one;
7-(6-(2-hydroxypropan-2-yl)pyridin-3-yl)-1-(2-(tetrahydro-2H-pyran-4-yl)ethyl)-3,4-dihydropyrazino[2,3-b]pyrazin-2(1H)-one;
7-(2-methyl-4-(1H-1,2,4-triazol-3-yl)phenyl)-1-(2-(tetrahydro-2H-pyran-4-yl)ethyl)-3,4-dihydropyrazino[2,3-b]pyrazin-2(1H)-one;
7-(4-(1H-1,2,4-triazol-5-yl)phenyl)-1-(2-(tetrahydro-2H-pyran-4-yl)ethyl)-3,4-dihydropyrazino[2,3-b]pyrazin-2(1H)-one;
1-(1-hydroxypropan-2-yl)-7-(2-methyl-6-(1H-1,2,4-triazol-3-yl)pyridin-3-yl)-3,4-dihydropyrazino[2,3-b]pyrazin-2(1H)-one; and
1-(2-hydroxyethyl)-7-(2-methyl-6-(1H-1,2,4-triazol-3-yl)pyridin-3-yl)-3,4-dihydropyrazino[2,3-b]pyrazin-2(1H)-one,
and pharmaceutically acceptable salts, clathrates, solvates, stereoisomers, tautomers, and prodrugs thereof.

### 4.4 METHODS FOR MAKING TOR KINASE INHIBITORS

The TOR kinase inhibitors can be obtained via standard, well-known synthetic methodology, see *e.g.,* March, J. Advanced Organic Chemistry; Reactions Mechanisms, and Structure, 4th ed., 1992. Starting materials useful for preparing compounds of formula (III) and intermediates therefore, are commercially available or can be prepared from commercially available materials using known synthetic methods and reagents.

Particular methods for preparing compounds of formula (I) are disclosed in U.S. Patent No. 7,981,893, issued July 19, 2011. Particular methods for preparing compounds of formula (II) are disclosed in U.S. Patent No. 7,968,556, issued June 28, 2011. Particular methods for preparing compounds of formula (III) and (IV) are disclosed in U.S. Patent No. 8,110,578, issued February 7, 2012, and U.S. Publication No. 2011/0137028, filed October 25, 2010.

### 4.5 COMPOUND I FOR USE IN METHODS OF TREATMENT

Provided herein is Compound 1 for use in methods for treating or preventing E-twenty six (ETS) overexpressing Ewings sarcoma, comprising administering an effective amount of Compound 1 to a patient having ETS overexpressing Ewings sarcoma. In certain embodiments, Compound 1 is administered to a patient who has locally advanced, recurrent or metastatic, ETS overexpressing Ewings sarcoma not amenable to curative surgical resection. In another embodiment, Compound 1 is administered to a patient who has received at least one prior line of platinum based chemotherapy. In some embodiments, Compound 1 is administered to a patient who has a tumor showing DNA-PK overexpression.

In some embodiments, provided herein is Compound 1 for use in methods for treating E-twenty six (ETS) overexpressing Ewings sarcoma, the methods comprising administering an effective amount of Compound 1 to a patient having E-twenty six (ETS) overexpressing Ewings sarcoma, wherein the treatment results in one or more of inhibition of disease progression, inhibition of tumor growth, reduction of primary tumor, relief of tumor-related symptoms, inhibition of tumor secreted factors (including tumor secreted hormones, such as those that contribute to carcinoid syndrome), delayed appearance of primary or secondary tumors, slowed development of primary or secondary tumors, decreased occurrence of primary or secondary tumors, slowed or decreased severity of secondary effects of disease, arrested tumor growth and regression of tumors, increased Time To Progression (TTP), increased Progression Free Survival (PFS), and/or increased Overall Survival (OS), among others.

In one embodiment, provided herein is Compound 1 for use in methods for improving the Response Evaluation Criteria in Solid Tumors (RECIST 1.1) (*see* Eisenhauer E.A., Therasse P., Bogaerts J., et al. New response evaluation criteria in solid tumours: Revised RECIST guideline (version 1.1). European J. Cancer; 2009; (45) 228-247) of a patient comprising administering an effective amount of Compound 1 to a patient having ETS overexpressing Ewings sarcoma.

Also described herein are methods for inhibiting phosphorylation of S6RP, 4E-BP1 and/or AKT in a patient having ETS overexpressing Ewings sarcoma, comprising administering an effective amount of a TOR kinase inhibitor to said patient. The inhibition of phosphorylation may be assessed in a biological sample of the patient, such as in circulating blood and/or tumor cells, skin biopsies and/or tumor biopsies or aspirate. The amount of inhibition of phosphorylation may be assessed by comparison of the amount of phospho- S6RP, 4E-BP1 and/or AKT before and after administration of the TOR kinase inhibitor. Also described herein are methods for measuring inhibition of phosphorylation of S6RP, 4E-BP1 or AKT in a patient having ETS overexpressing Ewings sarcoma, comprising administering an effective amount of a TOR kinase inhibitor to said patient, measuring the amount of phosphorylated S6RP, 4E BP1 and/or AKT in said patient, and comparing said amount of phosphorylated S6RP, 4E BP1 and/or AKT to that of said patient prior to administration of an effective amount of a TOR kinase inhibitor.

Also described herein are methods for inhibiting phosphorylation of S6RP, 4E-BP1 and/or AKT in a biological sample of a patient having ETS overexpressing Ewings sarcoma, comprising administering an effective amount of a TOR kinase inhibitor to said patient and comparing the amount of phosphorylated S6RP, 4E-BP1 and/or AKT in a biological sample of a patient obtained prior to and after administration of said TOR kinase inhibitor, wherein less phosphorylated S6RP, 4E-BP1 and/or AKT in said biological sample obtained after administration of said TOR kinase inhibitor relative to the amount of phosphorylated S6RP, 4E-BP1 and/or AKT in said biological sample obtained prior to administration of said TOR kinase inhibitor indicates inhibition.

Also described herein are methods for inhibiting DNA-dependent protein kinase (DNA-PK) activity in a patient having ETS overexpressing Ewings sarcoma, comprising administering an effective amount of a TOR kinase inhibitor to said patient. DNA-PK inhibition may be assessed in the skin of the patient having ETS overexpressing Ewings sarcoma, in one example in a UV light-irradiated skin sample of said patient. DNA-PK inhibition may be assessed in a tumor biopsy or aspirate of a patient having ETS overexpressing Ewings sarcoma. Inhibition may be assessed by measuring the amount of phosphorylated DNA-PK S2056 (also known as pDNA-PK S2056) before and after administration of the TOR kinase inhibitor. Also described herein are methods for measuring inhibition of phosphorylation of DNA-PK S2056 in a skin sample of a patient having ETS overexpressing Ewings sarcoma, comprising administering an effective amount of a TOR kinase inhibitor to said patient, measuring the amount of phosphorylated DNA-PK S2056 present in the skin sample and comparing said amount of phosphorylated DNA-PK S2056 to that in a skin sample from said patient prior to administration of an effective amount of a TOR kinase inhibitor. The skin sample may be irradiated with UV light.

Also described herein are methods for inhibiting DNA-dependent protein kinase (DNA-PK) activity in a skin sample of a patient having ETS overexpressing Ewings sarcoma, comprising administering an effective amount of a TOR kinase inhibitor to said patient and comparing the amount of phosphorylated DNA-PK in a biological sample of a patient obtained prior to and after administration of said TOR kinase inhibitor, wherein less phosphorylated DNA-PK in said biological sample obtained after administration of said TOR kinase inhibitor relative to the amount of phosphorylated DNA-PK in said biological sample obtained prior to administration of said TOR kinase inhibitor indicates inhibition.

The TOR kinase inhibitor is Compound 1 (a TOR kinase inhibitor set forth herein having molecular formula C₁₆H₁₆N₈O) wherein Compound 1 is 1-ethyl-7-(2-methyl-6-(1H-1,2,4-triazol-3-yl)pyridin-3-yl)-3,4-dihydropyrazino[2,3-b]pyrazin-2(1H)-one.

A TOR kinase inhibitor can be combined with radiation therapy or surgery. In certain embodiments, Compound 1 is administered to patient who is undergoing radiation therapy, has previously undergone radiation therapy or will be undergoing radiation therapy. In certain embodiments, Compound 1 is administered to a patient who has undergone tumor removal surgery.

Further provided herein is Compound 1 for use in methods for treating patients who have been previously treated for ETS overexpressing Ewings sarcoma, but are non-responsive to standard therapies, as well as those who have not previously been treated. Further provided herein is Compound 1 for use in methods for treating patients who have undergone surgery in an attempt to treat the condition at issue, as well as those who have not. Because patients with ETS overexpressing Ewings sarcoma may have heterogenous clinical manifestations and varying clinical outcomes, the treatment given to a patient may vary, depending on his/her prognosis. The skilled clinician will be able to readily determine without undue experimentation specific secondary agents, types of surgery, and types of non-drug based standard therapy that can be effectively used to treat an individual patient with ETS overexpressing Ewings sarcoma.

In certain embodiments, the ETS overexpressing Ewings sarcoma is Ewing's sarcoma of the bone, extraosseus Ewing's sarcoma (also known as extraskeletal Ewing's sarcoma), primitive neuroectodermal tumor (PNET), peripheral neuroepithelioma, Askin's tumor (also known as chest wall Ewing's sarcoma) or atypical Ewing's sarcoma.

In one embodiment, the ETS overexpressing Ewings sarcoma is that in which the PI3K/mTOR pathway is activated. In certain embodiments, the ETS overexpressing Ewings sarcoma is that in which the PI3K/mTOR pathway is activated due to PTEN loss, a PIK3Ca mutation or EGFR overexpression, or a combination thereof.

### 4.6 PHARMACEUTICAL COMPOSITIONS AND ROUTES OF ADMINISTRATION

Provided herein are compositions, comprising an effective amount of a TOR kinase inhibitor, and compositions comprising an effective amount of a TOR kinase inhibitor and a pharmaceutically acceptable carrier or vehicle. In some embodiments, the pharmaceutical compositions described herein are suitable for oral, parenteral, mucosal, transdermal or topical administration.

Compound 1 can be administered to a patient orally or parenterally in the conventional form of preparations, such as capsules, microcapsules, tablets, granules, powder, troches, pills, suppositories, injections, suspensions and syrups. Suitable formulations can be prepared by methods commonly employed using conventional, organic or inorganic additives, such as an excipient *(e.g.,* sucrose, starch, mannitol, sorbitol, lactose, glucose, cellulose, talc, calcium phosphate or calcium carbonate), a binder *(e.g.,* cellulose, methylcellulose, hydroxymethylcellulose, polypropylpyrrolidone, polyvinylpyrrolidone, gelatin, gum arabic, polyethyleneglycol, sucrose or starch), a disintegrator *(e.g.,* starch, carboxymethylcellulose, hydroxypropylstarch, low substituted hydroxypropylcellulose, sodium bicarbonate, calcium phosphate or calcium citrate), a lubricant *(e.g.,* magnesium stearate, light anhydrous silicic acid, talc or sodium lauryl sulfate), a flavoring agent *(e.g.,* citric acid, menthol, glycine or orange powder), a preservative *(e.g,* sodium benzoate, sodium bisulfite, methylparaben or propylparaben), a stabilizer *(e.g.,* citric acid, sodium citrate or acetic acid), a suspending agent *(e.g.,* methylcellulose, polyvinyl pyrroliclone or aluminum stearate), a dispersing agent *(e.g.,* hydroxypropylmethylcellulose), a diluent *(e.g.,* water), and base wax *(e.g.,* cocoa butter, white petrolatum or polyethylene glycol). The effective amount of the TOR kinase inhibitor in the pharmaceutical composition may be at a level that will exercise the desired effect; for example, about 0.005 mg/kg of a patient's body weight to about 10 mg/kg of a patient's body weight in unit dosage for both oral and parenteral administration.

The dose of Compound 1 to be administered to a patient is rather widely variable and can be subject to the judgment of a health-care practitioner. In general, Compound 1 can be administered one to four times a day in a dose of about 0.005 mg/kg of a patient's body weight to about 10 mg/kg of a patient's body weight in a patient, but the above dosage may be properly varied depending on the age, body weight and medical condition of the patient and the type of administration. In one embodiment, the dose is about 0.01 mg/kg of a patient's body weight to about 5 mg/kg of a patient's body weight, about 0.05 mg/kg of a patient's body weight to about 1 mg/kg of a patient's body weight, about 0.1 mg/kg of a patient's body weight to about 0.75 mg/kg of a patient's body weight, about 0.25 mg/kg of a patient's body weight to about 0.5 mg/kg of a patient's body weight, or about 0.007 mg/kg of a patient's body weight to about 1.7 mg/kg of patient's body weight. In one embodiment, one dose is given per day. In another embodiment, two doses are given per day. In any given case, the amount of Compound 1 administered will depend on such factors as the solubility of the active component, the formulation used and the route of administration.

In another embodiment, provided herein is Compound 1 for use in methods for the treatment or prevention of ETS overexpressing Ewings sarcoma, comprising the administration of about 0.375 mg/day to about 750 mg/day, about 0.75 mg/day to about 375 mg/day, about 3.75 mg/day to about 75 mg/day, about 7.5 mg/day to about 55 mg/day, about 18 mg/day to about 37 mg/day, about 0.5 mg/day to about 60 mg/day, or about 0.5 mg/day to about 128 mg/day of Compound 1 to a patient in need thereof. In another embodiment, provided herein is Compound 1 for use in methods for the treatment or prevention of ETS overexpressing Ewings sarcoma, comprising the administration of about 0.5 mg/day to about 1200 mg/day, about 10 mg/day to about 1200 mg/day, about 100 mg/day to about 1200 mg/day, about 400 mg/day to about 1200 mg/day, about 600 mg/day to about 1200 mg/day, about 400 mg/day to about 800 mg/day or about 600 mg/day to about 800 mg/day of Compound 1 to a patient in need thereof. In a particular embodiment, the methods disclosed herein comprise the administration of 0.5 mg/day, 1 mg/day, 2 mg/day, 4 mg/day, 8 mg/day, 16 mg/day, 20 mg/day, 25 mg/day, 30 mg/day, 45 mg/day, 60 mg/day, 90 mg/day, or 120 mg/day of Compound 1 to a patient in need thereof.

In another embodiment, provided herein are unit dosage formulations that comprise between about 0.1 mg and about 2000 mg, about 1 mg and 200 mg, about 35 mg and about 1400 mg, about 125 mg and about 1000 mg, about 250 mg and about 1000 mg, or about 500 mg and about 1000 mg of Compound 1.

In a particular embodiment, provided herein are unit dosage formulation comprising about 0.1 mg, 0.25 mg, 0.5 mg, 1 mg, 5 mg, 7.5 mg, 10 mg, 15 mg, 20 mg, 30 mg, 45 mg, 50 mg, 60 mg, 75 mg, 100 mg, 125 mg, 150 mg, 200 mg, 250 mg, 300 mg, 400 mg, 600 mg or 800 mg of Compound 1.

In another embodiment, provided herein are unit dosage formulations that comprise 0.1 mg, 0.25 mg, 0.5 mg, 1 mg, 2.5 mg, 5 mg, 7.5 mg, 10 mg, 15 mg, 20 mg, 30 mg, 35 mg, 50 mg, 70 mg, 100 mg, 125 mg, 140 mg, 175 mg, 200 mg, 250 mg, 280 mg, 350 mg, 500 mg, 560 mg, 700 mg, 750 mg, 1000 mg or 1400 mg of Compound 1. In a particular embodiment, provided herein are unit dosage formulations that comprise 5 mg, 7.5 mg, 10 mg, 15 mg, 20 mg, 30 mg, 45 mg or 60 mg of Compound 1.

Compound 1 can be administered once, twice, three, four or more times daily.

Compound 1 can be administered orally for reasons of convenience. In one embodiment, when administered orally, Compound 1 is administered with a meal and water. In another embodiment, Compound 1 is dispersed in water or juice (*e.g.,* apple juice or orange juice) and administered orally as a suspension. In another embodiment, when administered orally, Compound 1 is administered in a fasted state.

Compound 1 can also be administered intradermally, intramuscularly, intraperitoneally, percutaneously, intravenously, subcutaneously, intranasally, epidurally, sublingually, intracerebrally, intravaginally, transdermally, rectally, mucosally, by inhalation, or topically to the ears, nose, eyes, or skin. The mode of administration is left to the discretion of the health-care practitioner, and can depend in-part upon the site of the medical condition.

In one embodiment, provided herein are capsules containing Compound 1 without an additional carrier, excipient or vehicle.

In another embodiment, provided herein are compositions, comprising an effective amount of Compound 1 and a pharmaceutically acceptable carrier or vehicle, wherein a pharmaceutically acceptable carrier or vehicle can comprise an excipient, diluent, or a mixture thereof. In one embodiment, the composition is a pharmaceutical composition.

The compositions can be in the form of tablets, chewable tablets, capsules, solutions, parenteral solutions, troches, suppositories and suspensions and the like. Compositions can be formulated to contain a daily dose, or a convenient fraction of a daily dose, in a dosage unit, which may be a single tablet or capsule or convenient volume of a liquid. In one embodiment, the solutions are prepared from water-soluble salts, such as the hydrochloride salt. In general, all of the compositions are prepared according to known methods in pharmaceutical chemistry. Capsules can be prepared by mixing Compound 1 with a suitable carrier or diluent and filling the proper amount of the mixture in capsules. The usual carriers and diluents include, but are not limited to, inert powdered substances such as starch of many different kinds, powdered cellulose, especially crystalline and microcrystalline cellulose, sugars such as fructose, mannitol and sucrose, grain flours and similar edible powders.

Tablets can be prepared by direct compression, by wet granulation, or by dry granulation. Their formulations usually incorporate diluents, binders, lubricants and disintegrators as well as the compound. Typical diluents include, for example, various types of starch, lactose, mannitol, kaolin, calcium phosphate or sulfate, inorganic salts such as sodium chloride and powdered sugar. Powdered cellulose derivatives are also useful. In one embodiment, the pharmaceutical composition is lactose-free. Typical tablet binders are substances such as starch, gelatin and sugars such as lactose, fructose, glucose and the like. Natural and synthetic gums are also convenient, including acacia, alginates, methylcellulose, polyvinylpyrrolidine and the like. Polyethylene glycol, ethylcellulose and waxes can also serve as binders.

A lubricant might be necessary in a tablet formulation to prevent the tablet and punches from sticking in the die. The lubricant can be chosen from such slippery solids as talc, magnesium and calcium stearate, stearic acid and hydrogenated vegetable oils. Tablet disintegrators are substances that swell when wetted to break up the tablet and release the compound. They include starches, clays, celluloses, algins and gums. More particularly, corn and potato starches, methylcellulose, agar, bentonite, wood cellulose, powdered natural sponge, cation-exchange resins, alginic acid, guar gum, citrus pulp and carboxymethyl cellulose, for example, can be used as well as sodium lauryl sulfate. Tablets can be coated with sugar as a flavor and sealant, or with film-forming protecting agents to modify the dissolution properties of the tablet. The compositions can also be formulated as chewable tablets, for example, by using substances such as mannitol in the formulation.

When it is desired to administer Compound 1 as a suppository, typical bases can be used. Cocoa butter is a traditional suppository base, which can be modified by addition of waxes to raise its melting point slightly. Water-miscible suppository bases comprising, particularly, polyethylene glycols of various molecular weights are in wide use.

The effect of Compound 1 can be delayed or prolonged by proper formulation. For example, a slowly soluble pellet of Compound 1 can be prepared and incorporated in a tablet or capsule, or as a slow-release implantable device. The technique also includes making pellets of several different dissolution rates and filling capsules with a mixture of the pellets. Tablets or capsules can be coated with a film that resists dissolution for a predictable period of time. Even the parenteral preparations can be made long-acting, by dissolving or suspending Compound 1 in oily or emulsified vehicles that allow it to disperse slowly in the serum.

### 4.7 KITS

Also described herein are kits comprising a TOR kinase inhibitor.

Also described herein are kits comprising a TOR kinase inhibitor and means for monitoring patient response to administration of said TOR kinase inhibitor. The patient may have ETS overexpressing Ewings sarcoma. In particular embodiments, the patient response measured is inhibition of disease progression, inhibition of tumor growth, reduction of primary and/or secondary tumor(s), relief of tumor-related symptoms, improvement in quality of life, delayed appearance of primary and/or secondary tumors, slowed development of primary and/or secondary tumors, decreased occurrence of primary and/or secondary tumors, slowed or decreased severity of secondary effects of disease, arrested tumor growth or regression of tumor.

Also described herein are kits comprising a TOR kinase inhibitor and means for measuring the amount of inhibition of phosphorylation of S6RP, 4E-BP1 and/or AKT in a patient. The kits may comprise means for measuring inhibition of phosphorylation of S6RP, 4E-BP1 and/or AKT in circulating blood or tumor cells and/or skin biopsies or tumor biopsies/aspirates of a patient. Also described herein are kits comprising a TOR kinase inhibitor and means for measuring the amount of inhibition of phosphorylation as assessed by comparison of the amount of phospho- S6RP, 4E-BP1 and/or AKT before, during and/or after administration of the TOR kinase inhibitor. The patient may have ETS overexpressing Ewings sarcoma.

Also described herein are kits comprising a TOR kinase inhibitor and means for measuring the amount of inhibition of DNA-dependent protein kinase (DNA-PK) activity in a patient. The kits may comprise means for measuring the amount of inhibition of DNA-dependent protein kinase (DNA-PK) activity in a skin sample and/or a tumor biopsy/aspirate of a patient. The kits may comprise a means for measuring the amount of pDNA-PK S2056 in a skin sample and/or a tumor biopsy/aspirate of patient. The skin sample may be irradiated by UV light. Also described herein are kits comprising a TOR kinase inhibitor and means for measuring the amount of inhibition of DNA-dependent protein kinase (DNA-PK) activity before, during and/or after administration of the TOR kinase inhibitor. Also described herein are kits comprising a TOR kinase inhibitor and means for measuring the amount of phosphorylated DNA-PK S2056 before, during and/or after administration of the TOR kinase inhibitor. The patient may have ETS overexpressing Ewings sarcoma.

The kits described herein may comprise an amount of a TOR kinase inhibitor effective for treating or preventing ETS overexpressing Ewings sarcoma. The kits described herein may comprise a TOR kinase inhibitor having molecular formula C₁₆H₁₆N₈O. The kits described herein may comprise Compound 1.

The kits described herein may further comprise instructions for use, such as for administering a TOR kinase inhibitor and/or monitoring patient response to administration of a TOR kinase inhibitor.

### 5. EXAMPLES

### 5.1 BIOLOGICAL EXAMPLES

### 5.1.1 Biochemical assays

**mTOR HTR-FRET Assay.** The following is an example of an assay that can be used to determine the TOR kinase inhibitory activity of a test compound. TOR kinase inhibitors were dissolved in DMSO and prepared as 10 mM stocks and diluted appropriately for the experiments. Reagents were prepared as follows:

"Simple TOR buffer" (used to dilute high glycerol TOR fraction): 10 mM Tris pH 7.4, 100 mM NaCl, 0.1% Tween-20, 1 mM DTT. Invitrogen mTOR (cat#PV4753) was diluted in this buffer to an assay concentration of 0.200 µg/mL.

ATP/Substrate solution: 0.075 mM ATP, 12.5 mM MnCl₂, 50 mM Hepes, pH 7.4, 50 mM β-GOP, 250 nM Microcystin LR, 0.25 mM EDTA, 5 mM DTT, and 3.5 µg/mL GST-p70S6.

Detection reagent solution: 50 mM HEPES, pH 7.4, 0.01% Triton X-100, 0.01% BSA, 0.1 mM EDTA, 12.7 µg/mL Cy5-αGST Amersham (Cat#PA92002V), 9 ng/mL α-phospho p70S6 (Thr389) (Cell Signaling Mouse Monoclonal #9206L), 627 ng/mL α-mouse Lance Eu (Perkin Elmer Cat#AD0077).

To 20 µL of the Simple mTor buffer is added 0.5 µL of test compound in DMSO. To initiate the reaction 5 µL of ATP/Substrate solution was added to 20 µL of the Simple TOR buffer solution (control) and to the compound solution prepared above. The assay was stopped after 60 min by adding 5 µL of a 60 mM EDTA solution; 10 µL of detection reagent solution was then added and the mixture was allowed to sit for at least 2 hours before reading on a Perkin-Elmer Envision Microplate Reader set to detect LANCE Eu TR-FRET (excitation at 320 nm and emission at 495/520 nm).

TOR kinase inhibitors were tested in the mTOR HTR-FRET assay and were found to have activity therein, with certain compounds having an IC₅₀ below 10 µM in the assay, with some compounds having an IC₅₀ between and 0.005 nM and 250 nM, others having an IC₅₀ between and 250 nM and 500 nM, others having an IC₅₀ between 500 nM and 1 µM, and others having an IC₅₀ between 1 µM and 10 µM.

**DNA-PK assay.** DNA-PK assays were performed using the procedures supplied in the Promega DNA-PK assay kit (catalog # V7870). DNA-PK enzyme was purchased from Promega (Promega cat#V5811).

Selected TOR kinase inhibitors as described herein have, or are expected to have, an IC₅₀ below 10 µM in this assay, with some TOR kinase inhibitors as described herein having an IC₅₀ below 1 µM, and others having an IC₅₀ below 0.10 µM.

### 5.1.2 Cell based assays

CADO-ES1 (DSMZ), RD-ES (ATCC), COG-10 and COG-258 Ewing's sarcoma cell lines and the EWS-fusion negative A-204 (ATCC) and SA-O2 (ATCC) are grown in RPMI 1640 (Invitrogen, Carlsbad, CA) supplemented with 10% FBS (Invitrogen) in 5% CO₂ cell culture incubator. All cultures are maintained with 50 units/mL of penicillin/streptomycin (Invitrogen).

**Chemosensitivity assay.** Five thousand cells are plated in each well of a 96-well plate in sets of ten. Cells are treated with a single dose of TOR kinase inhibitor for 72 hours. WST assays (Roche) are performed according to company protocol. Briefly, 10% WST was added to each well, the plates were incubated at 37 °C with 5% CO₂ for 4 hours and the OD was measured.

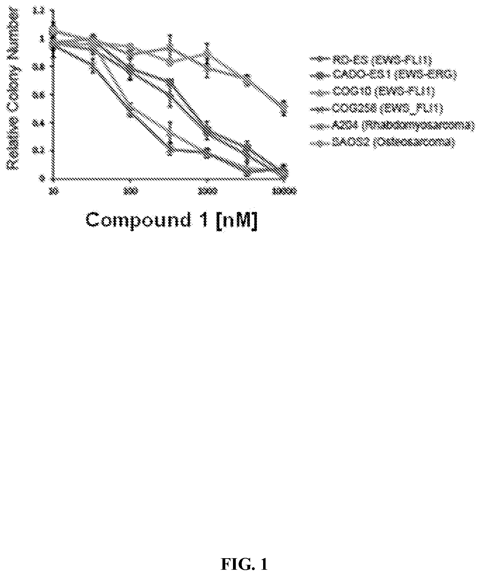

**Soft agar colony formation.** A solid layer of 1% agarose is used to coat each well of a 96-well plate. After allowing two hours to cool, five hundred Ewing's cells are suspended in a 33% agarose (10% solution), 33% media with 2x FBS and 33% cell suspension in quadruplicate wells of a 96-well plate. Cells are treated with or without TOR kinase inhibitor in an upper layer of media. Every three days, the media is exchanged for fresh TOR kinase inhibitor containing media. After 3-weeks in culture, images are taken on a 10x objective lens and colonies are counted for each condition. This assay is run in three independent experiments and data is shown in FIG. 1. As can be seen, ETS overexpressing Ewing sarcoma cell lines are more sensitive to Compound 1 inhibition.

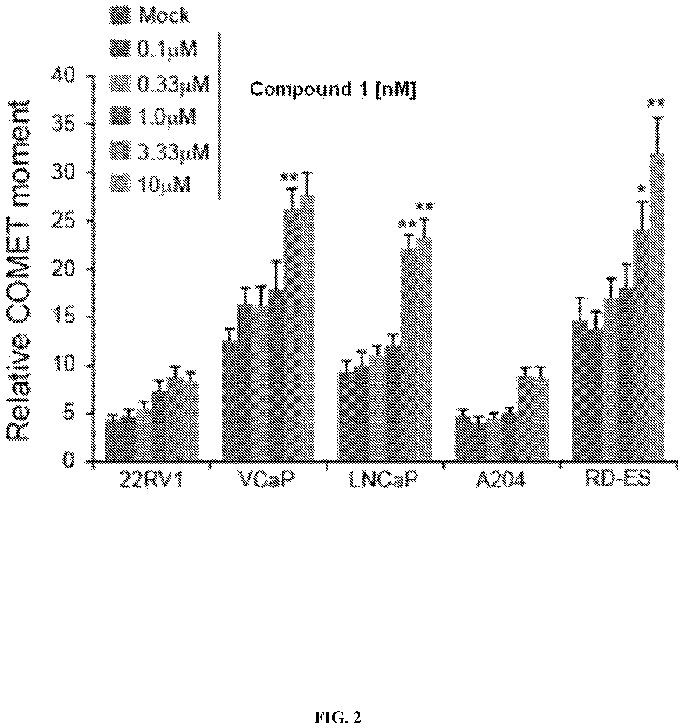

**DNA damage assays: COMET assay.** Ewing's sarcoma cell lines are seeded at 250,000 cells/mL in a 6-well plate 24 hours prior to treatment with TOR kinase inhibitor or vehicle control. After 48 hours, cells are trypsinized, harvested by centrifugation and re-suspended in PBS. Cell counts are normalized to 1 x 10⁵ cells/mL. Suspended cells (25 µL) are mixed with 250 µL 1.0% ultrapure low melting point agarose (Invitrogen) made in 1x Tris-Borate buffer. The agarose-cell mixture is dropped onto slides allowed to solidify at 4 °C in the dark for 20 minutes before immersion in COMET assay lysis solution (Trevigen, Gaithersburg, MD) at 4 °C in the dark for 45 minutes. Excess buffer is removed and slides are submerged in freshly prepared neutral solution (Tris Base 60.57 g, sodium acetate 204.12 g, dissolved in 450 ml of dH₂O, adjusted to pH = 9.0 with glacial acetic acid.) at room temperature in the dark for 40 minutes. Slides are washed twice by immersing in 1 X TBE buffer prior to neutral electrophoresis at 20 volts for 60 minutes. Slides are fixed in 70% ethanol for 5 minutes. Following air drying of the agarose, slides are stained with SYBR Green dye (Invitrogen) and images are collected with a 10x and 40x objective lens. COMET tail moments are assessed using COMETscore.v1.5 (AutoCOMET.com, Sumerduck, VA) image processing software as described by the manufacturer with greater than 100 cells analyzed in triplicate experiments (FIG. 2). As can be seen, Compound 1 potentiates DNA damage in ETS positive cells. A similar effect was observed in prostate cancer cell lines that were ETS fusion protein negative (22RV1) or positive (VCaP and LNCaP).

### 5.1.3 In vivo Assays:

Five week-old male SCID mice (CB.17. SCID), are purchased from Charles River, Inc. (Charles River Laboratory, Wilmington, MA). RD-ES cells (2 × 10⁶ cells/injection), or control A-204 cells are resuspended in 100 µL of saline with 50% Matrigel (BD Biosciences, Becton Drive, NJ) and are implanted subcutaneously into the left and right flank regions of the mice. Mice are anesthetized using a cocktail of xylazine (80-120 mg/kg, IP) and ketamine (10 mg/kg, IP) for chemical restraint before tumor implantation. All tumors are staged for two weeks before starting TOR kinase inhibitor treatment. At the beginning of the third week, mice with RD-ES tumors (10 tumors per treatment group, average size 150-200 mm³) are treated with TOR kinase inhibitor. Growth in tumor volume is recorded weekly by using digital calipers and tumor volumes are calculated using the formula (π/6) (L × W²), where L = length of tumor and W = width. Loss of body weight during the course of the study is also monitored weekly. At the end of the xenograft studies, mice are sacrificed, and tumors are harvested. All tumors will be divided for collection of protein, RNA and creation of formalin-fixed, paraffin-embedded blocks, and will be analyzed for phosphorylation of DNA-PK and analysis of TOR pathway biomarkers. Because the RD-ES xenograft model is known to metastasize to lungs, lungs are harvested, maintained in formalin, and subsequently embedded in paraffin. The number of metastases per lung is quantitated via light microscopy.

### 5.1.4 Clinical Study

### Phase 1A/1B, Multi-Center, Open-Label, Dose Finding Study to Assess the Safety, Tolerability, Pharmacokinetics and Preliminary Efficacy of Compound 1 Administered Orally to Subjects with ETS Overexpressing Ewings Sarcoma

Compound 1 (a TOR kinase inhibitor set forth herein having molecular formula C₁₆H₁₆N₈O) will be administered orally to subjects with ETS overexpressing Ewings sarcoma. The safety and tolerability of Compound 1 in humans, as well as the efficacy, will be evaluated in this study. The study will be conducted in two parts: dose escalation (Part A) and dose expansion (Part B). Subjects will be enrolled sequentially in Part A. Enrollment in Part B will be stratified by tumor type.

Compound 1 will be available in three strengths (0.25 mg, 1.0 mg and 5.0 mg) presented in gelatin capsules containing only the active pharmaceutical ingredient. The capsules will be packaged in high density polyethylene (HDPE) bottles, fitted with induction seals and child-resistant polypropylene closures. Research pharmacists will repackage and dispense appropriately for each subject.

Between 30 and 60 subjects will be enrolled in Part A, designed to establish initial toxicity.

Part B will consist of approximately 100 subjects with prespecified types of tumors, including E-twenty six (ETS) overexpressing Ewings sarcoma, to further assess the safety profile of Compound 1 and provide efficacy information. Tumor response rate will be assessed by tumor type and dose level. The Part B population will be defined by the efficacy seen during Part A and by data from ongoing preclinical studies.

The overall study design will be comprised of a Screening Period (Day -28 to Day 1), a Treatment and Evaluation Period (28-day QD (and/or BID) cycles until tumor progression, unacceptable toxicity or subject/physician decision to discontinue adiministration of Compound 1) and an End of Treatment and Follow-up Period (end of treatment procedures within 21 days of last dose; follow-up for 28 days after last dose for final safety assessment).

Subjects will start Compound 1 QD or BID dosing (or other suitable regimen) on Cycle 1 Day 1 and receive daily treatment in 28-day cycles. Compound 1 may be discontinued when there is evidence of tumor progression, but subjects can continue to receive study drug as long as the Investigator considers they are deriving benefit. Compound 1 administration will be discontinued when there is unacceptable toxicity, or the subject decides to withdraw from the study.

Compound 1 will be administered orally either once or twice daily (or other suitable dosing regimen) with no rest period between cycles. Each QD dose will be taken in the morning with at least 200 mL of water, with the subject having fasted overnight (minimum of 6 hours). Food intake will be delayed until at least 90 minutes after dosing on the days Compound 1 is taken at home. On clinic visit days, the morning Compound 1 dose will be administered in the clinic after any predose tests have been completed. Food may be taken after all fasting tests have been completed but in no case earlier than 90 minutes after dosing (3 hours after dosing on Day 15). For subjects receiving Compound 1 QD where troublesome related GI symptoms, fatigue or other symptoms persist beyond the end of Cycle 1, dosing may be moved to later in the day providing the subject can maintain a 3-hour separation between Compound 1 administration and the last intake of food and a 90-minute delay before ingesting further food. Compound 1 may be taken up to 12 hours late if dosing has been delayed on a single day; otherwise that dose should be omitted.

Compound 1 will be administered initially as a QD regimen.

Doses will be administered in an escalating manner following satisfactory review of safety data from the lower doses. There will be a minimum of 28 days after the first dose has been administered to the last subject between dose escalations. Within each cohort, enrollment will be staggered so that there is a minimum of 24 hours between Cycle 1 Day 1 for each subject in order to evaluate initial toxicity.

Each cycle of Compound 1 lasts 28 days and there is no rest period between cycles. Subjects may continue to receive Compound 1 for as long as they derive benefit from treatment, as judged by the Investigator. Compound 1 administration will be discontinued when there is evidence of disease progression, unacceptable toxicity or when either the subject or Investigator decides to discontinue it.

In Part A, cohorts of subjects will initially receive QD ascending doses of Compound 1 to measure PK and to identify the MTD. In Part A, 0.5 mg QD is the Compound 2 starting dose. A modified accelerated titration design (Simon, R., Freidlin, B., Rubinstein, L., et al. Accelerated titration designs for Phase I clinical. trials in oncology, J Nat Canc Institute 1997;.89, (15): 1138-1147) will be used to establish initial toxicity. During the accelerated phase, initial cohorts of one subject will be given Compound 1 at dose increments of 100% until the first instance of first-Cycle grade 2 or higher toxicity suspected to be drug-related, at which point the accelerated phase will stop and this particular cohort will be expanded to a total of 6 subjects. Subsequently, a standard escalation dosing schedule with approximately 50% dose increments and 6 subjects per cohort will be initiated in order to establish the NTD and MTD. Smaller increments and additional subjects within a dose cohort may also be evaluated, if necessary, based on toxicity, PK/PD results or tumor biopsy findings.

Based on interim PK and PD results from initial dose cohorts, a twice-daily (BID) dosing regimen will also be evaluated in part A. This will be initiated in cohorts of 6 subjects at or below a total daily dose level already shown to be tolerable, but divided into two equal doses administered approximately 12 hours apart. Thereafter, dose escalation for QD and BID dosing cohorts may occur independently. Intermittent dosing schedules of comparable or lower dose intensity than continuous daily dosing may also be considered for evaluation..

A dose will be considered to be non-tolerated if 2 or more out of 6 evaluable subjects in a dose cohort experience DLT during Cycle 1. When a NTD is defined, dose escalation will be stopped. The MTD will be defined as the last dose tested below the NTD with 0 or 1 out of 6 evaluable subjects experiencing DLT during Cycle 1. An intermediate dose (*i.e.,* one between the NTD and the last dose level before the NTD) or additional subjects within any dose cohort may be required to more precisely determine the MTD more precisely, as may alternate regimens if emerging PK-PD results suggest these may be appropriate.

In Part B, subjects may start Compound 1 on a QD or BID regimen at the MTD and/or lower dose levels based on safety, PK and PD data from Part A. In Part B, approximately 100 subjects will be evaluated for safety and antitumor activity after every two cycles of therapy.

All subjects who receive at least one dose of Compound 1 will be evaluable for safety. In Part A, a subject evaluable for dose-limiting toxicity (DLT) is defined as one who, in the first 28 days after Cycle 1 dosing began, either (a) received at least 21 of the planned 28 doses of Compound 1 at the cohort-specified dose and has sufficient data for safety evaluation by the SRC, or (b) experienced study drug-related DLT. Non-evaluable subjects will be replaced in the dosing cohort. In Part B, an efficacy evaluable subject for tumor response is defined as one who received at least one cycle of Compound 1, and have baseline and at least one post-baseline efficacy assessment.

In Parts A and B, dose reductions are permitted in any cycle, including Cycle 1. Dose reductions that occur in Cycle 1 during Part A will constitute DLT, but subjects will be allowed to continue on study drug at the reduced dose. National Cancer Institute Common Terminology Criteria for Adverse Events (NCI CTCAE) Version 4, 2009 will be used to grade AEs.

When a dose reduction is indicated, the next lower dose level will be on a QD or BID schedule will be selected. For BID dose reductions below the starting dose of 10 mg BID, 8 mg BID and 4 mg BID will be selected. Two dose reductions are allowed. Additional PK evaluations may be conducted at modified dose level(s) in order to characterize intrasubject PK profiles with alternate doses.

In Part A, intrasubject dose escalation beyond the dose initially assigned to a subject is not permitted in Cycle 1. Those continuing to take Compound 1 beyond Cycle 1 may, following approval by the SRC, have the dose level increased providing the alternative dose level has been shown to be well tolerated by at least one cohort of other subjects in this study. In these instances, additional PK evaluation at the higher dose level may be conducted. In Part B, no dose escalation beyond the MTD is allowed. Subjects with Ewings sarcoma, who start dosing one dose level below the selected Part B dose for all other tumors, may increase their dose if no DLT is observed during Cycle 1.

The primary objectives of this Phase 1a/1b study are to determine the safety, tolerability, NTD and MTD of Compound 1 when administered orally to adult subjects and to determine the PK characteristics of oral Compound 1. The secondary objectives are to evaluate the extent of inhibition of phosphorylation of S6RP and/or 4E-BP1 for mTORC1 activity and AKT and/or other relevant biomarkers for mTORC2 activity in blood, skin and/or tumor biopsies/aspirates and to explore the antitumor activity of Compound 1 at selected dose levels/regimens by tumor type. Further secondary objectives are to evaluate the inhibition of DNA-PK activity in skin samples irradiated by UV light and/or tumor biopsies/aspirates using pDNA-PK S2056 and other relevant biomarkers for DNA damage pathways before and during Compound 1 treatment.

In the following, statistical analyses will be performed by study phase, dose level, dosing regimen and tumor cohort as needed or applicable.

The study population definitions are as follows: (a) Intent-to-Treat (ITT) Population - All subjects who take at least one dose of Compound 1; (b) Safety Population - All subjects who take at least one dose of Compound 1, which is the same as ITT population for this study; (c) Efficacy Evaluable (EE) Population - All ITT subjects who meet eligibility criteria, complete at least one cycle of Compound 1, and have baseline and at least one valid post-baseline efficacy assessment.

Subject enrollment will be curtailed when up to 20 evaluable subjects have been enrolled in each tumor type and dose level/regimen. In Part B as a whole, sample sizes are not based on statistical calculation but rather on clinical empirical and practical considerations traditionally used for Phase 1 studies of this kind.

All efficacy evaluable subjects in the Part B portion will be included for efficacy analysis. Efficacy will be analyzed by each tumor type once all subjects have withdrawn from the study or completed 6 cycles. Two-sided ninety-five percent confidence intervals of the response rate will be provided by tumor type. A case-by-case description of all subjects who exhibited a complete or partial response during the Part A segment will be provided. A descriptive analysis of other evidence of anti-tumor activity will be provided based on clinical, radiographic, and biologic assessments of efficacy.

Subjects will be evaluated for efficacy during even-numbered cycles. The primary efficacy variable is response rate. Tumor response will be based on the Response Evaluation Criteria in Solid Tumors (RECIST 1.1) for Ewings sarcoma. Other supplementary efficacy variables, including CTC assessments, will be summarized using frequency tabulations for categorical variables or descriptive statistics for continuous variables.

For both the dose escalation and dose expansion parts of this protocol, inclusion criteria are: (a) Understand and voluntarily sign an informed consent document before any study-related assessments/procedures are conducted; (b) Men and women, 18 years or older, with histological or cytological confirmation of ETS overexpressing Ewings sarcoma, including those who have progressed on (or not been able to tolerate) standard anticancer therapy or for whom no other conventional therapy exists; (c) Consent to screening tumor biopsy (Part A optional; Part B mandatory except as specified for individual tumor types below); (d) ECOG PS of 0 or 1; (e) The following laboratory values: (1) Absolute neutrophil count (ANC) ≥ 1.5 x 109/L; (2) Hemoglobin (Hgb) ≥ 9 g/dl; (3) Platelets (plt) ≥ 100 x 109/L; (4) Potassium within normal range, or correctable with supplements; (5) AST/SGOT and ALT/SGPT ≤ 2.5 x Upper Limit of Normal (ULN) or ≤ 5.0 x ULN if liver tumor is present; (6) Serum total bilirubin ≤ 1.5 x ULN; (7) Serum creatinine ≤ 1.5 x ULN, or 24-hr clearance ≥ 50 mL/min; and (8) Negative serum or urine pregnancy test within 72 hrs before starting study treatment in females of childbearing potential; and (f) Able to adhere to the study visit schedule and other protocol requirements.

For the dose expansion part (Part B) of this protocol, inclusion criteria are: (a) Subject consent to retrieve formalin-fixed, paraffin-embedded (FFPE) archival tumor tissue, either in tumor blocks or sectioned/mounted specimens; and (b) Histologically-confirmed ETS overexpressing Ewings sarcoma (recurrent or metastatic Ewings sarcoma following standard multimodality cancer treatment; age 12 years or older, with appropriate assent for subjects under 18; measurable disease according to RECIST v1.1;
bisphosphonates or denusomab are allowed in stable doses; and optional paired tumor biopsies if tumor is accessible).

For both the dose escalation and dose expansion parts of this protocol, exclusion criteria are: (a) Symptomatic central nervous system metastases; (b) Known acute or chronic pancreatitis; (c) Any peripheral neuropathy ≥ NCI CTCAE grade 2; (d) Persistent diarrhea or malabsorption ≥ NCI CTCAE grade 2, despite medical management. Impaired ability to swallow; (e) Impaired cardiac function or clinically significant cardiac diseases; (f) Diabetes mellitus on active treatment; (g) Other concurrent severe and/or uncontrolled concomitant medical conditions (e.g. active or uncontrolled infection) that could cause unacceptable safety risks or compromise compliance with the protocol; (h) Prior systemic cancer-directed treatments or investigational modalities ≤ 5 half lives or 4 weeks, whichever is shorter, prior to starting study drug or who have not recovered from side effects of such therapy; (i) Major surgery ≤ 2 weeks prior to starting study drug or who have not recovered from side effects of such therapy; (j) Pregnancy or breast feeding; (k) Adults of reproductive potential not employing two forms of birth control; (1) Known HIV infection; (m) Known chronic hepatitis B or C virus (HBV/HCV) infection, unless this is comorbidity in subjects with HCC; (n) Any significant medical condition, laboratory abnormality, or psychiatric illness, including the inability to swallow capsules, that would prevent subjects from participating in the study; (o) Any condition including the presence of laboratory abnormalities, which places subjects at unacceptable risk if they were to participate in the study; (p) Any condition that confounds the ability to interpret study data; or (q) Concurrent active second malignancy for which the subject is receiving therapy, excluding non-melanomatous skin cancer or carcinoma in situ of the cervix.

For the dose expansion part (Part B) of this protocol, exclusion criteria are: Prior treatment with agents targeting both mTOR complexes (dual TORC1+TORC2 inhibitors) and/or PI3K/AKT pathways. However, prior treatment with isolated TORC1 inhibitors (e.g., rapalogs) is allowed in both parts of this study.

In some embodiments, patients undergoing the clinical protocol provided herein will show a positive tumor response, such as inhibition of tumor growth or a reduction in tumor size. In certain embodiments, patients undergoing the clinical protocol provided herein will achieve a Response Evaluation Criteria in Solid Tumors (for example, RECIST 1.1) of complete response, partial response or stable disease after administration of an effective amount of Compound 1. In certain embodiments, patients undergoing the clinical protocol provided herein will show increased survival without tumor progression. In some embodiments, patients undergoing the clinical protocol provided herein will show inhibition of disease progression, inhibition of tumor growth, reduction of primary tumor, relief of tumor-related symptoms, inhibition of tumor secreted factors (including tumor secreted hormones, such as those that contribute to carcinoid syndrome), delayed appearance of primary or secondary tumors, slowed development of primary or secondary tumors, decreased occurrence of primary or secondary tumors, slowed or decreased severity of secondary effects of disease, arrested tumor growth and regression of tumors, increased Time To Progression (TTP), increased Progression Free Survival (PFS), and/or increased Overall Survival (OS), among others.

## Claims

1. 1-ethyl-7-(2-methyl-6-(1H-1,2,4-triazol-3-yl)pyridin-3-yl)-3,4-dihydropyrazino[2,3-b]pyrazin-2(1H)-one or a pharmaceutically acceptable salt, clathrate, solvate, stereoisomer, tautomer or isotopologue thereof for use in a method for treating E-twenty six (ETS) overexpressing Ewings sarcoma, the method comprising administering an effective amount of 1-ethyl-7-(2-methyl-6-(1H-1,2,4-triazol-3-yl)pyridin-3-yl)-3,4-dihydropyrazino[2,3-b]pyrazin-2(1H)-one or a pharmaceutically acceptable salt, clathrate, solvate, stereoisomer, tautomer or isotopologue thereof to a patient having ETS overexpressing Ewings sarcoma.

2. 1-ethyl-7-(2-methyl-6-(1H-1,2,4-triazol-3-yl)pyridin-3-yl)-3,4-dihydropyrazino[2,3-b]pyrazin-2(1H)-one or a pharmaceutically acceptable salt, clathrate, solvate, stereoisomer, tautomer or isotopologue thereof for use of claim 1, wherein said patient is administered about 0.5 mg/day to about 120 mg/day of 1-ethyl-7-(2-methyl-6-(1H-1,2,4-triazol-3-yl)pyridin-3-yl)-3,4-dihydropyrazino[2,3-b]pyrazin-2(1H)-one or a pharmaceutically acceptable salt, clathrate, solvate, stereoisomer, tautomer or isotopologue thereof.

3. 1-ethyl-7-(2-methyl-6-(1H-1,2,4-triazol-3-yl)pyridin-3-yl)-3,4-dihydropyrazino[2,3-b]pyrazin-2(1H)-one or a pharmaceutically acceptable salt, clathrate, solvate, stereoisomer, tautomer or isotopologue thereof for use of claim 2, wherein said patient is administered 0.5 mg/day, 1 mg/day, 2 mg/day, 4 mg/day, 8 mg/day, 16 mg/day, 20 mg/day, 30 mg/day, 45 mg/day, 60 mg/day, 90 mg/day, or 120 mg/day of 1-ethyl-7-(2-methyl-6-(1H-1,2,4-triazol-3-yl)pyridin-3-yl)-3,4-dihydropyrazino[2,3-b]pyrazin-2(1H)-one or a pharmaceutically acceptable salt, clathrate, solvate, stereoisomer, tautomer or isotopologue thereof.

4. 1-ethyl-7-(2-methyl-6-(1H-1,2,4-triazol-3-yl)pyridin-3-yl)-3,4-dihydropyrazino[2,3-b]pyrazin-2(1H)-one or a pharmaceutically acceptable salt, clathrate, solvate, stereoisomer, tautomer or isotopologue thereof for use of claim 1, wherein said patient is administered a unit dosage form comprising 0.25 mg, 1.0 mg 5.0 mg, 7.5 mg, or 10 mg of 1-ethyl-7-(2-methyl-6-(1H-1,2,4-triazol-3-yl)pyridin-3-yl)-3,4-dihydropyrazino[2,3-b]pyrazin-2(1H)-one or a pharmaceutically acceptable salt, clathrate, solvate, stereoisomer, tautomer or isotopologue thereof.

5. 1-ethyl-7-(2-methyl-6-(1H-1,2,4-triazol-3-yl)pyridin-3-yl)-3,4-dihydropyrazino[2,3-b]pyrazin-2(1H)-one or a pharmaceutically acceptable salt, clathrate, solvate, stereoisomer, tautomer or isotopologue thereof for use in a method for improving the Response Evaluation Criteria in Solid Tumors (RECIST 1.1) of a patient, the method comprising administering an effective amount of 1-ethyl-7-(2-methyl-6-(1H-1,2,4-triazol-3-yl)pyridin-3-yl)-3,4-dihydropyrazino[2,3-b]pyrazin-2(1H)-one or a pharmaceutically acceptable salt, clathrate, solvate, stereoisomer, tautomer or isotopologue thereof to a patient having E-twenty six (ETS) overexpressing Ewings sarcoma.

6. 1-ethyl-7-(2-methyl-6-(1H-1,2,4-triazol-3-yl)pyridin-3-yl)-3,4-dihydropyrazino[2,3-b]pyrazin-2(1H)-one or a pharmaceutically acceptable salt, clathrate, solvate, stereoisomer, tautomer or isotopologue thereof for use of claim 1 or 5, wherein the E-twenty six (ETS) overexpressing Ewings sarcoma is that in which the PI3K/mTOR pathway is activated.

7. 1-ethyl-7-(2-methyl-6-(1H-1,2,4-triazol-3-yl)pyridin-3-yl)-3,4-dihydropyrazino[2,3-b]pyrazin-2(1H)-one or a pharmaceutically acceptable salt, clathrate, solvate, stereoisomer, tautomer or isotopologue thereof for use of claim 6, wherein the E-twenty six (ETS) overexpressing Ewings sarcoma is that in which the PI3K/mTOR pathway is activated due to PTEN loss, a PIK3Ca mutation or EGFR overexpression, or a combination thereof.

## Patentansprüche

1. 1-Ethyl-7-(2-methyl-6-(1H-1,2,4-triazol-3-yl)pyridin-3-yl)-3,4-dihydropyrazino[2,3-b]pyrazin-2(1H)-on oder ein pharmazeutisch verträgliches Salz, Clathrat, Solvat, Stereoisomer, Tautomer oder Isotopologes davon zur Verwendung in einem Verfahren zum Behandeln von E-twenty six (ETS) überexprimierendem Ewing-Sarkom, wobei das Verfahren Verabreichen einer wirksamen Menge an 1-Ethyl-7-(2-methyl-6-(1H-1,2,4-triazol-3-yl)pyridin-3-yl)-3,4-dihydropyrazino[2,3-b]pyrazin-2(1H)-on oder einem pharmazeutisch verträglichen Salz, Clathrat, Solvat, Stereoisomer, Tautomer oder Isotopologen davon an einen Patienten mit ETS überexprimierendem Ewing-Sarkom umfasst.

2. 1-Ethyl-7-(2-methyl-6-(1H-1,2,4-triazol-3-yl)pyridin-3-yl)-3,4-dihydropyrazino[2,3-b]pyrazin-2(1H)-on oder ein pharmazeutisch verträgliches Salz, Clathrat, Solvat, Stereoisomer, Tautomer oder Isotopologes davon zur Verwendung nach Anspruch 1, wobei dem Patienten etwa 0,5 mg/Tag bis etwa 120 mg/Tag 1-Ethyl-7-(2-methyl-6-(1H-1,2,4-triazol-3-yl)pyridin-3-yl)-3,4-dihydropyrazino[2,3-b]pyrazin-2(1H)-on oder ein pharmazeutisch verträgliches Salz, Clathrat, Solvat, Stereoisomer, Tautomer oder Isotopologes davon verabreicht werden.

3. 1-Ethyl-7-(2-methyl-6-(1H-1,2,4-triazol-3-yl)pyridin-3-yl)-3,4-dihydropyrazino[2,3-b]pyrazin-2(1H)-on oder ein pharmazeutisch verträgliches Salz, Clathrat, Solvat, Stereoisomer, Tautomer oder Isotopologes davon zur Verwendung nach Anspruch 2, wobei dem Patienten 0,5 mg/Tag, 1 mg/Tag, 2 mg/Tag, 4 mg/Tag, 8 mg/Tag, 16 mg/Tag, 20 mg/Tag, 30 mg/Tag, 45 mg/Tag, 60 mg/Tag, 90 mg/Tag oder 120 mg/Tag 1-Ethyl-7-(2-methyl-6-(1H-1,2,4-triazol-3-yl)pyridin-3-yl)-3,4-dihydropyrazino[2,3-b]pyrazin-2(1H)-on oder ein pharmazeutisch verträgliches Salz, Clathrat, Solvat, Stereoisomer, Tautomer oder Isotopologes davon verabreicht werden.

4. 1-Ethyl-7-(2-methyl-6-(1H-1,2,4-triazol-3-yl)pyridin-3-yl)-3,4-dihydropyrazino[2,3-b]pyrazin-2(1H)-on oder ein pharmazeutisch verträgliches Salz, Clathrat, Solvat, Stereoisomer, Tautomer oder Isotopologes davon zur Verwendung nach Anspruch 1, wobei dem Patienten eine Einheitsdosierungsform, umfassend 0,25 mg, 1,0 mg, 5,0 mg, 7,5 mg oder 10 mg 1-Ethyl-7-(2-methyl-6-(1H-1,2,4-triazol-3-yl)pyridin-3-yl)-3,4-dihydropyrazino[2,3-b]pyrazin-2(1H)-on oder ein pharmazeutisch verträgliches Salz, Clathrat, Solvat, Stereoisomer, Tautomer oder Isotopologes davon, verabreicht wird.

5. 1-Ethyl-7-(2-methyl-6-(1H-1,2,4-triazol-3-yl)pyridin-3-yl)-3,4-dihydropyrazino[2,3-b]pyrazin-2(1H)-on oder ein pharmazeutisch verträgliches Salz, Clathrat, Solvat, Stereoisomer, Tautomer oder Isotopologes davon zur Verwendung in einem Verfahren zum Verbessern der Bewertung des Ansprechens der Behandlung bei soliden Tumoren (RECIST 1.1) von einem Patienten, wobei das Verfahren Verabreichen einer wirksamen Menge an 1-Ethyl-7-(2-methyl-6-(1H-1,2,4-triazol-3-yl)pyridin-3-yl)-3,4-dihydropyrazino[2,3-b]pyrazin-2(1H)-on oder einem pharmazeutisch verträglichen Salz, Clathrat, Solvat, Stereoisomer, Tautomer oder Isotopologen davon an einen Patienten mit E-twenty six (ETS) überexprimierendem Ewing-Sarkom umfasst.

6. 1-Ethyl-7-(2-methyl-6-(1H-1,2,4-triazol-3-yl)pyridin-3-yl)-3,4-dihydropyrazino[2,3-b]pyrazin-2(1H)-on oder ein pharmazeutisch verträgliches Salz, Clathrat, Solvat, Stereoisomer, Tautomer oder Isotopologes davon zur Verwendung nach Anspruch 1 oder 5, wobei das E-twenty six (ETS) überexprimierende Ewing-Sarkom jenes ist, bei dem der PI3K/mTOR-Pathway aktiviert ist.

7. 1-Ethyl-7-(2-methyl-6-(1H-1,2,4-triazol-3-yl)pyridin-3-yl)-3,4-dihydropyrazino[2,3-b]pyrazin-2(1H)-on oder ein pharmazeutisch verträgliches Salz, Clathrat, Solvat, Stereoisomer, Tautomer oder Isotopologes davon zur Verwendung nach Anspruch 6, wobei das E-twenty six (ETS) überexprimierende Ewing-Sarkom jenes ist, bei dem der PI3K/mTOR-Pathway auf Grund von PTEN-Verlust, einer PIK3Ca-Mutation oder EGFR-Überexpression oder einer Kombination davon aktiviert ist.

## Revendications

1. 1-éthyl-7-(2-méthyl-6-(1H-1,2,4-triazol-3-yl)pyridin-3-yl)-3,4-dihydropyrazino[2,3-b]pyrazin-2(1H)-one ou un sel, clathrate, solvate, stéréo-isomère, tautomère ou isotopologue de ce dernier, acceptable d'un point de vue pharmaceutique pour une utilisation dans un procédé pour traiter le sarcome d'Ewing surexprimant E-Vingt-six (ETS), le procédé comprenant l'administration d'une quantité efficace de 1-éthyl-7-(2-méthyl-6-(1H-1,2,4-triazol-3-yl)pyridin-3-yl)-3,4-dihydropyrazino[2,3-b]pyrazin-2(1H)-one ou un sel, clathrate, solvate, stéréo-isomère, tautomère ou isotopologue de ce dernier, acceptable d'un point de vue pharmaceutique, à un patient ayant un sarcome d'Ewing surexprimant ETS.

2. 1-éthyl-7-(2-méthyl-6-(1H-1,2,4-triazol-3-yl)pyridin-3-yl)-3,4-dihydropyrazino[2,3-b]pyrazin-2(1H)-one ou un sel, clathrate, solvate, stéréo-isomère, tautomère ou isotopologue de ce dernier, acceptable d'un point de vue pharmaceutique pour une utilisation selon la revendication 1, dans lequel ledit patient se voit administrer environ 0,5 mg/jour à environ 120 mg/jour de 1-éthyl-7-(2-méthyl-6-(1H-1,2,4-triazol-3-yl)pyridin-3-yl)-3,4-dihydropyrazino[2,3-b]pyrazin-2(1H)-one ou un sel, clathrate, solvate, stéréo-isomère, tautomère ou isotopologue de ce dernier, acceptable d'un point de vue pharmaceutique.

3. 1-éthyl-7-(2-méthyl-6-(1H-1,2,4-triazol-3-yl)pyridin-3-yl)-3,4-dihydropyrazino[2,3-b]pyrazin-2(1H)-one ou un sel, clathrate, solvate, stéréo-isomère, tautomère ou isotopologue de ce dernier, acceptable d'un point de vue pharmaceutique pour une utilisation selon la revendication 2, dans lequel ledit patient se voit administrer 0,5 mg/jour, 1 mg/jour, 2 mg/jour, 4 mg/jour, 8 mg/jour, 16 mg/jour, 20 mg/jour, 30 mg/jour, 45 mg/jour, 60 mg/jour, 90 mg/jour, ou 120 mg/jour de 1-éthyl-7-(2-méthyl-6-(1H-1,2,4-triazol-3-yl)pyridin-3-yl)-3,4-dihydropyrazino[2,3-b]pyrazin-2(1H)-one ou un sel, clathrate, solvate, stéréo-isomère, tautomère ou isotopologue de ce dernier, acceptable d'un point de vue pharmaceutique.

4. 1-éthyl-7-(2-méthyl-6-(1H-1,2,4-triazol-3-yl)pyridin-3-yl)-3,4-dihydropyrazino[2,3-b]pyrazin-2(1H)-one ou un sel, clathrate, solvate, stéréo-isomère, tautomère ou isotopologue de ce dernier, acceptable d'un point de vue pharmaceutique pour une utilisation selon la revendication 1, dans lequel ledit patient se voit administrer une forme de dosage unitaire comprenant 0,25 mg, 1,0 mg, 5,0 mg, 7,5 mg, ou 10 mg de 1-éthyl-7-(2-méthyl-6-(1H-1,2,4-triazol-3-yl)pyridin-3-yl)-3,4-dihydropyrazino[2,3-b]pyrazin-2(1H)-one ou un sel, clathrate, solvate, stéréo-isomère, tautomère ou isotopologue de ce dernier, acceptable d'un point de vue pharmaceutique.

5. 1-éthyl-7-(2-méthyl-6-(1H-1,2,4-triazol-3-yl)pyridin-3-yl)-3,4-dihydropyrazino[2,3-b]pyrazin-2(1H)-one ou un sel, clathrate, solvate, stéréo-isomère, tautomère ou isotopologue de ce dernier, acceptable d'un point de vue pharmaceutique pour une utilisation dans un procédé pour améliorer les Critères d'Évaluation de Réponse pour des Tumeurs Solides (RECIST 1.1) d'un patient, le procédé comprenant l'administration d'une quantité efficace de 1-éthyl-7-(2-méthyl-6-(1H-1,2,4-triazol-3-yl)pyridin-3-yl)-3,4-dihydropyrazino[2,3-b]pyrazin-2(1H)-one ou d'un sel, clathrate, solvate, stéréo-isomère, tautomère ou isotopologue de ce dernier, acceptable d'un point de vue pharmaceutique, à un patient ayant un sarcome d'Ewing surexprimant E-Vingt-six (ETS).

6. 1-éthyl-7-(2-méthyl-6-(1H-1,2,4-triazol-3-yl)pyridin-3-yl)-3,4-dihydropyrazino[2,3-b]pyrazin-2(1H)-one ou un sel, clathrate, solvate, stéréo-isomère, tautomère ou isotopologue de ce dernier, acceptable d'un point de vue pharmaceutique pour une utilisation selon la revendication 1 ou 5, dans lequel le sarcome d'Ewing surexprimant E-Vingt-six (ETS) est celui dans lequel le chemin PI3K/mTOR est activé.

7. 1-éthyl-7-(2-méthyl-6-(1H-1,2,4-triazol-3-yl)pyridin-3-yl)-3,4-dihydropyrazino[2,3-b]pyrazin-2(1H)-one ou un sel, clathrate, solvate, stéréo-isomère, tautomère ou isotopologue de ce dernier, acceptable d'un point de vue pharmaceutique, pour une utilisation selon la revendication 6, dans lequel le sarcome d'Ewing surexprimant E-Vingt-six (ETS) est celui dans lequel le chemin PI3K/mTOR est activé en raison d'une perte de PTEN, d'une mutation de PIK3Ca ou d'une surexpression d'EGFR, ou d'une combinaison de celles-ci.
